# EUROPEAN PATENT SPECIFICATION

(11) **EP 2 877 159 B1**
(45) Date of publication and mention of the grant of the patent: **08.11.2017**
(21) Application number: 13745549.9
(22) Date of filing: 26.07.2013
(51) Int. Cl.: A61K 9/14, A61K 31/473, A61K 9/51

(54) **EFFLUX INHIBITOR COMPOSITIONS AND METHODS OF TREATMENT USING THE SAME**
EFFLUX-INHIBITORZUSAMMENSETZUNGEN UND BEHANDLUNGSVERFAHREN DAMIT
COMPOSITIONS INHIBITRICES D'ÉCOULEMENT ET MÉTHODES DE TRAITEMENT À L'AIDE CELLES-CI

(30) Priority: 27.07.2012 US 201261676689 P
(43) Date of publication of application: 03.06.2015
(73) Proprietor: Izumi Technology, LLC., Lexington, MA 02420 (US)
(72) Inventor: VAN TELLINGEN, Olaf, NL-1507 TC Zaandam (NL)
(74) Representative: FRKelly
(86) International application number: PCT/US2013/052402
(87) International publication number: WO 2014/018932

(56) References cited:
- US-A1- 2009 170 880
- BREEDVELD P ET AL: "The Effect of Bcrp1 (Abcg2) on the In vivo Pharmacokinetics and Brain Penetration of Imatinib Mesylate (Gleevec): Implications for the Use of Breast Cancer Resistance Protein and P-Glycoprotein Inhibitors to Enable the Brain Penetration of Imatinib in Patients", CANCER RESEARCH, AMERICAN ASSOCIATION FOR CANCER RESEARCH, US, vol. 65, no. 7, 1 April 2005 (2005-04-01), pages 2577-2582, XP007901255, ISSN: 0008-5472, DOI: 10.1158/0008-5472.CAN-04-2416
- DE VRIES N A ET AL: "P-Glycoprotein and breast cancer resistance protein: two dominant transporters working together in limiting the brain penetration of topotecan", CLINICAL CANCER RESEARCH, THE AMERICAN ASSOCIATION FOR CANCER RESEARCH, US, vol. 13, no. 21, 1 November 2007 (2007-11-01), pages 6440-6449, XP002502538, ISSN: 1078-0432, DOI: 10.1158/1078-0432.CCR-07-1335

## Description

### FIELD OF THE INVENTION

The present invention relates to efflux inhibitor compositions and methods of using these agents for treating conditions where the activity of efflux transporter proteins (e.g., Breast Cancer Resistance Protein (BCRP) and P-Glycoprotein (P-GP)) inhibit effective delivery of a therapeutic agent to a target tissue (e.g., brain, spinal cord, nerves, cerebrospinal fluid, testis, eyeballs, retina, inner ear, placenta, mammary gland, liver, biliary tract, kidney, intestines, lung, adrenal cortex, endometrium, hematopoietic cells, and/or stem cells).

### BACKGROUND

Neurofibromatosis (NF) is a genetic disorder of the nervous system, which causes tumors to form on nerve tissues, such as the brain, spinal cord, and peripheral nerves. Particularly, Type 1 neurofibromatosis (NF1) occurs in one out of every 3,000 children and affects approximately 100,000 people in the United States. NF1 can lead to blindness, disfigurement, malignancies, and learning disabilities in more than 50% of the affected children. Currently, there is no proven drug treatment for NF1.

In another example, the incidence of breast cancer brain metastasis (BCBM) in patients is approximately 30% and 20,000-34,000 patients develop BCBM each year. The standard of care for these patients is palliative care and includes steroids, anti-epilepsy drugs, pain medications, radiotherapy, and surgery. The life expectancy for these patients is only twelve months. Effective treatment for BCBM remains to be developed.

A significant challenge in the treatment of neurological disorders/conditions such as NF1 and BCBM is the efficient delivery of therapeutic agents across the blood-brain and/or the blood-nerve barriers to target lesions in the central and peripheral nervous systems. Physiologically, the blood-brain barrier and the blood-nerve barrier act to protect the brain and the endoneurial microenvironment from, for example, rapid fluctuations in the composition of the blood or of the extra neural spaces. However, in the process of protecting the nervous systems, the blood-brain and the blood-nerve barriers also present obstacles for delivering potentially useful therapeutic agents to the brain and the endoneurial microenvironment. Accordingly, there remains a need for new methods of enhancing the distribution of therapeutic agents into diseased tissues or cells that are protected by the blood-organ barrier and/or the efflux transporters P-GP and or BCRP for the prevention and/or treatment of conditions where treatment with a therapeutic agent is inhibited by BCRP and/or P-GP activity, e.g., neurological conditions.

### SUMMARY OF THE INVENTION

The present invention is based in part on the discovery that a composition comprising at least one efflux inhibitor (e.g., elacridar) enhances the penetration of one or more therapeutic agents (e.g., imatinib and lapatinib) across the blood-brain barrier and/or the blood-nerve barrier in mammals (e,g., humans). Accordingly, the present invention provides compositions and methods for treating conditions (e.g., NF1 and BCBM) where the activity of efflux transport proteins (e.g., BCRP and/or P-GP) inhibit effective delivery of a therapeutic agent to a target tissue (e.g., brain, spinal cord, nerves, cerebrospinal fluid, testis, eyeballs, retina, inner ear, placenta, mammary gland, liver, biliary tract, kidney, intestines, lung, adrenal cortex, endometrium, hematopoietic cells, and/or stem cells).

In a first aspect, the invention provides compositions comprising an efflux inhibitor, wherein the efflux inhibitor is formulated to achieve one or more of: 1) a Cmax of at least 500ng/ml (e.g., about 500, 550, 600, 650, 700, 750, 800, 850, 900, 950, or 1000 ng/ml); 2) a bioavailability of at least 0.1 (e.g., about 0.1, 0.2, 0.3, 0.4, 0.5, 0.6, 0.7, 0.8, 0.9, or 1.0); 3) an AUC(0-48h) of at least 900 ug/ml*min (e.g., about 900, 1000, 1100, 1200, 1300, 1400, 1500, 1600, 1700, 1800, 1900, 2000, 2500, 3000, 3500, 4000, 4500, or 5000 ug/ml*min); 4) an AUC(0-∞) of at least 1100 ug/ml*min (e.g., about 1100, 1200, 1300, 1400, 1500, 1600, 1700, 1800, 1900, 2000, 2500, 3000, 3500, 4000, 4500, or 5000 ug/ml*min); and, 5) an elimination half-life (T1/2) of at least 10 h (e.g., 10, 11, 12, 13, 14, 15, 16, 17, 18, 19, 20, 21, 22, 23, or 24 h), when the composition is administered by oral gavage at 100 mg/kg to fasted, female Sprague-Dawley rats.

In certain embodiments, the composition comprises a nanoparticle formulation of the efflux inhibitor. In certain embodiments, the nanoparticles have a mean diameter of between about 1 and 200 nM (e.g., about 1, 10, 20, 30, 40, 50, 60, 70, 80, 90, 100, 110, 120, 0130, 140, 150, 160, 170, 180, 190, or 200, 300, 400, 500, 600, 700, 800, 900, 100, 1100, 1200, 1300, 1400, 1500, 1600, 1700, 1800, 1900, or 2000 nM).

In certain embodiments, the efflux inhibitor is selected from one or more members of the group consisting of a Breast Cancer Resistance Protein (BCRP) inhibitor, and a P-Glycoprotein (P-GP) inhibitor.

In certain embodiments, the efflux inhibitor is a BCRP inhibitor selected from the group consisting of chrysin, gefitinib, Ko143, fumitremorgin C, diethylstilbestrol, cyclosporine-A, prazosin, saquinavir, ritonavir, β - estradiol, verapamil, tamoxifen, Hoechst 33342, quercetin, omeprazole, methotrexate, ergocristine, nicardipine, ethinylestradiol, astemizole, felodipine, glibenclamide, ketoconazole, chlorprotixene, nitrendipine, chlorpromazine, progesterone, mifepristone, dipyridamole, lopinavir, amiodarone, simvastatin, loperamide, terfenadine, clotrimazol, spironolactone, maprotiline, digoxin, quinine, fexofenadine, diltiazem, erythromycin, etoposide, prednisone, trimethoprim, chlorzoxazone, folic acid, lansoprazol, ranitidine, cimetidine, indomethacin, prednisolone, propranolol, timolol, desipramine, pravastatin, hydrocortisone, sulfinpyrazone, fenofibrate, tipranavir, erlotinib, flupentixol, celecoxib, thioridazine, isradipine, fendiline, medroxyprogesterone, pramoxine, piroxicam, terazosin, diazoxide, oxazepam, propafenone, tinidazole, meclizine, tetracycline, budesonide, desmethyldiazepam, nevirapine, diazepam, zanamivir, flurbiprofen, neomycin sulfate, nitrofurantoin, valacyclovir, carbamazepine, chenodeoxycholic acid, hydrochlorothiazide, amantadine, amoxicillin, phenytoin, antipyrine, bendroflumethiazide, ganciclovir, metoclopramide, pindolol, warfarin, amiloride, bupivacaine, carisoprodol, nizatidine, orphenadrine, procyclidine, acyclovir, atropine, captopril, furosemide, hydralazine, levothyroxine, salicylic acid, sotalol, valganciclovir, levodopa, methimazole, sulindac, metoprolol, zidovudine, gliclazide, mesalazine, bupropion, and sulfasalazine.

In certain embodiments, the efflux inhibitor is a P-GP inhibitor selected from the group consisting of alfentanil, amiloride, amiodarone, amitripyline, astemizole, atovaquone, atorvastatin, azelastine, azidopine, azithromycin, bepidil, biricodar, bromocriptine, carbamazepine, carvedilol, chloroquine, chlorpromazine, clarithromycin, cyclosporin, cyproheptadine, darunavir, desethylamiodarone, desipramine, dexniguldipine, dexrazoxane, diltiazem, dipyridamole, disulfiram, doxazosin, elicridqr, emetine, erythromycin, felodipine, fenofibrate, fentanyl, flavonoids, fluoxetine, fluphenazine, fluvoxamine, fucidin, gallpamil, glyburide, gramicidin D, grapefruit juice, garlic, green tea (catechins), haloperidol, hydrocortisone, hyroxyzine, josamycin, ketoconazole, imipramine, itraconazole, ivermectin, ketoconazole, laniquidar, lansoprazole, levothyroxin, lidocaine, loperamide, lopinavir-acute, loratadine, lovastatin, maprotiline, mefloquine, methadone, mibefradil, midazolam, mitomycin C, nefazodone, nelfinavir, nicardipine, nitrendipine, nobilitin, norverapamil, omeprazole, orange juice-Seville, ofloxacin, paroxetine, phenothiazines, piperine, pimozide, probenecid, progesterone, promethazine, propafenone, propranolol, quercetin, quinacrine, quinidine, quinine, reserpine, ritonavir, saquinavir, sertraline, simvastatin, spironolactone, sufentanil, tacrolimus, tamoxifen, tariquidar, telithromycin, terfenadine, testosterone, tetrabenzine, thioridazine, trifluoperazine, trifluopromazine, trimipramine, valinomycin, vanadate, venlafaxine, verapamil, vinblastine, FK506, RU486 (mifepristone), Valspodar PSG 833, zosuquidar, 2npropylquinoline, and ONT-093.

In certain embodiments, the efflux inhibitor is a dual BCRP and P-GP inhibitor. In certain embodiments, the efflux inhibitor is selected from the group consisting of elacridar, biricodar, pantoprazole, and tariquidar. In certain embodiments, the efflux inhibitor is elacridar.

In certain embodiments, the composition or nanoparticle formulation comprises at least 1 % elacridar weight/weight (w/w) (e.g., about 1, 2, 3, 4, 5, 6, 7, 8, 9, 10, 11, 12, 13, 14, 15, 16, 17, 18, 19, 20, 25, 30, 35, 40, 45, 50 % % w/w).

In certain embodiments, the composition or nanoparticle formulation further comprises a permeation enhancer. Suitable permeation enhancers include, without limitation, D-α-Tocopherol polyethylene glycol succinate (TPGS), dioctyl sodium sulfosuccinate, sodium caprate, sodium N-[8(-2-hydroxybenzoyl)amino]caprylate (SNAC), sodium lauryl sulfate, sodium salicylate, oleic acid, lecithin, dehydrated alcohol, Tween, Span, polyoxyl 40 stearate, polyoxy ethylene 50 stearate, polyethylene glycol, polyvinyl alcohol, polyvinylpyrrolidone (e.g., polyvinylpyrrolidone K29-32), hydroxy propyl methyl cellulose, polyvinylpyrrolidone/vinyl acetate (VP/VA) copolymer, poly(lactic-co-glycolic acid), edetate disodium, propylene glycol, glycerol monooleate, fusieates, bile salts, octoxynol, non-ionic surfactants, anionic surfactants and cationic surfactants. In certain embodiments, the permeation enhancer is TPGS. In certain embodiments, the composition or nanoparticle formulation comprises at least about 1 % TPGS w/w (e.g., about 1, 2, 3, 4, 5, 6, 7, 8, 9, 10, 11, 12, 13, 14, 15, 16, 17, 18, 19, 20, 25, 30, 35, 40, 45, 50% w/w). In certain embodiments, the composition or nanoparticle formulation comprises at least about 16% TPGS w/w.

In certain embodiments, the composition or nanoparticle formulation further comprises a solubility enhancer. Suitable solubility enhancers include, without limitation, TPGS, polyethylene glycol 300, polyethylene glycol 400, ethanol, propylene glycol, glycerin, *N-methyl-2-pyrrolidone,* dimethylacetamide, and dimethylsulfoxide, Cremophor EL, Cremophor RH 40, Cremophor RH 60, polysorbate 20, polysorbate 80, Solutol HS 15, sorbitan monooleate, poloxamer 407, Labrafil M-1944CS, Labrafil M-2125CS, Labrasol, Gellucire 44/14, Softigen 767, mono- and di-fatty acid esters of PEG 300, 400, or 1750, water-insoluble lipids, organic liquids/semi-solids, and cyclodextrins. In certain embodiments, the solubility enhancer is poloxamer 407. In certain embodiments, the composition or nanoparticle formulation comprises at least about 1% poloxamer 407 w/w (e.g., about 1, 2, 3, 4, 5, 6, 7, 8, 9, 10, 11, 12, 13, 14, 15, 16, 17, 18, 19, 20, 25, 30, 35, 40, 45, 50 % w/w).

In certain embodiments, the composition or nanoparticle formulation further comprises a therapeutic agent. In certain embodiments, the therapeutic agent is a modulator of a biological target. Suitable biological targets include, without limitation, enzymes, receptors, ion channels, nucleic acids, ribosomes, hormones, vitamins, cytokine, and chemokines.

In certain embodiments, the therapeutic agent is a kinase inhibitor. Suitable kinase inhibitors include, without limitation ABT-869, afatinib (BIBW-2992), AMG-706, AMN-107, amuvatinib, AST-487, axitinib (AG-013736), AZD-152HQPA, AZD-2171, BIBF-1120, BIRB-796, BMS-540215, bosutinib, cabozantinib, canertinib (CI-1 033), CHIR-258/TKI-258, crizotinib, dasatinib, DMBI, dovitinib, erlotinib, everolimus, EXEL-2880/GSK-1363089, gefitinib, GW-786034, imatinib, JNJ-28312141, Ki-20227, Ki8751, lapatinib, masitinib (AB-1 01 0), midostaurin (PKC-412), motesanib, neratinib (HKI-272), nilotinib, OSI-930, pazopanib, PD-173955, PLX-4720, ponatinib, PTK-787, quizartinib (AC220), R406, regorafenib, SKI-606, sorafenib, staurosporine, SU-14813, sunitinib, tandutinib (MLN-518), telatinib, temsirolimus, tivozanib, vandetanib, vatalanib, and vemurafenib.

In certain embodiments, the invention provides a composition comprising a nanoparticle formulation of elacridar, wherein the nanoparticle formulation comprises elacridar and TPGS. In certain embodiments, the nanoparticle formulation comprises about 5% elacridar and about 1 % TPGS w/w. In certain embodiments, the nanoparticle formulation is diluted in a TPGS aqueous solution (e.g., 1, 2, 3, 4, 5, 6, 7, 8, 9, or 10 fold) to a final concentration of at least 16% TPGS in the composition.

In certain embodiments, the invention provides a composition comprising a nanoparticle formulation of elacridar, wherein the nanoparticle formulation comprises elacridar and poloxamer 407. In certain embodiments, the nanoparticle formulation comprises about 5% elacridar and about 5% poloxamer 407 w/w. In certain embodiments, the nanoparticle formulation is diluted (e.g., 1, 2, 3, 4, 5, 6, 7, 8, 9, or 10 fold) in an aqueous solvent.

In second aspect, the invention provides a method for treating a condition in a subject wherein treatment with a therapeutic agent is inhibited by BCRP and/or P-GP activity, the method comprising administering to the subject a therapeutic amount of the first aspect of the invention, and a therapeutic agent useful for treating the condition, wherein the composition enhances the concentration of the therapeutic agent in the target tissue or cell.

In certain embodiments, the condition is a neurological condition. Any art recognized neurological condition (including those disclosed herein) can be treated using the methods of the invention. Exemplary neurological conditions include neurofibromatosis, neuro-cardio-facial-cutaneous syndromes, primary brain cancer, secondary brain metastasis, multiple sclerosis, and Alzheimer's disease. In one particular embodiment, the neurological condition is neurofibromatosis. In one particular embodiment, the neurological condition is glioblastoma multiforme. In one particular embodiment, the neurological condition is breast cancer brain metastasis.

In certain embodiments, the composition is administered transmucosally. In certain embodiments, the composition is administered rectally, vaginally, sublingually, bucally, intranasally, intracisternally, intraperitoneally, or intra-aurally. In certain embodiments, the composition is administered in a suppository, or hydrogel.

In certain embodiments, the therapeutic agent is a modulator (e.g., an inhibitor, activator, antagonist, or agonist) of a biological target. Suitable biological targets include, without limitation, enzymes, receptors, ion channels, nucleic acids, ribosomes, hormones, vitamins, cytokine, chemokines, substrates, metabolites, proteins, transport molecules, physiochemical mechanisms, antigen-antibody interactions.

In certain embodiments, the therapeutic agent is a kinase inhibitor. Suitable kinase inhibitors include, without limitation ABT-869, afatinib (BIBW-2992), AMG-706, AMN-107, amuvatinib, AST-487, axitinib (AG-013736), AZD-152HQPA, AZD-2171, BIBF-1120, BIRB-796, BMS-540215, bosutinib, cabozantinib, canertinib (CI-1 033), CHIR-258/TKI-258, crizotinib, dasatinib, DMBI, dovitinib, erlotinib, everolimus, EXEL-2880/GSK-1363089, gefitinib, GW-786034, imatinib, JNJ-28312141, Ki-20227, Ki8751, lapatinib, masitinib (AB-1 01 0), midostaurin (PKC-412), motesanib, neratinib (HKI-272), nilotinib, OSI-930, pazopanib, PD-173955, PLX-4720, ponatinib, PTK-787, quizartinib (AC220), R406, regorafenib, SKI-606, sorafenib, staurosporine, SU-14813, sunitinib, tandutinib (MLN-518), telatinib, temsirolimus, tivozanib, vandetanib, vatalanib, and vemurafenib.

In certain embodiments, the composition and the therapeutic agent are administered simultaneously to the subject. In certain embodiments, the composition and the therapeutic agent are administered simultaneously to the subject via separate routes of administration.

In certain embodiments, the composition comprises the therapeutic agent.

In certain embodiments, the invention provides methods for preventing or treating a neurological condition (or any disease present in sanctuary sites, e.g., brain, spinal cord, nerves, cerebrospinal fluid, testis, eyeballs, retina, inner ear, placenta, mammary gland, liver, biliary tract, kidney, intestines, lung, adrenal cortex, endometrium, hematopoietic cells, and/or stem cells) in a mammalian (e.g., human) subject, by co-administering to the subject at least one inhibitor of tyrosine kinase and a nanoparticle or similar composition comprising at least one inhibitor of BCRP and/or P-GP. The present invention is useful in preventing or treating, for example, neurological conditions such as neurofibromatosis, neuro-cardio-facial-cutaneous syndromes, primary brain cancers including but not limited to astrocytic, oligodendroglial, oligoastrocytic, ependymal, choroid plexus, neuroepithelial, neuronal and mixed neuronal-glial, pineal, embryonal, cranial and paraspinal nerve, meningeal, and sellar region tumors (e.g., glioblastoma multiforme, tumors of the brain stem, hypophtalmic glioma, cerebellar astrocytoma, cerebral astrocytoma, medulloblastoma, ependymoma, neuroectodermal or pineal tumor), secondary brain metastases (e.g., breast cancer brain metastasis (BCBM)), multiple sclerosis, HIV-associated neurological disorders, epilepsy, Amyotrophic lateral sclerosis (ALS), Huntington's Disease, Parkinson's disease (PD), and Alzheimer's disease (AD) in a mammalian (e.g., human) subject.

In certain embodiments, the invention provides methods for enhancing the distribution into diseased sanctuary tissues or cells, protected by the blood-organ barrier and or the efflux transporters P-GP and or BCRP, of one or more therapeutic agents, that are substrates of either P-GP and or BCRP, such as at least one inhibitor of tyrosine kinase for the prevention and/or treatment of diseases of such sanctuary tissues or cells including neurological conditions in a mammalian (e.g., human) subject in need thereof, by co-administering to the subject at least one inhibitor of tyrosine kinase and a nanoparticle or similar composition comprising at least one inhibitor of BCRP and/or P-GP.

The co-administration of the one or more inhibitors of tyrosine kinase and the one or more inhibitors of BCRP and/or P-GP may be sequential. In one embodiment, the one or more inhibitors of tyrosine kinase are administered to the subject after administering the one or more inhibitors of BCRP and/or P-GP to the subject. In another embodiment, the one or more inhibitors of tyrosine kinase are administered to the subject prior to administering the one or more inhibitors of BCRP and/or P-GP to the subject. Alternatively, the one or more inhibitors of tyrosine kinase and the one or more inhibitors of BCRP and/or P-GP are administered simultaneously.

The present invention contemplates the use of at least one tyrosine kinase inhibitor such as, for example, inhibitors of c-Kit and/or Platelet-Derived Growth Factor Receptor (PDGFR), BCR-ABL, VEGFR, FLT3, RAF, MEK, ERK, SRC, BRAF, ALK, HGFRcMET, Hedgehog, TIE2, RET, MET, TRKB, and/or Epidermal Growth Factor Receptor (EGFR). Exemplary inhibitors of c-Kit and/or PDGFR include, but are not limited to, ABT-869, AMG-706, AMN-107, amuvatinib, AST-487, axitinib (AG-013736), AZD-1152HOPA, AZD-2171, BIBF-1120, BIRB-796, BMS-540215, bosutinib, CHIR-258/TKI-258, dasatinib, DMBI, dovitinib, EXEL-2880/GSK-1363089, GW-786034, imatinib, JNJ-28312141, Ki-20227, Ki8751, masitinib (AB-1010), midostaurin (PKC-412), motesanib, nilotinib, OSI-930, pazopanib, PD-173955, PLX-4720, ponatinib, PTK-787, quizartinib (AC220), R406, regorafenib, sorafenib, staurosporine, SU-14813, sunitinib, tandutinib (MLN-518), telatinib, tivozanib, and vatalanib. In an embodiment, the tyrosine kinase inhibitor is imatinib. Exemplary inhibitors of EGFR include, but are not limited to, afatinib (BIBW-2992), canertinib (CI-1033), erlotinib, gefitinib, neratinib (HKI-272), lapatinib, SKI-606, and vandetanib. In an embodiment, the tyrosine kinase inhibitor is lapatinib. The one or more tyrosine kinase inhibitors may be administered about once per week, about once per day, or more than once daily. In an embodiment, the one or more tyrosine kinase inhibitors are administered orally. In another embodiment, the one or more tyrosine kinase inhibitors are administered parenterally, for example, intravenously. In a further embodiment, the one or more tyrosine kinase inhibitors are administered topically (*e.g.,* to lesions on the skin or to the eye). In certain embodiments, the one or more tyrosine kinase inhibitors are administered transmucosally. In certain embodiments, the one or more tyrosine kinase inhibitors are administered rectally, vaginally, sublingually, bucally, or intranasally. In certain embodiments, the one or more tyrosine kinase inhibitors are administered in a suppository, or hydrogel.

The one or more tyrosine kinase inhibitors may be administered at a daily dose of about 1 mg to about 2,000 mg. For example, the one or more tyrosine kinase inhibitors may be administered at a daily dose of about 400 mg. In another example, the one or more tyrosine kinase inhibitors may be administered at a daily dose of about 1,500 mg. In one embodiment, the one or more tyrosine kinase inhibitors are administered at a dosage of about 1 mg per kg to about 250 mg per kg of body weight.

The present invention also contemplates the use of a composition comprising at least one inhibitor of BCRP and/or P-GP. It is believed that the composition comprising at least one inhibitor of BCRP and/or P-GP enhances the permeability of the blood-brain barrier and/or the blood-nerve barrier to one or more tyrosine kinase inhibitors in mammalian (e.g., human) subject. In an embodiment, the inhibitor of BCRP and/or P-GP is a dual inhibitor of BCRP and P-GP. Exemplary dual inhibitors of BCRP and P-GP include, but are not limited to, elacridar, biricodar, pantoprazole, and tariquidar. In another embodiment, the use of at least one BCRP inhibitor is contemplated. Exemplary inhibitors of BCRP include, but are not limited to, chrysin, gefitinib, Ko143, fumitremorgin C, diethylstilbestrol, cyclosporine-A, prazosin, saquinavir, ritonavir, β-estradiol, verapamil, tamoxifen, Hoechst 33342, quercetin, omeprazole, methotrexate, ergocristine, nicardipine, ethinylestradiol, astemizole, felodipine, glibenclamide, ketoconazole, chlorprotixene, nitrendipine, chlorpromazine, progesterone, mifepristone, dipyridamole, lopinavir, amiodarone, simvastatin, loperamide, terfenadine, clotrimazol, spironolactone, maprotiline, digoxin, quinine, fexofenadine, diltiazem, erythromycin, etoposide, prednisone, trimethoprim, chlorzoxazone, folic acid, lansoprazol, ranitidine, cimetidine, indomethacin, prednisolone, propranolol, timolol, desipramine, pravastatin, hydrocortisone, sulfinpyrazone, fenofibrate, tipranavir, erlotinib, flupentixol, celecoxib, thioridazine, isradipine, fendiline, medroxyprogesterone, pramoxine, piroxicam, terazosin, diazoxide, oxazepam, propafenone, tinidazole, meclizine, tetracycline, budesonide, desmethyldiazepam, nevirapine, diazepam, zanamivir, flurbiprofen, neomycin sulfate, nitrofurantoin, valacyclovir, carbamazepine, chenodeoxycholic acid, hydrochlorothiazide, amantadine, amoxicillin, phenytoin, antipyrine, bendroflumethiazide, ganciclovir, metoclopramide, pindolol, warfarin, amiloride, bupivacaine, carisoprodol, nizatidine, orphenadrine, procyclidine, acyclovir, atropine, captopril, furosemide, hydralazine, levothyroxine, salicylic acid, sotalol, valganciclovir, levodopa, methimazole, sulindac, metoprolol, zidovudine, gliclazide, mesalazine, bupropion, and sulfasalazine. In a further embodiment, the use of at lest one P-GP inhibitor is contemplated. Exemplary inhibitors of P-GP include, but are not limited to, alfentanil, amiloride, amiodarone, amitripyline, astemizole, atovaquone, atorvastatin, azelastine, azidopine, azithromycin, bepidil, biricodar, bromocriptine, carbamazepine, carvedilol, chloroquine, chlorpromazine, clarithromycin, cyclosporin, cyproheptadine, darunavir, desethylamiodarone, desipramine, dexniguldipine, dexrazoxane, diltiazem, dipyridamole, disulfiram, doxazosin, elicridqr, emetine, erythromycin, felodipine, fenofibrate, fentanyl, flavonoids, fluoxetine, fluphenazine, fluvoxamine, fucidin, gallpamil, glyburide, gramicidin D, grapefruit juice, garlic, green tea (catechins), haloperidol, hydrocortisone, hyroxyzine, josamycin, ketoconazole, imipramine, itraconazole, ivermectin, ketoconazole, laniquidar, lansoprazole, levothyroxin, lidocaine, loperamide, lopinavir-acute, loratadine, lovastatin, maprotiline, mefloquine, methadone, mibefradil, midazolam, mitomycin C, nefazodone, nelfinavir, nicardipine, nitrendipine, nobilitin, norverapamil, omeprazole, orange juice-Seville, ofloxacin, paroxetine, phenothiazines, piperine, pimozide, probenecid, progesterone, promethazine, propafenone, propranolol, quercetin, quinacrine, quinidine, quinine, reserpine, ritonavir, saquinavir, sertraline, simvastatin, spironolactone, sufentanil, tacrolimus, tamoxifen, tariquidar, telithromycin, terfenadine, testosterone, tetrabenzine, thioridazine, trifluoperazine, trifluopromazine, trimipramine, valinomycin, vanadate, (venlafaxine), verapamil, vinblastine, FK506, RU486 (mifepristone), Valspodar PSC 833, zosuquidar, 2n-propylquinoline, and ONT-093.

The one or more inhibitors of BCRP and/or P-GP may be administered about once per week, about once per day, or more than once daily. In an embodiment, the one or more inhibitors of BCRP and/or P-GP are administered orally. In another embodiment, the one or more inhibitors of BCRP and/or P-GP are administered parenterally, for example, intravenously. In a further embodiment, the one or more inhibitors of BCRP and/or P-GP are administered topically (*e.g.,* to lesions on the skin or to the eye). The one or more inhibitors of BCRP and/or P-GP may be administered at a daily dose of about 1 mg to about 1,500 mg. For example, the one or more inhibitors of BCRP and/or P-GP may be administered at a daily dose of about 200 mg. In one embodiment, the one or more inhibitors of BCRP and/or P-GP are administered at a dosage of about 1 mg to about 250 mg per kg of body weight.

### DESCRIPTION OF FIGURES

FIGURE 1 is a graph showing the results of an MDCK cell permeability assay testing the permeability of elacridar.
FIGURE 2 is a graph of a plasma concentration-time curve of elacridar after intravenous (i.v.) administration to rats.
FIGURE 3 is a graph of plasma concentration-time curves (mean ± SE) of elacridar formulations after oral administration to rats

### DETAILED DESCRIPTION OF THE INVENTION

The present invention relates to compositions and methods, which are useful in preventing or treating conditions (e.g., neurological conditions such as NF1 and BCBM) where the activity of efflux transport proteins (e.g., BCRP and/or P-GP) inhibit effective delivery of a therapeutic agent to a target tissue (e.g., brain, spinal cord, nerves, testis, eyeballs, retina, inner ear, cerebrospinal fluid, testis, eyeballs, retina, inner ear, placenta, mammary gland, endometrium,).. More specifically, the present invention is based in part on the discovery that a nanoparticle or similar composition comprising at least one inhibitor of Breast Cancer Resistance Protein (BCRP) and/or P-Glycoprotein (P-GP) enhances the penetration of one or more inhibitors of tyrosine kinase across the blood-brain barrier and/or the blood-nerve barrier into nervous tissues in mammalian (e.g., human) subjects. Accordingly, the present invention provides compositions and methods for preventing or treating a neurological condition in a human subject by co-administering to a mammalian (e.g., human) subject in need thereof one or more therapeutic agents (e.g., tyrosine kinase inhibitors) with a nanoparticle or similar composition comprising at least one efflux inhibitor (e.g,an inhibitor of BCRP and/or P-GP). It is contemplated that the present invention provides specific therapeutic advantages such as sufficient drug concentration in diseased sanctuary tissues or cells, and/or enhanced efficacy of treatment, ease of use, novel indications and/or reduced side effects.

Various neurological conditions are associated with abnormal activation of tyrosine kinases. These conditions include, for example, neurofibromatosis, neuro-cardio-facial-cutaneous syndromes, primary brain cancers including but not limited to astrocytic, oligodendroglial, oligoastrocytic, ependymal, choroid plexus, other neuroepithelial, neuronal and mixed neuronal-glial, pineal, embryonal, cranial and paraspinal nerve, meningeal, and sellar region tumors (*e.g.,* glioblastoma multiforme, tumors of the brain stem, hypophtalmic glioma, cerebellar astrocytoma, cerebral astrocytoma, medulloblastoma, ependymoma, neuroectodermal or pineal tumor), secondary brain metastasis (*e.g.,* breast cancer brain metastasis (BCBM)), and multiple sclerosis. Accordingly, tyrosine kinase inhibitors have great potential as therapeutic agents for neurological conditions such as NF1 and BCBM.

Nonetheless, while systemic use of known tyrosine kinase inhibitors such as imatinib have demonstrated clinical efficacy in peripheral tumors such as gastrointestinal stromal tumor (GIST), such drugs have failed to demonstrate efficacy in tumors of the central and peripheral nervous systems. For example, when imatinib was tested in a Phase II clinical trial in NF1 patients with plexiform neurofibromas, the response rate was much lower than expected. Furthermore, when imatinib was tested in multiple Phase II clinical trials in adult and pediatric patients with primary brain cancer, again the response rate was much lower than expected. This may be attributed in part to the poor penetration of the drug into the brain and the peripheral nervous system. Particularly, Imatinib has been demonstrated to be a substrate for the ATP Binding Cassette (ABC) transporter, P-glycoprotein (P-GP), which may prevent the efficient penetration of imatinib across the blood-brain and/or the blood-nerve barriers (*See* Dai et al., (2003) J Pharmacol Exp Ther., 304: 1085 -1092). Furthermore, Imatinib has been demonstrated to be a substrate for the ATP Binding Cassette (ABC) transporter, Breast Cancer Resistance Protein (BCRP), which may also prevent the efficient penetration of imatinib across the blood-brain and/or the blood-nerve barriers. Furthermore, more recently P-GP and BCRP have been shown to work synergistically in effluxing or pumping drugs out of sanctuary tissues or cells (see e.g., Agarwal et al., (2011) Curr Pharm Des.; 17(26): 2793-2802, which is incorporated by reference herein in its entirety).

The development of P-GP inhibitors for increasing the intracellular concentrations of toxic chemotherapy agents in humans has been pursued (*See* Deeken et al., (2007) Clin. Can. Res., 13: 1663-1674). However, human clinical trials in both solid and hematologic malignancies testing P-GP inhibitors with cytotoxic P-GP substrates to overcome cancer cell resistance or multi drug resistance (MDR) have been disappointing. Particularly Phase III trials had to be stopped due to lack of efficacy and/or unacceptable toxicities. These negative results have put in doubt the strategy of overcoming drug resistance by the use of P-GP inhibitors in mammalian (e.g., human) subjects. However, the potential utility of P-GP and BCRP inhibitors with cytotoxic P-GP and/or BCRP substrates to overcome cancer cell resistance or multi drug resistance (MDR), as well as the utility of P-GP and/or BCRP inhibitors in overcoming the blood-brain and blood-nerve barriers in mammals (e.g., humans) are still open questions. Phase I dose-finding studies, including the combination of the dual BCRP and P-GP inhibitor elacridar and topotecan in cancer patients for assessing the dosing schedule and oral bioavailability of topotecan, are the only human clinical data reported to date.

Accordingly, there remains a need for new methods of enhancing the penetration of active agents such as imatinib across the blood-brain and/or the blood-nerve barriers for the prevention and/or treatment of neurological conditions in mammalian (e.g., human) subjects.

### Efflux inhibitors

The present invention utilizes compositions comprising at least one efflux inhibitor. As used herein the term "efflux inhibitor" refers to any agent that reduces or inhibits the expression and/or activity of at least one transport protein (e.g., BCRP and/or P-GP). In certain embodiments, the transport protein is BCRP and/or P-GP. Inhibitors of BCRP and/or P-GP are known in the art.

In one embodiment, at least one dual inhibitor of BCRP and P-GP is utilized. Exemplary dual inhibitors of BCRP and P-GP include, but are not limited to, elacridar, biricodar, pantoprazole, and tariquidar. In particular, elacridar has the following structure:

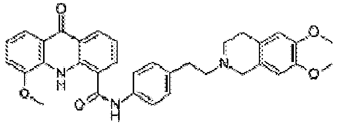

In another embodiment, the use of at least one BCRP inhibitor is contemplated. Exemplary inhibitors of BCRP include, but are not limited to, chrysin, gefitinib, Ko143, fumitremorgin C, diethylstilbestrol, cyclosporine-A, prazosin, saquinavir, ritonavir, β-estradiol, verapamil, tamoxifen, Hoechst 33342, quercetin, omeprazole, methotrexate, ergocristine, nicardipine, ethinylestradiol, astemizole, felodipine, glibenclamide, ketoconazole, chlorprotixene, nitrendipine, chlorpromazine, progesterone, mifepristone, dipyridamole, lopinavir, amiodarone, simvastatin, loperamide, terfenadine, clotrimazol, spironolactone, maprotiline, digoxin, quinine, fexofenadine, diltiazem, erythromycin, etoposide, prednisone, trimethoprim, chlorzoxazone, folic acid, lansoprazol, ranitidine, cimetidine, indomethacin, prednisolone, propranolol, timolol, desipramine, pravastatin, hydrocortisone, sulfinpyrazone, fenofibrate, tipranavir, erlotinib, flupentixol, celecoxib, thioridazine, isradipine, fendiline, medroxyprogesterone, pramoxine, piroxicam, terazosin, diazoxide, oxazepam, propafenone, tinidazole, meclizine, tetracycline, budesonide, desmethyldiazepam, nevirapine, diazepam, zanamivir, flurbiprofen, neomycin sulfate, nitrofurantoin, valacyclovir, carbamazepine, chenodeoxycholic acid, hydrochlorothiazide, amantadine, amoxicillin, phenytoin, antipyrine, bendroflumethiazide, ganciclovir, metoclopramide, pindolol, warfarin, amiloride, bupivacaine, carisoprodol, nizatidine, orphenadrine, procyclidine, acyclovir, atropine, captopril, furosemide, hydralazine, levothyroxine, salicylic acid, sotalol, valganciclovir, levodopa, methimazole, sulindac, metoprolol, zidovudine, gliclazide, mesalazine, bupropion, and sulfasalazine.

In a further embodiment, the use of at least one P-GP inhibitor is contemplated. Exemplary inhibitors of P-GP include, but are not limited to, alfentanil, amiloride, amiodarone, amitripyline, astemizole, atovaquone, atorvastatin, azelastine, azidopine, azithromycin, bepidil, biricodar, bromocriptine, carbamazepine, carvedilol, chloroquine, chlorpromazine, clarithromycin, cyclosporin, cyproheptadine, darunavir, desethylamiodarone, desipramine, dexniguldipine, dexrazoxane, diltiazem, dipyridamole, disulfiram, doxazosin, elicridqr, emetine, erythromycin, felodipine, fenofibrate, fentanyl, flavonoids, fluoxetine, fluphenazine, fluvoxamine, fucidin, gallpamil, glyburide, gramicidin D, grapefruit juice, garlic, green tea (catechins), haloperidol, hydrocortisone, hyroxyzine, josamycin, ketoconazole, imipramine, itraconazole, ivermectin, ketoconazole, laniquidar, lansoprazole, levothyroxin, lidocaine, loperamide, lopinavir-acute, loratadine, lovastatin, maprotiline, mefloquine, methadone, mibefradil, midazolam, mitomycin C, nefazodone, nelfinavir, nicardipine, nitrendipine, nobilitin, norverapamil, omeprazole, orange juice-Seville, ofloxacin, paroxetine, phenothiazines, piperine, pimozide, probenecid, progesterone, promethazine, propafenone, propranolol, quercetin, quinacrine, quinidine, quinine, reserpine, ritonavir, saquinavir, sertraline, simvastatin, spironolactone, sufentanil, tacrolimus, tamoxifen, tariquidar, telithromycin, terfenadine, testosterone, tetrabenzine, thioridazine, trifluoperazine, trifluopromazine, trimipramine, valinomycin, vanadate, (venlafaxine), verapamil, vinblastine, FK506, RU486 (mifepristone), Valspodar PSC 833, zosuquidar, 2n-propylquinoline, and ONT-093.

In one embodiment, the one or more inhibitors of tyrosine kinase are used in combination with a nanoparticle or similar composition comprising at least one dual BCRP and P-GP inhibitor. In another embodiment, the one or more inhibitors of tyrosine kinase are used in combination with a nanoparticle or similar composition comprising at least one BCRP inhibitor and at least one P-GP inhibitor.

Furthermore, the present invention contemplates the use of prodrugs of any of the therapeutic agents described herein that convert *in vivo* to the selective therapeutic agents.

### Therapeutic Agents

The present invention utilizes one or more therapeutic agents. As used herein the term "therapeutic agent" refers to a compound useful for treating or preventing a disease or disorder, or restoring or correcting a physiological function in a mammalian (e.g., human) subject. Any therapeutic agent that is a substrate for a transport protein (e.g., BCRP and/or P-GP) will be potentiated by the compositions disclosed herein.

In certain embodiments, the therapeutic agent is an enzyme inhibitor. In one embodiment, the enzyme inhibitor is a tyrosine kinase inhibitor. Any agent that reduces or inhibits the expression and/or activity of tyrosine kinases (*e.g.*, c-kit, PDGFR, EGFR) is contemplated herein. Small molecule inhibitors of tyrosine kinases are known in the art. For example, imatinib, a c-kit inhibitor (commercially available as GLEEVEC™ from Novartis Pharmaceuticals) is disclosed in U.S. Patent Nos. 5,521,184, 6,894,051, 6,958,335, and 7,544,799, and has the following chemical structure:

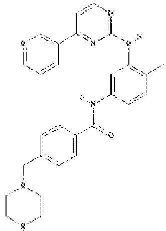

Another compound, nilotinib, (commercially available as TASIGNA™ from Novartis Pharmaceuticals) is disclosed in U.S. Patent No. 7,169,791. Yet another small molecule tyrosine kinase inhibitor is dasatinib (commercially available as SPRYCEL® by Bristol-Myers Squibb, Inc.), is detailed, for example, in U.S. Patent Nos. 6,596,746 and 7,125,875. Additional examples of inhibitors of tyrosine kinases include, for example, inhibitors of c-Kit and/or PDGFR such as ABT-869, AMG-706, AMN-107, amuvatinib, AST-487, axitinib (AG-013736), AZD-1152HOPA, AZD-2171, BIBF-1120, BIRB-796, BMS-540215, bosutinib, CHIR-258/TKI-258, DMBI, dovitinib, EXEL-2880/GSK-1363089, GW-786034, JNJ-28312141, Ki-20227, Ki8751, masitinib (AB-1010), midostaurin (PKC-412), motesanib, OSI-930, pazopanib, PD-173955, PLX-4720, ponatinib, PTK-787, quizartinib (AC220), R406, regorafenib, sorafenib, staurosporine, SU-14813, sunitinib, tandutinib (MLN-518), telatinib, tivozanib, and vatalanib. Other examples of inhibitors of tyrosine kinases include, for example, inhibitors of EGFR such as afatinib (BIBW-2992), canertinib (CI-1033), erlotinib, gefitinib, neratinib (HKI-272), lapatinib, SKI-606, and vandetanib. In an exemplary embodiment, the tyrosine kinase is imatinib. In another exemplary embodiment, the tyrosine kinase is lapatinib.

In certain embodiments, the therapeutic agent is a microtubule inhibitor (e.g.. a taxane or vinca alkaloids). Suitable microtubule inhibitors include, for example, paclitaxel and docetaxel.

In certain embodiments, the therapeutic agent is a receptor agonist or antagonist. In one embodiment, the therapeutic agent is a G-protein coupled receptor (GPCR) agonist or antagonist. Suitable GPCR agonist or antagonists include opioids and analogues thereof (e.g., loperamide).

In certain embodiments, the therapeutic agent is selected from the group consisting of irinotecan, atorvastatin, methotrexate, rosuvastatin, sulfasalazine, topotecan, ximelagatran, tenofovir, talinolol, tacrolimus, omeprazole, nelfinavir, morphine 6-glucuronide, morphine, idarubicin, fexofenadine (terfenadine carboxylate), ezetimibe, etoposide, doxorubicin, daunorubicin, erythromycin, loperamide, (R)-fexofenadine, (R)-talinolol, (R)-verapamil, (S)-fexofenadine, aliskiren, amitriptyline, amprenavir, atazanavir, atenolol, buprenorphine, carvedilol, cyclosporine, dabigatran, dabigatran etexilate, darunavir, dicloxacillin, digoxin, erythromycin, ezetimibe, indinavir, irinotecan, lapatinib, linezolid, lopinavir, maraviroc, metronidazole, moxifloxacin, ornidazole, phenytoin, ranitidine, risperidone, ritonavir, ritonavir, saquinavir, and simvastatin.

In certain embodiments, the therapeutic agent is an anti-cancer agent. Suitable anti-cancer agents include, without limitation, 20-epi-1, 25 dihydroxyvitamin D3,4-ipomeanol, 5-ethynyluracil, 9-dihydrotaxol, abiraterone, acivicin, aclarubicin, acodazole hydrochloride, acronine, acylfiilvene, adecypenol, adozelesin, aldesleukin, all-tk antagonists, altretamine, ambamustine, ambomycin, ametantrone acetate, amidox, amifostine, aminoglutethimide, aminolevulinic acid, amrubicin, amsacrine, anagrelide, anastrozole, andrographolide, angiogenesis inhibitors, antagonist D, antagonist G, antarelix, anthramycin, anti-dorsalizdng morphogenetic protein-1, antiestrogen, antineoplaston, antisense oligonucleotides, aphidicolin glycinate, apoptosis gene modulators, apoptosis regulators, apurinic acid, ARA-CDP-DL-PTBA, arginine deaminase, asparaginase, asperlin, asulacrine, atamestane, atrimustine, axinastatin 1, axinastatin 2, axinastatin 3, azacitidine, azasetron, azatoxin, azatyrosine, azetepa, azotomycin, baccatin III derivatives, balanol, batimastat, benzochlorins, benzodepa, benzoylstaurosporine, beta lactam derivatives, beta-alethine, betaclamycin B, betulinic acid, BFGF inhibitor, bicalutamide, bisantrene, bisantrene hydrochloride, bisazuidinylspermine, bisnafide, bisnafide dimesylate, bistratene A, bizelesin, bleomycin, bleomycin sulfate, BRC/ABL antagonists, breflate, brequinar sodium, bropirimine, budotitane, busulfan, buthionine sulfoximine, cactinomycin, calcipotriol, calphostin C, calusterone, camptothecin derivatives, canarypox IL-2, capecitabine, caraceraide, carbetimer, carboplatin, carboxamide-amino-triazole, carboxyamidotriazole, carest M3, carmustine, earn 700, cartilage derived inhibitor, carubicin hydrochloride, carzelesin, casein kinase inhibitors, castanosperrnine, cecropin B, cedefingol, cetrorelix, chlorambucil, chlorins, chloroquinoxaline sulfonamide, cicaprost, cirolemycin, cisplatin, cis-porphyrin, cladribine, clomifene analogs, clotrimazole, collismycin A, collismycin B, combretastatin A4, combretastatin analog, conagenin, crambescidin 816, crisnatol, crisnatol mesylate, cryptophycin 8, cryptophycin A derivatives, curacin A, cyclopentanthraquinones, cyclophosphamide, cycloplatam, cypemycin, cytarabine, cytarabine ocfosfate, cytolytic factor, cytostatin, dacarbazine, dacliximab, dactinomycin, daunorubicin hydrochloride, decitabine, dehydrodidemnin B, deslorelin, dexifosfamide, dexormaplatin, dexrazoxane, dexverapamil, dezaguanine, dezaguanine mesylate, diaziquone, didemnin B, didox, diethyhiorspermine, dihydro-5-azacytidine, dioxamycin, diphenyl spiromustine, docetaxel, docosanol, dolasetron, doxifluridine, doxorubicin, doxorubicin hydrochloride, droloxifene, droloxifene citrate, dromostanolone propionate, dronabinol, duazomycin, duocannycin SA, ebselen, ecomustine, edatrexate, edelfosine, edrecolomab, eflomithine, eflomithine hydrochloride, elemene, elsarnitrucin, emitefur, enloplatin, enpromate, epipropidine, epirubicin, epirubicin hydrochloride, epristeride, erbulozole, erythrocyte gene therapy vector system, esorubicin hydrochloride, estramustine, estramustine analog, estramustine phosphate sodium, estrogen agonists, estrogen antagonists, etanidazole, etoposide, etoposide phosphate, etoprine, exemestane, fadrozole, fadrozole hydrochloride, fazarabine, fenretinide, filgrastim, finasteride, flavopiridol, flezelastine, floxuridine, fluasterone, fludarabine, fludarabine phosphate, fluorodaunorunicin hydrochloride, fluorouracil, fluorocitabine, forfenimex, formestane, fosquidone, fostriecin, fostriecin sodium, fotemustine, gadolinium texaphyrin, gallium nitrate, galocitabine, ganirelix, gelatinase inhibitors, gemcitabine, gemcitabine hydrochloride, glutathione inhibitors, hepsulfam, heregulin, hexamethylene bisacetamide, hydroxyurea, hypericin, ibandronic acid, idarubicin, idarubicin hydrochloride, idoxifene, idramantone, ifosfamide, ihnofosine, ilomastat, imidazoacridones, imiquimod, immunostimulant peptides, insulin-like growth factor-1 receptor inhibitor, interferon agonists, interferon alpha-2A, interferon alpha-2B, interferon alpha-N1, interferon alpha-N3, interferon beta-IA, interferon gamma-IB, interferons, interleukins, iobenguane, iododoxorubicin, iproplatm, irinotecan, irinotecan hydrochloride, iroplact, irsogladine, isobengazole, isohomohalicondrin B, itasetron, jasplakinolide, kahalalide F, lamellarin-N triacetate, lanreotide, lanreotide acetate, leinamycin, lenograstim, lentinan sulfate, leptolstatin, letrozole, leukemia inhibiting factor, leukocyte alpha interferon, leuprolide acetate, leuprolide/estrogen/progesterone, leuprorelin, levamisole, liarozole, liarozole hydrochloride, linear polyamine analog, lipophilic disaccharide peptide, lipophilic platinum compounds, lissoclinamide, lobaplatin, lombricine, lometrexol, lometrexol sodium, lomustine, lonidamine, losoxantrone, losoxantrone hydrochloride, lovastatin, loxoribine, lurtotecan, lutetium texaphyrin lysofylline, lytic peptides, maitansine, mannostatin A, marimastat, masoprocol, maspin, matrilysin inhibitors, matrix metalloproteinase inhibitors, maytansine, mechlorethamine hydrochloride, megestrol acetate, melengestrol acetate, melphalan, menogaril, merbarone, mercaptopurine, meterelin, methioninase, methotrexate, methotrexate sodium, metoclopramide, metoprine, meturedepa, microalgal protein kinase C uihibitors, MIF inhibitor, mifepristone, miltefosine, mirimostim, mismatched double stranded RNA, mitindomide, mitocarcin, mitocromin, mitogillin, mitoguazone, mitolactol, mitomalcin, mitomycin, mitomycin analogs, mitonafide, mitosper, mitotane, mitotoxin fibroblast growth factor-saporin, mitoxantrone, mitoxantrone hydrochloride, mofarotene, molgramostim, monoclonal antibody, human chorionic gonadotrophin, monophosphoryl lipid a/myobacterium cell wall SK, mopidamol, multiple drug resistance gene inhibitor, multiple tumor suppressor 1-based therapy, mustard anticancer agent, mycaperoxide B, mycobacterial cell wall extract, mycophenolic acid, myriaporone, n-acetyldinaline, nafarelin, nagrestip, naloxone/pentazocine, napavin, naphterpin, nartograstim, nedaplatin, nemorubicin, neridronic acid, neutral endopeptidase, nilutamide, nisamycin, nitric oxide modulators, nitroxide antioxidant, nitrullyn, nocodazole, nogalamycin, n-substituted benzamides, O6-benzylguanine, octreotide, okicenone, oligonucleotides, onapristone, ondansetron, oracin, oral cytokine inducer, ormaplatin, osaterone, oxaliplatin, oxaunomycin, oxisuran, paclitaxel, paclitaxel analogs, paclitaxel derivatives, palauamine, palmitoylrhizoxin, pamidronic acid, panaxytriol, panomifene, parabactin, pazelliptine, pegaspargase, peldesine, peliomycin, pentamustine, pentosan polysulfate sodium, pentostatin, pentrozole, peplomycin sulfate, perflubron, perfosfamide, perillyl alcohol, phenazinomycin, phenylacetate, phosphatase inhibitors, picibanil, pilocarpine hydrochloride, pipobroman, piposulfan, pirarubicin, piritrexim, piroxantrone hydrochloride, placetin A, placetin B, plasminogen activator inhibitor, platinum complex, platinum compounds, platinum-triamine complex, plicamycin, plomestane, porfimer sodium, porfiromycin, prednimustine, procarbazine hydrochloride, propyl bis-acridone, prostaglandin J2, prostatic carcinoma antiandrogen, proteasome inhibitors, protein A-based immune modulator, protein kinase C inhibitor, protein tyrosine phosphatase inhibitors, purine nucleoside phosphorylase inhibitors, puromycin, puromycin hydrochloride, purpurins, pyrazorurin, pyrazoloacridine, pyridoxylated hemoglobin polyoxyethylene conjugate, RAF antagonists, raltitrexed, ramosetron, RAS farnesyl protein transferase inhibitors, RAS inhibitors, RAS-GAP inhibitor, retelliptine demethylated, rhenium RE 186 etidronate, rhizoxin, riboprine, ribozymes, RH retinamide, RNAi, rogletimide, rohitukine, romurtide, roquinimex, rubiginone B1, ruboxyl, safingol, safingol hydrochloride, saintopin, sarcnu, sarcophytol A, sargramostim, SDI1 mimetics, semustine, senescence derived inhibitor 1, sense oligonucleotides, signal transduction inhibitors, signal transduction modulators, simtrazene, single chain antigen binding protein, sizofuran, sobuzoxane, sodium borocaptate, sodium phenylacetate, solverol, somatomedin binding protein, sonermin, sparfosafe sodium, sparfosic acid, sparsomycin, spicamycin D, spirogermanium hydrochloride, spiromustine, spiroplatin, splenopentin, spongistatin 1, squalamine, stem cell inhibitor, stem-cell division inhibitors, stipiamide, streptonigrin, streptozocin, stromelysin inhibitors, sulfinosine, sulofenur, superactive vasoactive intestinal peptide antagonist, suradista, suramin, swainsonine, synthetic glycosaminoglycans, talisomycin, tallimustine, tamoxifen methiodide, tauromustine, tazarotene, tecogalan sodium, tegafur, tellurapyrylium, telomerase inhibitors, teloxantrone hydrochloride, temoporfin, temozolomide, teniposide, teroxirone, testolactone, tetrachlorodecaoxide, tetrazomine, thaliblastine, thalidomide, thiamiprine, thiocoraline, thioguanine, thiotepa, thrombopoietin, thrombopoietin mimetic, thymalfasin, thymopoietin receptor agonist, thymotrinan, thyroid stimulating hormone, tiazofurin, tin ethyl etiopurpurin, tirapazamine, titanocene dichloride, topotecan hydrochloride, topsentin, toremifene, toremifene citrate, totipotent stem cell factor, translation inhibitors, trestolone acetate, tretinoin, triacetyluridine, triciribine, triciribine phosphate, trimetrexate, trimetrexate glucuronate, triptorelin, tropisetron, tubulozole hydrochloride, turosteride, tyrosine kinase inhibitors, tyrphostins, UBC inhibitors, ubenimex, uracil mustard, uredepa, urogenital sinus-derived growth inhibitory factor, urokinase receptor antagonists, vapreotide, variolin B, velaresol, veramine, verdins, verteporfin, vinblastine sulfate, vincristine sulfate, vindesine, vindesine sulfate, vinepidine sulfate, vinglycinate sulfate, vinleurosine sulfate, vinorelbine, vinorelbine tartrate, vinrosidine sulfate, vinxaltine, vinzolidine sulfate, vitaxin, vorozole, zanoterone, zeniplatin, zilascorb, zinostatin, zinostatin stimalamer, and zorubicin hydrochloride.

In certain embodiments, the therapeutic agent is an anti-epileptic agent. Suitable anti-epileptic agents include, without limitation, carbamazepine, ethosuximide, lamotrigine, levetiracetam, oxcarbazepine, sodium valproate, acetazolamide, clobazam, clonazepam, eslicarbazepine acetate, gabapentin, lacosamide, perampanel, phenobarbital, phenytoin, piracetam, pregabalin, primidone, retigabine, rufinamide, stiripentol, tiagabine, topiramate, vigabatrin, and zonisamide.

In certain embodiments, the therapeutic agent is an anti-depressant or anti-psychotic agent. Suitable anti-depressant or anti-psychotic agents include, without limitation, aripiprazole, chlorpromazine, clozapine, fluphenazine (generic only), haloperidol, iloperidone, loxapine, molindone, olanzapine, paliperidone, perphenazine (generic only), pimozide (for Tourette's syndrome), quetiapine, risperidone, thioridazine (generic only), thiothixene, trifluoperazine, ziprasidone, amitriptyline, amoxapine, bupropion, citalopram, clomipramine, desipramine, desvenlafaxine, doxepin, duloxetine, escitalopram, fluoxetine, fluvoxamine, imipramine, imipramine pamoate, isocarboxazid, maprotiline , mirtazapine, nortriptyline , paroxetine , paroxetine mesylate , phenelzine , protriptyline , selegiline, sertraline , tranylcypromine , trazodone, trimipramine , venlafaxine , carbamazepine, divalproex sodium, gabapentin, lamotrigine, lithium carbonate, lithium citrate , oxcarbazepine, topiramate, alprazolam, buspirone, chlordiazepoxide, clonazepam, clorazepate, diazepam, lorazepam, oxazepam , amphetamine, atomoxetine, dexmethylphenidate, dextroamphetamine, guanfacine, lisdexamfetamine dimesylate, methamphetamine, and methylphenidate.

In certain embodiments, the therapeutic agent is abilify (aripiprazole), abraxane (paclitaxel protein-bound particles for injectable suspension), abreva (docosanol), abstral (fentanyl sublingual tablets), accolate, accolate, accretropin (somatropin rdna original), aciphex (rabeprazole sodium), actemra (tocilizumab), actemra (tocilizumab), actiq, activella (estradiol/norethindrone acetate) tablets, actonel, actoplus met (pioglitazone hydrochloride and metformin hydrochloride), actos, acular (ketorolac tromethamine ophthalmic solution) 0.5%, acular (ketorolac tromethamine ophthalmic solution) 0.5%, acuvail (ketorolac tromethamine), acyclovir capsules, adcirca (tadalafil), adcretris (brentuximab vedotin), adderall (mixed salts of a single-entity amphetamine), adderall xr, advicor (extended-release niacin/lovastatin), afinitor (everolimus), afinitor (everolimus), afinitor (everolimus), afinitor (everolimus), agenerase (amprenavir), aggrenox, agrylin (anagrelide hcl), agrylin (anagrelide hcl), ak-con-a (naphazoline ophthalmic), akten (lidocaine hydrochloride), alamast, albenza (albendazole), aldara (imiquimod), aldurazyme (laronidase), alesse (100 mcg levonorgestrel/20 mcg ethinyl estradiol tablets), alimta (pemetrexed for injection), alinia (nitazoxanide), allegra (fexofenadine hydrochloride), allegra-d, alora, aloxi (palonosetron), alphagan (brimonidine), alphanine sd coagulation factor ix (human), alrex, altabax (retapamulin), altocor (lovastatin) extended-release tablets, alvesco (ciclesonide), amaryl (glimepiride), amerge, amevive (alefacept), amitiza (lubiprostone), amoxil (amoxicillin), ampyra (dalfampridine), amrix (cyclobenzaprine hydrochloride extended release), amturnide (aliskiren + amlodipine + hydrochlorothiazide), androderm (testosterone transdermal system), androgel testosterone gel, aneuvysion assay, anexsia, angiomax (bivalirudin), antizol injection, anturol (oxybutynin) gel, anzemet, anzemet, aphthasol, aplenzin (bupropion hydrobromide), apokyn (apomorphine hydrochloride), apthasol (amlexanox), aptivus (tipranavir), aptivus (tipranavir), arava, arcapta (indacaterol maleate inhalation powder), aredia (pamidronate disodium for injection), arestin (minocycline hydrochloride), argatroban injection, aricept (donepezil hydrochloride), arimidex (anastrozole), arixtra, aromasin tablets, arranon (nelarabine), arthrotec, arzerra (ofatumumab), asacol (mesalamine), astelin nasal spray, astepro (azelastine hydrochloride nasal spray), atacand (candesartan cilexetil), atacand (candesartan cilexetil), atacand (candesartan cilexetil), atracurium besylate injection, atridox, atridox, atrovent (ipratropium bromide), atryn (antithrombin recombinant lyophilized powder for reconstitution), aubagio (teriflunomide), augmentin (amoxicillin/clavulanate), avandamet (rosiglitazone maleate and metformin hcl), avandia (rosiglitazone maleate), avastin (bevacizumab), avastin (bevacizumab), avelox i.v. (moxifloxacin hydrochloride), avinza (morphine sulfate), avita gel, avita gel, avonex (interferon beta 1-a), axert (almotriptan malate) tablets, axid ar (nizatidine, axona (caprylidene), azasite (azithromycin), azmacort (triamcinolone acetonide) inhalation aerosol, azor (amlodipine besylate; olmesartan medoxomil), azulfidine en-tabs tablets (sulfasalazine delayed release tablets, usp), bactroban cream, bactroban nasal 2% (mupirocin calcium ointment), banzel (rufinamide), baraclude (entecavir), baycol (cerivastatin sodium), bayer extra strength asprin, belviq (lorcaserin hydrochloride), benefix (coagulation factor ix (recombinant)), benefix (coagulation factor ix (recombinant)), benicar, benlysta (belimumab), benzamycin (erythromycin 3%-benzoyl peroxide 5% topical gel), bepreve (bepotastine besilate ophthalmic solution), berinert (c1 esterase inhibitor (human)), besivance (besifloxacin ophthalmic suspension), betaxon, bextra, bexxar, biaxin xl (clarithromycin extended-release tablets), bidil (isosorbide dinitrate/hydralazine hydrochloride), bio-t-gel (testosterone gel), boniva (ibandronate), bosulif (bosutinib), botox (onabotulinumtoxina), botox (onabotulinumtoxina), botox cosmetic (botulinum toxin type a), bravelle (urofollitropin for injection, purified), breathe right, brilinta (ticagrelor), bromfenac, brovana (arformoterol tartrate), bss sterile irrigating solution, busulflex, butrans (buprenorphine) transdermal system, byetta (exenatide), caduet (amlodipine/atorvastatin), cafcit injection, cambia (diclofenac potassium for oral solution), campath, campostar, campral (acamprosate calcium), camptosar, canasa (mesalamine), cancidas, captopril and hydrochlorotiazide, captopril and hydrochlorotiazide, carbaglu (carglumic acid), carbatrol, cardizem (r) (diltiazem hc1 for injection) monvial (r), carrington patch, caverject (alprostadil), cayston (aztreonam for inhalation solution), cea-scan, cedax (ceftibuten), cefazolin and dextrose usp, ceftin (cefuroxime axetil), celexa, cellcept, cenestin, cenestin, cernevit, cervarix [human papillomavirus bivalent (types 16 and 18) vaccine, recombinant, cetrotide, chantix (varenicline), children's advil (pediatric ibuprofen), children's motrin cold, chloraprep (chlorhexidine gluconate), cialis (tadalafil), cimetadine hydrochloride oral solution 300mg/5ml, cimetidine hydrochloride oral solution, cimetidine hydrochloride oral solution, cimzia (certolizumab pegol), cimzia (certolizumab pegol), cinryze (c1 inhibitor (human)), cipro (ciprofloxacin hcl), cipro (ciprofloxacin hcl), cipro (ciprofloxacin) i.v. and cipro (ciprofloxacin hci) tablets, clarinex, clarithromycin (biaxin), claritin reditabs (10 mg loratadine rapidly-disintegrating tablet), claritin syrup (loratadine), claritin-d 24 hour extended release tablets (10 mg loratadine, 240 mg pseudoephedrine sulfate), clemastine fumarate syrup, cleocin (clindamycin phosphate), cleocin (clindamycin phosphate), cleviprex (clevidipine), climara, clindamycin phosphate topical gel, clindamycin phosphate topical solution usp 1%, clolar (clofarabine), clomipramine hydrochloride, clonazepam, coartem (artemether/lumefantrine), colazal (balsalazide disodium), colcrys (colchicine), combivir, complera (emtricitabine/rilpivirine/tenofovir disoproxil fumarate), comtan, concerta, condylox gel 0.5% (pokofilox), confide, copaxone, corlopam, corvert injection (ibutilide fumarate injection), cosopt, covera-hs (verapamil), crestor (rosuvastatin calcium), crinone 8% (progesterone gel), crixivan (indinavir sulfate), curosurf, cuvposa (glycopyrrolate), cycloset, bromocriptine mesylate, cylert, cymbalta (duloxetine), cystaran (cysteamine hydrochloride), dacogen (decitabine), daliresp (roflumilast), daptacel, degarelix (degarelix for injection), dentipatch (lidocaine transoral delivery system), depakote (divalproex sodium), depakote (divalproex sodium), depakote er (divalproex sodium), dermagraft-tc, desmopressin acetate (ddavp), desmopressin acetate (ddavp), desonate (desonide), detrol (tolterodine tartrate), detrol la (tolterodine tartrate), differin (adapalene gel) gel, 0.1%, dificid (fidaxomicin), diltiazem hcl, extended-release capsules, diovan (valsartan), diovan (valsartan), diovan hct (valsartan), ditropan xl (oxybutynin chloride), ditropan xl (oxybutynin chloride), doribax (doripenem), dostinex tablets (cabergoline tablets), doxil (doxorubicin hcl liposome injection), droxia, duexis (ibuprofen and famotidine), dulera (mometasone furoate + formoterol fumarate dihydrate), duoneb (albuterol sulfate and ipratropium bromide), durezol (difluprednate), dutasteride, dymista (azelastine hydrochloride and fluticasone propionate), dynabac, dynacirc cr, edarbi (azilsartan medoxomil), edarbyclor (azilsartan medoxomil and chlorthalidone), edex, edluar (zolpidem tartrate), edurant (rilpivirine), effexor (venlafaxin hcl), effexor xr (venlafaxin hci), efient (prasugrel), egrifta (tesamorelin for injection), elaprase (idursulfase), elelyso (taliglucerase alfa), elestrin (estradiol gel), elidel, eligard (leuprolide acetate), elitek (rasburicase), ella (ulipristal acetate), ellence, elliotts b solution (buffered intrathecal electrolyte/dextrose injection), elmiron (pentosan polysulfate sodium), eloxatin (oxaliplatin/5-fluorouracil/leucovorin), embeda (morphine sulfate and naltrexone hydrochloride), emend (aprepitant), enbrel (etanercept), entereg (alvimopan), entocort ec (budesonide), epivir (lamivudine), epivir (lamivudine), eraxis (anidulafungin), erbitux (cetuximab), erivedge (vismodegib), erwinaze (asparaginase erwinia chrysanthemi), esclim, estradiol tablets, estradiol tablets, estradiol transdermal system, estratab (.3 mg), estrogel (estradiol gel 0.06%), estrostep (norethindrone acetate and ethinyl estradiol), estrostep (norethindrone acetate and ethinyl estradiol), estrostep (norethindrone acetate and ethinyl estradiol), ethyol (amifostine), ethyol (amifostine), etodolac, etodolac, etodolac, eulexin (flutamide), evamist (estradiol), evista (raloxifene hydrochloride), evista (raloxifene hydrochloride), evista (raloxifene hydrochloride), evoxac, exalgo (hydromorphone hydrochloride) extended release, excedrin migraine, exelon (rivastigmine tartrate), exelon (rivastigmine tartrate), exparel (bupivacaine liposome injectable suspension), extavia (interferon beta-Ib), extina (ketoconazole), eylea (aflibercept), fabrazyme (agalsidase beta), famvir (famciclovir), famvir (famciclovir), fanapt (iloperidone), faslodex (fulvestrant), femara (letrozole), femhrt tablets, fempatch, femstat 3 (butoconazole nitrate 2%), femstat one, fenofibrate, feraheme (ferumoxytol), feridex i.v., ferriprox (deferiprone), ferrlecit, fertinex (urofollitropin for injection, purified), finacea (azelaic acid) gel, 15%, finevin, firazyr (icatibant), flagyl er, flomax, flonase nasal spray, flovent rotadisk, floxin otic, floxin tablets (ofloxacin tablets), flumist (influenza virus vaccine), fluzone preservative-free, focalin (dexmethylphenidate hcl), follistim (tm) (follitropin beta for injection), folotyn (pralatrexate injection), foradil aerolizer (formoterol fumarate inhalation powder), forteo (teriparatide), fortesta (testosterone gel), fortovase, fosamax (alendronate sodium), fosrenol, lanthanum carbonate, fragmin, frova (frovatriptan succinate), fusilev (levoleucovorin), fuzeon (enfuvirtide), galzin (zinc acetate), gardasil (quadrivalent human papillomavirus (types 6, 11, 16, 18) recombinant vaccine), gastrocrom oral concentrate (cromolyn sodium), gastromark, gelnique (oxybutynin chloride), gemzar (gemcitabine hcl), gemzar (gemcitabine hcl), generic transdermal nicotine patch, genotropin (somatropin) injection, genotropin (somatropin) lyophilized powder, geodon (ziprasidone mesylate), geref (sermorelin acetate for injection), gilenya (fingolimod), gleevec (imatinib mesylate), gleevec (imatinib mesylate), gliadel wafer (polifeprosan 20 with carmustine implant), glipizide tablets, glucagon, glucagon, glyburide tablets, glyburide tablets, glyburide tablets, glyset (miglitol), gonal-f (follitropin alfa for injection), gralise (gabapentin), halaven (eribulin mesylate), havrix, hectorol (doxercalciferol) injection, hepsera (adefovir dipivoxil), herceptin, herceptin (trastuzumab), hiberix (haemophilus b conjugate vaccine; tetanus toxoid conjugate), horizant (gabapentin enacarbil), horizant (gabapentin enacarbil), humalog (insulin lispro), humatrope (somatropin [rdna origin] for injection), humira (adalimumab), hycamtin (topotecan hydrochloride), hycamtin (topotecan hydrochloride), iamin, ilaris (canakinumab), imagent (perflexane lipid microspheres), imitrex (sumatriptan) injection and tablets, imitrex (sumatriptan) nasal spray, incivek (telaprevir), increlex (mecasermin), infanrix (diphtheria and tetanus toxoids and acellular pertussis vaccine adsorbed), infasurf, infergen (interferon alfacon-1), inform her-2/neu breast cancer test, inlyta (axitinib), innohep (tinzaparin sodium) injectable, inspra (eplerenone tablets), integrilin, intelence (etravirine), intermezzo (zolpidem tartrate sublingual tablet), interstim continence control therapy, intron a (interferon alfa-2b, recombinant), intron a (interferon alfa-2b, recombinant), intron a (interferon alfa-2b, recombinant), intuniv (guanfacine extended-release), invanz, invega (paliperidone), invirase (saquinavir), iontocaine, iressa (gefitinib), isentress (raltegravir), istodax (romidepsin), ivyblock, ixempra (ixabepilone), ixiaro (japanese encephalitis vaccine, inactivated, adsorbed), jakafi (ruxolitinib), jalyn (dutasteride + tamsulosin), januvia(sitagliptin phosphate), jentadueto (linagliptin plus metformin hydrochloride), jevtana (cabazitaxel), juvisync (sitagliptin and simvastatin), kadian, kalbitor (ecallantide), kaletra capsules and oral solution, kalydeco (ivacaftor), kapvay (clonidine hydrochloride), keppra, ketek (telithromycin), ketoprofen, kineret, klaron (sodium sulfacet amide lotion) lotion, 10%, kogenate fs (antihemophilic factor recombinant), korlym (mifepristone), krystexxa (pegloticase), kuvan (sapropterin dihydrochloride), kyprolis (carfilzomib), kytril (granisetron) solution, kytril (granisetron) tablets, lamictal (lamotrigine) chewable dispersible tablets, lamictal chewable dispersible tablets, lamisil (terbinafine hydrochloride) dermagel, 1%, lamisil (terbinafine hydrochloride) solution, 1%, lamisil (terbinafine hydrochloride) tablets, lamisil solution, 1%, lantus (insulin glargine [rdna origin] injection), lantus (insulin glargine [rdna origin] injection), latuda (lurasidone), laviv (azficel-t), lazanda (fentanyl citrate) nasal spray, lescol (fluvastatin sodium), lescol (fluvastatin sodium) capsules, rx, lescol xl (fluvastatin sodium) tablet, extended release, letairis (ambrisentan), leukine (sargramostim), leukine (sargramostim), levaquin, levitra (vardenafil), levo-t (levothyroxine sodium), levoxyl, lexapro (escitalopram oxalate), lexiva (fosamprenavir calcium), lexxel (enalapril maleate-felodipine er), lidoderm patch (lidocaine patch 5% ), linzess (linaclotide), lipitor (atorvastatin calcium), lithobid (lithium carbonate), livalo (pitavastatin), Iodine (etodolac), Iodine xl (etodolac), Iodine xl (etodolac), lotemax, lotrisone (clotrimazole/betamethasone diproprionate) lotion, lotronex (alosetron hcl) tablets, lovenox (enoxaparin sodium) injection, lovenox (enoxaparin sodium) injection, lucentis (ranibizumab injection), lucentis (ranibizumab), lumigan (bimatoprost ophthalmic solution), lunesta (eszopiclone), lupron depot (leuprolide acetate for depot suspension), lupron depot (leuprolide acetate for depot suspension), lusedra (fospropofol disodium), lustra, luvox (fluvoxamine maleate), luxiq (betamethasone valerate) foam, lyrica (pregabalin), lyrica (pregabalin), lysteda (tranexamic acid), macugen (pegaptanib), malarone (atovaquone; proguanil hydrochloride) tablet, malarone (atovaquone; proguanil hydrochloride) tablet, marplan tablets, marqibo (vincristine sulfate liposome injection), mavik (trandolapril), maxalt, mentax (1% butenafine hci cream), mentax (1% butenafine hcl cream), mentax (1% butenafine hcl cream), menveo (meningitis vaccine), meridia, merrem i.v. (meropenem), mesnex, metadate cd, metaglip (glipizide/metformin hcl), metaprotereol sulfate inhalation solution, 5%, metozolv odt (metoclopramide hydrochloride), metrolotion, mevacor (lovastatin) tablets, miacalcin (calcitonin-salmon) nasal spray, micardis (telmisartan), micardis hct (telmisartan and hydrochlorothiazide), microzide (hydrochlorothiazide), migranal, minoxidil topical solution 2% for women, miraluma test, mirapex, mircera (methoxy polyethylene glycol-epoetin beta), mircette, mirena (levonorgestrel-releasing intrauterine system), mobic (meloxicam) tablets, monistat 3 (miconazole nitrate), monistat 3 (miconazole nitrate), monurol, moxatag (amoxicillin), mozobil (plerixafor injection), multaq (dronedarone), muse, mylotarg (gemtuzumab ozogamicin), myobloc, myozyme (alglucosidase alfa), myrbetriq (mirabegron), naglazyme (galsulfase), naltrexone hydrochloride tablets, namenda (memantine hcl), naprelan (naproxen sodium), nasacort aq (triamcinolone acetonide) nasal spray, nasacort aq (triamcinolone acetonide) nasal spray, nasalcrom nasal spray, nascobal gel (cyanocobalamin, usp), nasonex nasal spray, natazia (estradiol valerate + dienogest), natazia (estradiol valerate and estradiol valerate/dienogest), natrecor (nesiritide), neulasta, neumega, neupogen, neupro (rotigotine transdermal system), neupro (rotigotine), neurontin (gabapentin), neurontin (gabapentin) oral solution, neurontin (gabapentin) oral solution, neutroval (tbo-filgrastim), nexavar (sorafenib), nexium (esomeprazole magnesium), niaspan, nicoderm cq, nicorette (nicotine polacrilex), nicotrol nasal spray, nicotrol transdermal patch, nitrostat (nitroglycerin) tablets, nolvadex, norco tablets (hydrocodone bitartrate/acetaminophen 10 mg/325 mg), norditropin (somatropin (rdna origin) for injection), noritate, normiflo, norvir (ritonavir), norvir (ritonavir), novantrone (mitoxantrone hydrochloride), novolog (insulin aspart), novolog mix 70/30, novothyrox (levothyroxine sodium), noxafil (posaconazole), nplate (romiplostim), nucynta (tapentadol), nuedexta (dextromethorphan hydrobromide and quinidine sulfate), nulojix (belatacept), nutropin (somatropin-rdna origin), nutropin (somatropin-rdna origin), nuvaring, nuvigil (armodafinil), ocuflox (ofloxacin opthalmic solution) 0.3%, ocuhist, oleptro (trazodone hydrochloride), omnicef, omontys (peginesatide), onfi (clobazam), onglyza (saxagliptin), onsolis (fentanyl buccal), oral cytovene, oravig (miconazole), orencia (abatacept), orencia (abatacept), orfadin (nitisinone), ortho evra, ortho tri-cyclen tablets (norgestimate/ethinyl estradiol), ortho-prefest, osmocyte pillow wound dressing, ovidrel (gonadotropin, chorionic human recombinant), oxecta (oxycodone hcl), oxycodone and aspirin, oxycodone with acetaminophen 5mg/325mg, oxycontin (oxycodone hcl controlled-release), oxytrol (oxybutynin transdermal system), ozurdex (dexamethasone), pancreaze (pancrelipase), panretin gel, patanase (olopatadine hydrochloride), paxil (paroxetine hydrochloride), paxil cr (paroxetine hydrochloride), paxil cr (paroxetine hydrochloride), pediarix vaccine, peg-intron (peginterferon alfa-2b), pegasys (peginterferon alfa-2a), pennsaid (diclofenac sodium topical solution), pentoxifylline, pepcid complete, periostat (doxycycline hyclate), periostat (doxycycline hyclate), perjeta (pertuzumab), phoslo, photodynamic therapy, photofrin, picato (ingenol mebutate) gel, pindolol, plavix (clopidogrel bisulfate), plavix (clopidogrel bisulfate), plenaxis (abarelix for injectable suspension), posicor, potiga (ezogabine), pradaxa (dabigatran etexilate mesylate), pramipexole, prandin, pravachol (pravastatin sodium), pravachol (pravastatin sodium), precose (acarbose), premarin (conjugated estrogens), prempro, prempro & premphase (conjugated estrogens/medroxyprogesterone acetate tablets), prevacid(r) (lansopraxole), preven ; emergency contraceptive kit, prevnar 13 (pneumococcal 13-valent conjugate vaccine), prevpac, prevpac, prezista (darunavir), priftin, prilosec (omeprazole), prilosec (omeprazole), prilosec (omeprazole), prilosec (omeprazole)/biaxin (clarithromycin) combination therapy, prinivil or zestril (lisinopril), proamatine (midodrine), procanbid (procainamide hydrochloride extended-release tablets), prochloroperazine, prochlorperazine, prograf, proleukin, prolia (denosumab), promacta (eltrombopag), prometrium, prometrium, propecia, proscar, protonix (pantoprazole sodium) delayed release tablets, protonix (pantoprazole sodium) delayed-release tablets, protonix (pantoprazole sodium) intravenous formulation, protopic (tacrolimus) ointment, provenge (sipuleucel-t), proventil hfa inhalation aerosol, prozac weekly (fluoxetine hcl), pulmozyme (dornase alfa), pulmozyme (dornase alfa), qnasl (beclomethasone dipropionate) nasal aerosol, qsymia (phentermine + topiramate extended-release), quadramet (samarium sm 153 lexidronam injection), quillivant xr (methylphenidate hydrochloride), quixin (levofloxacin), qutenza (capsaicin), qvar (beclomethasone dipropionate), ranexa (ranolazine), ranitidine capsules, ranitidine tablets, rapamune (sirolimus) oral solution, rapamune (sirolimus) tablets, raplon, raxar (grepafloxacin), rayos (prednisone) delayed-release tablets, rebetol (ribavirin), rebetron (tm) combination therapy, rebif (interferon beta-1a), reclast (zoledronic acid), reclast (zoledronic acid), rectiv (nitroglycerin) ointment 0.4%, redux (dexfenfluramine hydrochloride), refludan, regranex (becaplermin) gel, relenza, relpax (eletriptan hydrobromide), remeron (mirtazapine), remeron soltab (mirtazapine), remicade (infliximab), remicade (infliximab), reminyl (galantamine hydrobromide), remodulin (treprostinil), renagel (sevelamer hydrochloride), renagel (sevelamer hydrochloride), renagelrenagel (sevelamer hydrochloride), renova (tretinoin emollient cream), renvela (sevelamer carbonate), reopro, repronex(menotropins for injection, usp), requip (ropinirole hydrochloride), rescriptor tablets (delavirdine mesylate tablets), rescula (unoprostone isopropyl ophthalmic solution) 0.15%, respigam (respiratory syncitial virus immune globulin intravenous), restasis (cyclosporine ophthalmic emulsion), retavase (reteplase), retin-a micro (tretinoin gel) microsphere, 0.1%, revlimid (lenalidomide), reyataz (atazanavir sulfate), rhinocort aqua nasal spray, rid mousse, rilutek (riluzole), risperdal oral formulation, ritalin la (methylphenidate hcl), rituxan, rocephin, rocephin, rotarix (rotavirus vaccine, live, oral), rotateq (rotavirus vaccine, live oral pentavalent), rozerem (ramelteon), rythmol, sabril (vigabatrin), saizen, salagen tablets, samsca (tolvaptan), sanctura (trospium chloride), sancuso (granisetron), saphris (asenapine), savella (milnacipran hydrochloride), sclerosol intrapleural aerosol, seasonale, lo seasonale, seasonique (ethinylestradiol + levonorgestrel), secreflo (secretin), selegiline tablets, self-examination breast pad, selzentry (maraviroc), sensipar (cinacalcet), seprafilm, serevent, seroquel (r) (quetiapine fumarate) tablets, silenor (doxepin), simponi (golimumab), simulect, singulair, skelid (tiludronate disodium), skin exposure reduction paste against chemical warfare agents (serpacwa), sklice (ivermectin) lotion, soliris (eculizumab), soliris (eculizumab), somatuline depot (lanreotide acetate), somavert (pegvisomant), sonata, spectracef, spiriva handihaler (tiotropium bromide), sporanox (itraconazole), sprix (ketorolac tromethamine), sprycel (dasatinib), stavzor (valproic acid delayed release), stelara (ustekinumab), stendra (avanafil), stendra (avanafil), stivarga (regorafenib), strattera (atomoxetine hcl), stribild (elvitegravir, cobicistat, emtricitabine, tenofovir disoproxil fumarate), stromectol (ivermectin), subsys (fentanyl sublingual spray), subutex/suboxone (buprenorphine/naloxone), sulfamylon, supartz, supprelin la (histrelin acetate), surfaxin (lucinactant), sustiva, sutent (sunitinib malate), sutent (sunitinib), sylatron (peginterferon alfa-2b), symlin (pramlintide), synagis, synercid i.v., synthroid (levothyroxine sodium), synvisc, synvisc-one (hylan gf 20), tamiflu capsule, tarceva (erlotinib, osi 774), tasigna (nilotinib hydrochloride monohydrate), tasmar, tavist (clemastine fumarate), tavist (clemastine fumarate), taxol, taxotere (docetaxel), tazorac topical gel, teczem (enalapril maleate/diltiazem malate), teflaro (ceftaroline fosamil), tegretol (carbamazepine), tegretol xr (carbamazepine), tekamlo (aliskiren + amlodipine), tekturna (aliskiren), temodar, tequin, testim, testoderm tts ciii, teveten (eprosartan mesylate plus hydrochlorothiazide), teveten (eprosartan mesylate), thalomid, tiazac (diltiazem hydrochloride), tiazac (diltiazem hydrochloride), tiazac (diltiazem hydrochloride), tikosyn capsules, tilade (nedocromil sodium), tilade (nedocromil sodium), tilade (nedocromil sodium), timentin, timentin, tindamax, tinidazole, tobi, tolmetin sodium, topamax (topiramate), topamax (topiramate), toprol-xl (metoprolol succinate), torisel (temsirolimus), toviaz (fesoterodine fumarate), tracleer (bosentan), tradjenta (linagliptin), travatan (travoprost ophthalmic solution), trazadone 150mg, treanda (bendamustine hydrochloride), trelstar depot (triptorelin pamoate), trelstar la (triptorelin pamoate), tri-nasal spray (triamcinolone acetonide spray), tribenzor (olmesartan medoxomil + amlodipine + hydrochlorothiazide), tricor (fenofibrate), tricor (fenofibrate), trileptal (oxcarbazepine) tablets, trilipix (fenofibric acid), tripedia (diptheria and tetanus toxoids and acellular pertussis vaccine absorbed), trisenox (arsenic trioxide), trivagizole 3 (clotrimazole) vaginal cream, trivora-21 and trivora-28, trizivir (abacavir sulfate; lamivudine; zidovudine azt) tablet, trovan, tudorza pressair (aclidinium bromide inhalation powder), twinrix, tygacil (tigecycline), tykerb (lapatinib), tysabri (natalizumab), tysabri (natalizumab), tyvaso (treprostinil), tyzeka (telbivudine), uloric (febuxostat), ultracet (acetaminophen and tramadol hcl), ultraject, ultresa (pancrelipase) delayed-release capsules, uroxatral (alfuzosin hcl extended-release tablets), urso, uvadex sterile solution, valcyte (valganciclovir hcl), valstar, valtrex (valacyclovir hcl), vancenase aq 84 mcg double strength, vanceril 84 mcg double strength (beclomethasone dipropionate, 84 mcg) inhalation aerosol, vandetanib (vandetanib), vaprisol (conivaptan), vascepa (icosapent ethyl), vectibix (panitumumab), velcade (bortezomib), veltin (clindamycin phosphate and tretinoin), venofer (iron sucrose injection), ventolin hfa (albuterol sulfate inhalation aerosol), veramyst (fluticasone furoate), verapamil, verdeso (desonide), veregen (kunecatechins), versed (midazolam hci), vesicare (solifenacin succinate), vfend (voriconazole), viadur (leuprolide acetate implant), viagra, vibativ (telavancin), victoza (liraglutide), victrelis (boceprevir), vidaza (azacitidine), videx (didanosine), viibryd (vilazodone hydrochloride), vimovo (naproxen + esomeprazole), vimpat (lacosamide), viokace (pancrelipase) tablets, vioxx (rofecoxib), viracept (nelfinavir mesylate), viramune (nevirapine), viread (tenofovir disoproxil fumarate), viread (tenofovir disoproxil fumarate), viroptic, visicol tablet, visipaque (iodixanol), vistide (cidofovir), vistide (cidofovir), visudyne (verteporfin for injection), vitrasert implant, vitravene injection, vivelle (estradiol transdermal system), vivelle (estradiol transdermal system), vivelle-dot (estradiol transdermal system), vivitrol (naltrexone for extended-release injectable suspension), vivitrol (naltrexone for extended-release injectable suspension), voraxaze (glucarpidase), votrient (pazopanib), votrient (pazopanib), vpriv (velaglucerase alfa for injection), vyvanse (lisdexamfetamine dimesylate), warfarin sodium tablets, welchol (colesevelam hydrochloride), western blot confirmatory device, wilate (von willebrand factor/coagulation factor viii complex (human), xalkori (crizotinib), xarelto (rivaroxaban), xarelto (rivaroxaban), xeloda, xeloda, xenazine (tetrabenazine), xenical/orlistat capsules, xeomin (incobotulinumtoxina), xgeva (denosumab), xiaflex (collagenase clostridium histolyticum), xifaxan (rifaximin), xifaxan (rifaximin), xigris (drotrecogin alfa [activated]), xolair (omalizumab), xopenex, xtandi (enzalutamide), xyrem (sodium oxybate), xyzal (levocetirizine dihydrochloride), yasmin (drospirenone/ethinyl estradiol), yervoy (ipilimumab), zaditor, zagam (sparfloxacin) tablets, zaltrap (ziv-aflibercept), zanaflex (tizanidine hydrochloride), zantac 75 efferdose, zelboraf (vemurafenib), zelnorm (tegaserod maleate) tablets, zelnorm (tegaserod maleate) tablets, zemaira (alpha1-proteinase inhibitor), zemplar, zenapax, zenpep (pancrelipase), zerit (stavudine), zerit (stavudine), zevalin (ibritumomab tiuxetan), zingo (lidocaine hydrochloride monohydrate), zioptan (tafluprost ophthalmic solution), ziprasidone (ziprasidone hydrochloride), zipsor (diclofenac potassium), zirgan (ganciclovir ophthalmic gel), zithromax (azithromycin), zocor, zofran, zofran, zoladex (10.8 mg goserelin acetate implant), zoloft (sertraline hcl), zoloft (sertraline hcl), zoloft (sertraline hcl), zometa (zoledronic acid), zometa (zoledronic acid), zomig (zolmitriptan), zomig (zolmitriptan), zonegran (zonisamide) capsules, zortress (everolimus), zosyn (sterile piperacillin sodium/tazobactam sodium), zuplenz (ondansetron oral soluble film), zyban sustained-release tablets, zyclara (imiquimod), zyflo (zileuton), zymaxid (gatifloxacin ophthalmic solution), zyprexa, zyrtec (cetirizine hcl), and zytiga (abiraterone acetate).

### Administration

Co-administration of a therapeutic agent (e.g., one or more inhibitors of tyrosine kinase) and the compositions disclosed herein comprising one or more efflux inhibitors (e.g., inhibitors of BCRP and/or P-GP) can be simultaneous or sequential.

In some embodiments, the therapeutic agent (e.g., one or more inhibitors of tyrosine kinase) and the efflux inhibitor composition are administered to a mammalian (e.g., human) subject simultaneously. Administration of therapeutic agent (e.g., one or more inhibitors of tyrosine kinase) and the efflux inhibitor composition can be by simultaneous administration of a single formulation (*e.g.,* a formulation comprising one or more inhibitors of tyrosine kinase and one or more inhibitors of BCRP and/or P-GP) or of separate formulations (*e.g.,* a first formulation including one or more inhibitors of tyrosine kinase and a second formulation including one or more inhibitors of BCRP and/or P-GP).

Co-administration does not require the therapeutic agents to be administered simultaneously, if the timing of their administration is such that the pharmacological activities of therapeutic agent and the efflux inhibitor composition overlap in time, thereby exerting a combined therapeutic effect. For example, the therapeutic agent and the efflux inhibitor composition can be administered sequentially. The term "sequentially" as used herein means that the therapeutic agent and the efflux inhibitor composition are administered with a time separation of more than about 60 minutes. For example, the time between the sequential administration of the one or more inhibitors of tyrosine kinase and the one or more inhibitors of BCRP and/or P-GP can be more than 60 minutes, more than 2 hours, more than 5 hours, more than 10 hours, more than 1 day, more than 2 days, more than 3 days, or more than 1 week apart. The optimal administration times will depend on the rates of absorption, distribution, metabolism and/or excretion of the therapeutic agent and the efflux inhibitor composition being administered.

Either the therapeutic agent or the efflux inhibitor composition can be administered first. For example, the therapeutic agent can be administered to a mammalian (e.g., human) subject after the time at which the efflux inhibitor is administered. In this case, it can be desirable to administer the therapeutic prior to the time at which about 50% (*e.g.,* prior to the time at which about 40%, about 30%, about 20%, about 10%, or about 5%) of the inhibitor of BCRP and/or P-GP is metabolized or excreted by the mammalian (e.g., human) subject. In another example, a first dose of one or more efflux inhibitor is administered to the human subject, followed by administration of a single dose of the therapeutic agent, which is then followed by an additional dose of the one or more efflux inhibitors.

In accordance with certain embodiments of the invention, the one or more inhibitors of tyrosine kinase and the one or more inhibitors of BCRP and/or P-GP may each be administered, for example, more than once daily, about once per day, about every other day, about every third day, or about once a week.

Co-administration also does not require the therapeutic agent(s) and the efflux inhibitor (e.g. one or more inhibitors inhibitors of BCRP and/or P-GP) to be administered to the mammalian (e.g., human) subject by the same route of administration. Rather, each therapeutic agent can be administered by any appropriate route, for example, parenterally or non-parenterally. In an embodiment, the therapeutic agent(s) may be administered orally to the human subject. In another embodiment, the therapeutic agent(s) may be administered parenterally, including for example, intravenous and intra-arterial, among others. In a further embodiment, the therapeutic agent(s) may be administered topically. In yet another embodiment, the therapeutic agent(s) may be administered to the patient via intrathecal infusion. Alternatively, the therapeutic agent(s) and or the the efflux inhibitor (e.g. one or more inhibitors inhibitors of BCRP and/or P-GP) may be administered via alternative routes of administration to reduce first pass metabolism, and/or excretion, and/or to facilitate more effective drug delivery to the target tissues including but not limited to the transmucosal routes. (e.g., rectal, vaginal, sublingual, buccal, inhalation), systemic (IV, IM, SC, IP), topical (transdermal, ocular, and/or otic)

### Dosage Forms

The therapeutic agents (e.g., one or more inhibitors of tyrosine kinase) and the one or more efflux inhibitors (e.g., inhibitors of BCRP and/or P-GP) are administered to the mammalian (e.g., human) subject under conditions effective to deliver the inhibitors to the subject's brain or peripheral nervous system, including nerve endings ending in the skin. As one skilled in the art will recognize, the efflux inhibitor formulations may be made up, together or separately, in any suitable form appropriate for the desired use and route of administration. Examples of suitable dosage forms include, for example, oral, parenteral, and topical dosage forms.

Suitable dosage forms for oral use include, for example, tablets, dispersible powders, granules, capsules, suspensions, and syrups. Inert diluents and carriers for tablets include, for example, calcium carbonate, sodium carbonate, lactose, and talc. Tablets may also contain granulating and disintegrating agents, such as starch and alginic acid; binding agents, such as starch, gelatin, and acacia; and lubricating agents, such as magnesium stearate, stearic acid, and talc. Tablets may be uncoated or may be coated by known techniques to delay disintegration and absorption. Inert diluents and carriers, which may be used in capsules include, for example, calcium carbonate, calcium phosphate, and kaolin. Suspensions and syrups may contain conventional excipients, for example, methyl cellulose, tragacanth, sodium alginate; wetting agents, such as lecithin and polyoxyethylene stearate; and preservatives (including antioxidants), such as ethyl-p-hydroxybenzoate.

Dosage forms suitable for parenteral administration include, for example, solutions, suspensions, dispersions, emulsions, and the like. They may also be manufactured in the form of sterile solid compositions, which can be dissolved or suspended in sterile injectable medium immediately before use. They may contain suspending or dispersing agents known in the art.

Dosage forms for topical or transdermal administration include, for example, ointments, pastes, creams, lotions, gels, powders, solutions, sprays, inhalants, or patches. For example, the present invention contemplates the use of transdermal patches, which have the advantage of providing controlled delivery to skin lesions. Such dosage forms can be made by dissolving or dispensing the therapeutic agent(s) in the proper medium. Permeation enhancers can also be used to increase the flux of the efflux inhibitors and/or therapeutic agent(s) across the skin. In another embodiment, the present invention contemplates the use of eye drops. The rate can be controlled by either providing a rate controlling membrane or by dispersing the therapeutic agent(s) in a polymeric matrix or gel.

It is contemplated that each of the therapeutic agents may be administered *per se* as well as in various forms including pharmaceutically acceptable esters, salts, and other physiologically functional derivatives thereof. It is further contemplated that the therapeutic agents may be formulated solely, or together with other therapeutic agents. For example, the therapeutic agent and the efflux inhibitor may be part of a single formulation. Further, the formulations may include additional therapeutic agents, particularly, agents which have been identified as useful in the prevention, treatment and/or alleviation of neurological conditions.

The formulations comprising the inhibitors of the present invention may conveniently be presented in unit dosage forms and may be prepared by any of the methods well known in the art of pharmacy. Such methods generally include the step of bringing the therapeutic agents into association with a carrier, which constitutes one or more accessory ingredients. Typically, the formulations are prepared by uniformly and intimately bringing the therapeutic agent into association with a liquid carrier, a finely divided solid carrier, or both, and then, if necessary, shaping the product into dosage forms of the desired formulation.

It will be appreciated that the actual dose of the therapeutic agent (e.g., one or more inhibitors of tyrosine kinase) and the efflux inhibitor (e.g., one or more inhibitors of BCRP and/or P-GP) to be administered according to the present invention will vary according to the particular compound, the particular dosage form, and the mode of administration. Many factors that may modify the action of the one or more inhibitors of tyrosine kinase and the one or more inhibitors of BCRP and/or P-GP (*e.g.,* body weight, gender, diet, time of administration, route of administration, rate of excretion, condition of the subject, drug combinations, and reaction sensitivities and severities) can be taken into account by those skilled in the art. Administration can be carried out continuously or in one or more discrete doses within the maximum tolerated dose. Optimal administration rates for a given set of conditions can be ascertained by those skilled in the art using conventional dosage administration tests.

For example, a suitable dosage amount of the therapeutic agent (e.g., inhibitor of tyrosine kinase) is in a range of about 0.1 mg/kg to about 250 mg/kg of body weight of the mammalian (e.g., human) subject, for example, about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1 mg/kg, about 1.1 mg/kg, about 1.2 mg/kg, about 1.3 mg/kg, about 1.4 mg/kg, about 1.5mg/kg, about 1.6 mg/kg, about 1.7 mg/kg, about 1.8 mg/kg, about 1.9 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 20 mg/kg, about 25 mg/kg, about 30 mg/kg, about 35 mg/kg, about 40 mg/kg, about 45 mg/kg, about 50 mg/kg, about 55 mg/kg, about 60 mg/kg, about 65 mg/kg, about 70 mg/kg, about 75 mg/kg, about 80 mg/kg, about 85 mg/kg, about 90 mg/kg, about 95 mg/kg, about 100 mg/kg, about 125 mg/kg, about 150 mg/kg, about 175 mg/kg, about 200 mg/kg, about 225 mg/kg, or about 250 mg/kg body weight, inclusive of all values and ranges therebetween. In other embodiments, a suitable dosage amount of the inhibitor of tyrosine kinase is in a range of about 1 mg/kg to about 250 mg/kg of body weight, in a range of about 10 mg/kg to about 250 mg/kg of body weight, in a range of about 10 mg/kg to about 100 mg/kg of body weight, in a range of 10 mg/kg to about 50 mg/kg of body weight, in a range of about 10 mg/kg to about 25 mg/kg of body weight, in a range of about 0.1 mg/kg to about 150 mg/kg of body weight, in a range of about 0.1 mg/kg to about 100 mg/kg of body weight, in a range of 0.1 mg/kg to about 75 mg/kg of body weight, in a range of 0.1 mg/kg to about 50 mg/kg of body weight, in a range of 0.1 mg/kg to about 25 mg/kg of body weight, or in a range of about 0.1 mg/kg to about 10 mg/kg of body weight, or in a range of about 0.1 mg/kg to about 5 mg/kg of body weight, or in a range of about 0.1 mg/kg to about 2 mg/kg of body weight.

The desired dose of the tyrosine kinase inhibitor may be presented as one dose or two or more sub-doses administered at appropriate intervals throughout the dosing period (e.g., one hour, one day, one week etc.). Individual doses can be administered in unit dosage forms (e.g., tablets or capsules) containing, for example, from about 1 mg to about 2,000 mg, from about 1 mg to about 1,500 mg, from about 1 mg to about 1,000 mg, from about 1 mg to about 500 mg, or from about 1 mg to about 250 mg, from about 1 mg to about 100 mg, from about 10 mg to about 1,500 mg, from about 10 mg to about 1,000 mg, from about 100 mg to about 800 mg, or from about 100 mg to about 500 mg of active ingredient per unit dosage form. For example, the individual dose can be about 1 mg, about 5 mg, about 10 mg, about 15 mg, about 20 mg, about 25 mg, about 30 mg, about 40 mg, about 50 mg, about 60 mg, about 70 mg, about 75 mg about 80 mg, about 90 mg, about 100 mg, about 125 mg, about 150 mg, about 175 mg, about 200 mg, about 225 mg, about 250 mg, about 300 mg, about 350 mg, about 400 mg, about 450 mg, about 500 mg, about 550 mg, about 600 mg, about 650 mg, about 700 mg, about 750 mg, about 800 mg, about 850 mg, about 900 mg, about 950 mg, about 1000 mg, about 1,250 mg, about 1,500 mg, about 2,000 mg inclusive of all values and ranges therebetween.

In one embodiment, the therapeutic agent (e.g., inhibitor of tyrosine kinase) is administered at an amount of from about 1 mg to about 2,000 mg daily, about 1 mg to about 1,500 mg daily, about 1 mg to about 1,000 mg daily, from about 10 mg to about 1,000 mg daily, from about 100 mg to about 800 mg daily, from about 100 mg to about 500 mg daily, or from about 100 mg to about 250 mg daily. In an exemplary embodiment, the inhibitor of tyrosine kinase is administered at an amount of about 400 mg daily. In another exemplary embodiment, the inhibitor of tyrosine kinase is administered at an amount of about 1,500 mg daily. Alternatively, if the condition of the recipient so requires, the doses may be administered as a continuous infusion.

A suitable dosage amount of the efflux inhibitor (e.g., inhibitor of BCRP and/or P-GP) is in a range of about 0.1 mg/kg to about 250 mg/kg of body weight of the mammalian (e.g., human) subject, for example, about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1 mg/kg, about 1.1 mg/kg, about 1.2 mg/kg, about 1.3 mg/kg, about 1.4 mg/kg, about 1.5mg/kg, about 1.6 mg/kg, about 1.7 mg/kg, about 1.8 mg/kg, about 1.9 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 20 mg/kg, about 25 mg/kg, about 30 mg/kg, about 35 mg/kg, about 40 mg/kg, about 45 mg/kg, about 50 mg/kg, about 55 mg/kg, about 60 mg/kg, about 65 mg/kg, about 70 mg/kg, about 75 mg/kg, about 80 mg/kg, about 85 mg/kg, about 90 mg/kg, about 95 mg/kg, about 100 mg/kg, about 125 mg/kg, about 150 mg/kg, about 175 mg/kg, about 200 mg/kg, about 225 mg/kg, or about 250 mg/kg body weight, inclusive of all values and ranges therebetween. In other embodiments, a suitable dosage amount of the inhibitor of BCRP and/or P-GP is in a range of about 1 mg/kg to about 250 mg/kg of body weight, in a range of about 10 mg/kg to about 250 mg/kg of body weight, in a range of about 10 mg/kg to about 100 mg/kg of body weight, in a range of 10 mg/kg to about 50 mg/kg of body weight, in a range of about 10 mg/kg to about 25 mg/kg of body weight, in a range of about 0.1 mg/kg to about 150 mg/kg of body weight, in a range of about 0.1 mg/kg to about 100 mg/kg of body weight, in a range of 0.1 mg/kg to about 75 mg/kg of body weight, in a range of 0.1 mg/kg to about 50 mg/kg of body weight, in a range of 0.1 mg/kg to about 25 mg/kg of body weight, or in a range of about 0.1 mg/kg to about 10 mg/kg of body weight, or in a range of about 0.1 mg/kg to about 5 mg/kg of body weight, or in a range of about 0.1 mg/kg to about 2 mg/kg of body weight.

The desired dose of the efflux inhibitor (e.g., inhibitor of BCRP and/or P-GP) may be presented as one dose or two or more sub-doses administered at appropriate intervals throughout the dosing period (e.g., one hour, one day, one week etc). Individual doses can be administered in unit dosage forms (e.g., tablets or capsules) containing, for example, from about 1 mg to about 1,500 mg, from about 1 mg to about 1,000 mg, from about 1 mg to about 500 mg, or from about 1 mg to about 250 mg, from about 1 mg to about 100 mg, from about 10 mg to about 1,000 mg, from about 100 mg to about 800 mg, or from about 100 mg to about 500 mg of active ingredient per unit dosage form. For example, the individual dose can be about 1 mg, about 5 mg, about 10 mg, about 15 mg, about 20 mg, about 25 mg, about 30 mg, about 40 mg, about 50 mg, about 60 mg, about 70 mg, about 75 mg about 80 mg, about 90 mg, about 100 mg, about 125 mg, about 150 mg, about 175 mg, about 200 mg, about 225 mg, about 250 mg, about 300 mg, about 350 mg, about 400 mg, about 450 mg, about 500 mg, about 550 mg, about 600 mg, about 650 mg, about 700 mg, about 750 mg, about 800 mg, about 850 mg, about 900 mg, about 950 mg, about 1000 mg, about 1,250 mg, or about 1,500 mg inclusive of all values and ranges therebetween.

In one embodiment, the efflux inhibitor (e.g., inhibitor of BCRP and/or P-GP) is administered at an amount of from about 1 mg to about 1,500 mg daily, about 1 mg to about 1,000 mg daily, from about 10 mg to about 1,000 mg daily, from about 100 mg to about 800 mg daily, from about 100 mg to about 500 mg daily, or from about 100 mg to about 250 mg daily. In an exemplary embodiment, the inhibitor of BCRP and/or P-GP is administered at an amount of about 200 mg daily. Alternatively, if the condition of the recipient so requires, the doses may be administered as a continuous infusion.

The term "about" as used herein with regard to doses expressed in mg refers to an amount which is ± 0.1 mg, ± 0.2 mg, ± 0.3 mg, ± 0.4 mg, ± 0.5 mg, ± 0.6 mg, ± 0.7 mg, ± 0.8 mg, ± 0.9 mg, ± 1 mg, ± 2 mg, ± 3 mg, ± 4 mg, ± 5 mg, ± 6 mg, ± 7 mg, ± 8 mg, ± 9 mg, or ± 10 mg. The term "about" as used herein with regard to normalized doses expressed as mg/kg may also refer to an amount which is ± 0.01 mg/kg, ± 0.02 mg/kg, ± 0.03 mg/kg, ± 0.04 mg/kg, ± 0.05 mg/kg, ± 0.06 mg/kg, ± 0.07 mg/kg, ± 0.08 mg/kg, ± 0.09 mg/kg, ± 0.1 mg/kg, ± 0.2 mg/kg, ± 0.3 mg/kg, ± 0.4 mg/kg, ± 0.5 mg/kg, ± 0.6 mg/kg, ± 0.7 mg/kg, ± 0.8 mg/kg, ± 0.9 mg/kg, ± 1 mg/kg, ± 2 mg/kg, ± 3 mg/kg, ± 4 mg/kg, ± 5 mg/kg, ± 6 mg/kg, ± 7 mg/kg, ± 8 mg/kg, ± 9 mg/kg, or ± 10 mg/kg. Alternatively, the term "about", unless otherwise indicated, refers to an amount ± 1 %, ± 5%, ± 10% of the stated value, or up to 50% of the interval between values.

### Efflux Inhibitor Compositions

The present invention provides compositions comprising at least one efflux inhibitor (e.g., elacridar). The compositions of the invention are formulated to provide enhanced bioavailability of the efflux inhibitor (e.g., elacridar) compared to those formulations previously known in the art.

Any formulation chemistry or technique that allows for sufficient bioavailability of the efflux inhibitor (e.g., an EC90 for SB-487946 in mice and rats of at least 300 ng/ml) can be employed in compositions of the invention. Suitable methodologies include, without limitation, nanomilling, microemulsions, nanoparticulate dispersions, amorphous solid dispersions, and lipidic systems (e.g., liposomes).

In certain embodiments, the composition comprises a nanoparticle formulation of the efflux inhibitor (e.g., elacridar). As used herein, the term "nanoparticle formulation" refers to a pharmaceutical formulation of a compound (e.g., elacridar) into particles sized between about 1 and about 2000 nanometers (e.g., about 1-100 nM). Such compositions are particularly useful for enhancing the dissolution rate and absorption of the efflux inhibitor(s) (e.g., elacridar), enabling bioavailability, chronic and safe usage of the inhibitor(s). For example, the nanoparticle compositions described herein may comprise nanoparticles comprising an efflux (e.g., an inhibitor of BCRP and/or P-GP (e.g., elacridar)) and a carrier protein. Nanoparticles of poorly water soluble drugs are known in the art, and have been disclosed, for example, in U.S. Patent Nos. 5,916,596, 6,506,405, and 6,537,579. It is contemplated that commercially available nanoparticle platforms can also be utilized. These include, for example, the NanoCrystal® technology (Elan), the MicroPump (Flamel), the Insoluble Drug Delivery (IDD®) platform (SkyePharma), and the MeItDose®technology (Veloxis Pharma). Alternatively, any suitable platforms for dissolution rate and absorption of the inhibitors are contemplated herein. Technologies similar to nanoparticles, include reduction of particle size (of crystalline drug) or formulation of the drug in solution, liposomes, nanospheres and microspheres, as an amorphous system or lipid formulation, solid dispersions, soluble complexes, self-emulsifying drug delivery systems (SEDDS), nanocrystals and mesoporous inorganic carriers, micronization, self-emulsification, cyclodextrin complexation, co-crystallisation, super critical fluid technology, solubilisation by change in pH, salt formation, co-solvents, melt granulation, and solid dispersion, liposomal/niosomal formulations, micronized ingredient with surfactant, solid dispersion, melt granulation/extrusion, liquid or semisolid filled capsule, coating technology.

In certain embodiments, the compositions of the invention are formulated such that the efflux inhibitor achieves one or more of:
1) a Cmax of at least 500ng/ml;
2) a bioavailability of at least 0.1;
3) an AUC(0-48h) of at least 900 ug/ml*min;
4) an AUC(0-∞) of at least 1100 ug/ml*min;
5) an elimination half-life (T1/2) of at least 10 h;
when the composition is administered by oral gavage at 100 mg/kg to fasted, female Sprague-Dawley rats.

In certain embodiments, the compositions of the invention are formulated such that the efflux inhibitor achieves two or more of:
1) a Cmax of at least 500ng/ml;
2) a bioavailability of at least 0.1;
3) an AUC(0-48h) of at least 900 ug/ml*min;
4) an AUC(0-∞) of at least 1100 ug/ml*min;
5) an elimination half-life (T1/2) of at least 10 h;
when the composition is administered by oral gavage at 100 mg/kg to fasted, female Sprague-Dawley rats.

In certain embodiments, the compositions of the invention are formulated such that the efflux inhibitor achieves three or more of:
1) a Cmax of at least 500ng/ml;
2) a bioavailability of at least 0.1;
3) an AUC(0-48h) of at least 900 ug/ml*min;
4) an AUC(0-∞) of at least 1100 ug/ml*min;
5) an elimination half-life (T1/2) of at least 10 h;
when the composition is administered by oral gavage at 100 mg/kg to fasted, female Sprague-Dawley rats.

In certain embodiments, the compositions of the invention are formulated such that the efflux inhibitor achieves four or more of:
1) a Cmax of at least 500ng/ml;
2) a bioavailability of at least 0.1;
3) an AUC(0-48h) of at least 900 ug/ml*min;
4) an AUC(0-∞) of at least 1100 ug/ml*min;
5) an elimination half-life (T1/2) of at least 10 h;
when the composition is administered by oral gavage at 100 mg/kg to fasted, female Sprague-Dawley rats.

In certain embodiments, the compositions of the invention are formulated such that the efflux inhibitor achieves:
1) a Cmax of at least 500ng/ml;
2) a bioavailability of at least 0.1;
3) an AUC(0-48h) of at least 900 ug/ml*min;
4) an AUC(0-∞) of at least 1100 ug/ml*min;
5) an elimination half-life (T1/2) of at least 10 h;
when the composition is administered by oral gavage at 100 mg/kg to fasted, female Sprague-Dawley rats.

In certain of the foregoing embodiments, the Cmax is about 550, about 600, about 650, about 700, about 750, about 800, about 850, about 900, about 950, or about 1000 ng/ml. when the composition is administered by oral gavage at 100 mg/kg to fasted, female Sprague-Dawley rats.

In certain of the foregoing embodiments, the bioavailability is about 0.2, about 0.3, about 0.4, about 0.5, about 0.6, about 0.7, about 0.8, about 0.9, or about 1.0. when the composition is administered by oral gavage at 100 mg/kg to fasted, female Sprague-Dawley rats.

In certain of the foregoing embodiments, the AUC(0-48h) is at least 1000 ug/ml*min, about 1100, about 1200, about 1300, about 1400, about 1500, about 1600, about 1700, about 1800, about 1900, about 2000, about 2500, about 3000, about 3500, about 4000, about 4500, or about 5000 ug/ml*min. when the composition is administered by oral gavage at 100 mg/kg to fasted, female Sprague-Dawley rats.

In certain of the foregoing embodiments, the compositions of the invention are formulated such that the efflux inhibitor achieves an AUC(0-∞) of at least 1100 ug/ml*min. In some embodiments, the AUC(0-∞) is , about 1200, about 1300, about 1400, about 1500, about 1600, about 1700, about 1800, about 1900, about 2000, about 2500, about 3000, about 3500, about 4000, about 4500, or about 5000 ug/ml*min. when the composition is administered by oral gavage at 100 mg/kg to fasted, female Sprague-Dawley rats.

In certain of the foregoing embodiments, the elimination half-life (T1/2) is about 11, about 12, about 13, about 14, about 15, about 16, about 17, about 18, about 19, about 20, about 21, about 22, about 23, or about 24 h), when the composition is administered by oral gavage at 100 mg/kg to fasted, female Sprague-Dawley rats.

In some embodiments, the efflux inhibitor compositions (e.g., nanoparticles) described herein may comprise stabilizers to prevent aggregation of the inhibitor compositions (e.g., nanoparticles). In certain embodiments, a suitable stabilizer is a GRAS (Generally Regarded As Safe) stabilizer. For example, GRAS stabilizers can be milled with the efflux inhibitor (e.g., the BCRP and/or P-GP inhibitor) into nanoparticles by NanoCrystal® technology. Examples of such stabilizers include, but are not limited to, fatty acids and polymers such as aluminum mono-, di-, and tristearate, ammonium citrate, ammonium potassium hydrogen phosphate, calcium glycerophosphate, calcium phosphate, calcium hydrogen phosphate, calcium oleate, calcium acetate, calcium carbonate, calcium ricinoleate, calcium stearate, disodium hydrogen phosphate, magnesium glycerophosphate, magnesium stearate, magnesium phosphate, magnesium hydrogen phosphate, mono-, di-, and trisodium citrate, mono-, di-, and tripotassium citrate, potassium oleate, potassium stearate, sodium pyrophosphate, sodium stearate, sodium tetrapyrophosphate, stannous stearate, zinc orthophosphate, zinc resinate, or D-α-Tocopherol polyethylene glycol succinate (TPGS)or combinations thereof.

The the efflux inhibitor compositions (e.g., nanoparticles) described herein may comprise permeation enhancers. As used herein, the term "permeation enhancer" refers to a compound that enhances transepithelial penetration or membrane permeability of an agent (e.g., an efflux inhibitor). Some examples of permeation enhancers include, but are not limited to, cationic polymers, bioadhesive agents, surface active agents, fatty acids, and chelating agents. Exemplary permeation enhancers that can be used in accordance with the present invention include, but are not limited to, D-α-tocopheryl polyethylene glycol 1000 succinate (TPGS), dioctyl sodium sulfosuccinate, sodium caprate, sodium N-[8(-2-hydroxybenzoyl)amino]caprylate (SNAC), sodium lauryl sulfate, sodium salicylate, oleic acid, lecithin, dehydrated alcohol, Tween (*e.g.,* Tween 20, Tween 40, Tween 60, or Tween 80), Span (e.g., Span 20, Span 40, or Span 80), polyoxyl 40 stearate, polyoxy ethylene 50 stearate, polyethylene glycol (*e.g.,* PEG 3350), polyvinyl alcohol, polyvinylpyrrolidone (*e.g.,* polyvinylpyrrolidone K29-32), hydroxy propyl methyl cellulose (*e.g.,* HPMC 603), polyvinylpyrrolidone/vinyl acetate (VP/VA) copolymer (*e.g.,* Plasdone® S630), poly(lactic-co-glycolic acid), edetate disodium, propylene glycol, glycerol monooleate, fusieates, bile salts, octoxynol and combinations thereof. Suitable permeation enhancers can also include non-ionic, anionic and cationic surfactants or surfactant polyol (*e.g.,* Pluronic® F-127).

In certain embodiments, the nanoparticle or similar formulations further comprise a solubility enhancer (i.e., an agent that enhances the solubility of the efflux inhibitor). Suitable solubility enhancers include, without limitation, TPGS, water-soluble organic solvents (e.g., polyethylene glycol 300, polyethylene glycol 400, ethanol, propylene glycol, glycerin, *N-methyl-2-pyrrolidone,* dimethylacetamide, and dimethylsulfoxide), non-ionic surfactants (e.g., Cremophor EL, Cremophor RH 40, Cremophor RH 60, polysorbate 20, polysorbate 80, Solutol HS 15, sorbitan monooleate, poloxamer 407, Labrafil M-1944CS, Labrafil M-2125CS, Labrasol, Gellucire 44/14, Softigen 767, and mono- and di-fatty acid esters of PEG 300, 400, or 1750), water-insoluble lipids (e.g., castor oil, corn oil, cottonseed oil, olive oil, peanut oil, peppermint oil, safflower oil, sesame oil, soybean oil, hydrogenated vegetable oils, hydrogenated soybean oil, and medium-chain triglycerides of coconut oil and palm seed oil), organic liquids/semi-solids (e.g., beeswax, *d-*α*-tocopherol,* oleic acid, medium-chain mono- and diglyc- erides), cyclodextrins (e.g., α-cyclodextrin, β-cyclodextrin, hydroxypropyl-β-cyclodextrin, and sulfo-butylether-β-cyclodextrin), and phospholipids (hydrogenated soy phosphatidylcholine, distearoylphos- phatidylglycerol, L-α-dimyristoylphosphatidylcholine, L-α-dimyristoylphosphatidylglycerol).

In some embodiments, the composition comprises nanoparticles with an average or mean diameter of no greater than about 2000 nanometers (nm), such as no greater than about 900 nm, about 850 nm, about 800 nm, about 750 nm, about 700 nm, about 650 nm, about 600 nm, about 550 nm, about 500 nm, about 450 nm, about 400 nm, about 350 nm, about 300 nm, about 250 nm, about 200 nm, about 150 nm, about 100 nm, about 75 nm, about 50 nm, about 25 nm, and about 10 nm. In some embodiments, the average or mean diameter of the nanoparticles is no greater than about 100 nm. In some embodiments, the average or mean diameter of the nanoparticles is no greater than about 50 nm. In some embodiments, the average or mean diameter of the nanoparticles is no greater than about 10 nm. In some embodiments, the average or mean diameter of the nanoparticles is about 10 to about 400 nm. In some embodiments, the average or mean diameter of the nanoparticles is about 10 to about 200 nm. In some embodiments, the nanoparticles are sterile-filterable.

The nanoparticles described herein may be present in a dry formulation (such as lyophilized composition) or suspended in a biocompatible medium. Suitable biocompatible media include, but are not limited to, water, buffered aqueous media, saline, buffered saline, optionally buffered solutions of amino acids, optionally buffered solutions of proteins, optionally buffered solutions of sugars, optionally buffered solutions of vitamins, optionally buffered solutions of synthetic polymers, lipid-containing emulsions, and the like.

The nanoparticle compositions described herein may also be formulated as part of a sustained release formulation or a controlled release formulation. As used herein, the term "sustained release formulation" or "controlled release formulation" refers to a formulation that releases its active ingredient(s) in a controlled fashion, for example in specified doses at timed intervals. The sustained or controlled release formulations do not release all of the active ingredient(s) immediately. In certain embodiments, a sustained release formulation of nanoparticles comprising efflux inhibitors (e.g., BCRP and/or P-GP inhibitors) releases no greater than about 95 wt %, about 90 wt %, about 85 wt %, about 80 wt %, about 75 wt %, about 70 wt %, about 65 wt %, about 60 wt %, about 55 wt %, about 50 wt %, about 45 wt %, about 40 wt %, about 35 wt %, about 30 wt %, about 25 wt %, about 20 wt %, about 15 wt %, or about 10 wt % of the inhibitor(s) from the dosage form during the first 2 hours after administration. In this example, the time to release at least 80 wt % of the BCRP and/or P-GP inhibitors from the dosage form may be at least about 4 hours, at least about 6 hours, at least about 8 hours, at least about 10 hours, at least about 12 hours, at least about 15 hours, at least about 20 hours, at least about 30 hours, or at least about 40 hours after administration. It is contemplated that such sustained release or controlled release formulation provides certain blood levels of, for example, elacridar, following administration.

Alternatively, the nanoparticle compositions described herein may also be formulated as part of a delayed released formulation. As used herein, the term "delayed released formulation" refers to a formulation that releases its active ingredient(s) at some point in time after administration, but not immediately. For example, release of the BCRP and/or P-GP inhibitors may be delayed, for example, about 1 hour, about 2 hours, about 3 hours, about 4 hours, about 5 hours, about 6 hours, about 7 hours, about 8 hours, about 9 hours, about 10 hours, about 12 hours, about 15 hours, about 20 hours, about 25 hours, about 30 hours, or about 40 hours after administration.

In certain embodiments, the efflux inhibitor compositions described herein may be formulated into a gastrorentative formulation. Any gastrorentative formulation known in the art me employed in the compositions described herein.

The release profile of the formulations as described herein may be measured by *in vitro* or direct tests, which are well known in the art.

### Methods of Treatment

The present invention provides methods of treating a disease or disorder. As used herein, the terms "treat," "treating," and "treatment" refer to therapeutic or preventative measures to prevent, cure, delay, reduce the severity of, or ameliorate one or more symptoms of a condition (e.g., a disease or disorder) in order to prolong the survival of a subject beyond that expected in the absence of such treatment.

The methods and compositions disclosed herein are particularly useful for treating conditions where the activity of transport proteins (e.g., BCRP and/or P-GP) inhibit effective delivery to, and concentration of, a therapeutic agent to a sanctuary target tissue (e.g., brain, spinal cord, nerves, cerebrospinal fluid, testis, eyeballs, retina, inner ear, placenta, mammary gland, endometrium, liver, biliary tract, kidney, intestines, lung, adrenal cortex, hematopoietic cells, and/or stem cells).

In certain embodiments, the methods and compositions disclosed herein are used in the treatment of neurological conditions. As used herein, the term "neurological condition" refers to any disease or disorder or the nervous system. Exemplary neurological conditions include, without limitation, cancer (including brain metastasis), depression, Acid Lipase Disease, Acid Maltase Deficiency, Acquired Epileptiform Aphasia, Acute Disseminated Encephalomyelitis, ADHD, Adie's Pupil, Adie's Syndrome, Adrenoleukodystrophy, Agenesis of the Corpus Callosum, Agnosia, Aicardi Syndrome, Aicardi-Goutieres Syndrome Disorder, AIDS - Neurological Complications, Alexander Disease, Alpers' Disease, Alternating Hemiplegia, Alzheimer's Disease, Amyotrophic Lateral Sclerosis (ALS), Anencephaly, Aneurysm, Angelman Syndrome, Angiomatosis, Anoxia, Antiphospholipid Syndrome, Aphasia, Apraxia, Arachnoid Cysts, Arachnoiditis, Arnold-Chiari Malformation, Arteriovenous Malformation, Asperger Syndrome, Ataxia, Ataxia Telangiectasia, Ataxias and Cerebellar or Spinocerebellar Degeneration, Atrial Fibrillation and Stroke, Attention Deficit-Hyperactivity Disorder, Autism, Autonomic Dysfunction, Barth Syndrome, Batten Disease, Becker's Myotonia, Behcet's Disease, Bell's Palsy, Benign Essential Blepharospasm, Benign Focal Amyotrophy, Benign Intracranial Hypertension, Bernhardt-Roth Syndrome, Binswanger's Disease, Blepharospasm, Bloch-Sulzberger Syndrome, Brachial Plexus Birth Injuries, Brachial Plexus Injuries, Bradbury-Eggleston Syndrome, Brain and Spinal Tumors, Brain Aneurysm, Brain Injury, Brown-Sequard Syndrome, Bulbospinal Muscular Atrophy, Canavan Disease, Carpal Tunnel Syndrome, Causalgia, Cavernomas, Cavernous Angioma, Cavernous Malformation, Central Cervical Cord Syndrome, Central Cord Syndrome, Central Pain Syndrome, Central Pontine Myelinolysis, Cephalic Disorders, Ceramidase Deficiency, Cerebellar Degeneration, Cerebellar Hypoplasia, Cerebral Aneurysms, Cerebral Arteriosclerosis, Cerebral Atrophy, Cerebral Beriberi, Cerebral Cavernous Malformation, Cerebral Gigantism, Cerebral Hypoxia, Cerebral Palsy, Cerebro-Oculo-Facio-Skeletal Syndrome (COFS), Charcot-Marie-Tooth Disease, Chiari Malformation, Cholesterol Ester Storage Disease, Chorea, Choreoacanthocytosis, Chronic Inflammatory Demyelinating Polyneuropathy (CIDP), Chronic Orthostatic Intolerance, Chronic Pain, Cockayne Syndrome Type II, Coffin Lowry Syndrome, Colpocephaly, Coma, Complex Regional Pain Syndrome, Congenital Facial Diplegia, Congenital Myasthenia, Congenital Myopathy, Congenital Vascular Cavernous Malformations, Corticobasal Degeneration, Cranial Arteritis, Craniosynostosis, Cree encephalitis, Creutzfeldt-Jakob Disease, Cumulative Trauma Disorders, Cushing's Syndrome, Cytomegalic Inclusion Body Disease, Cytomegalovirus Infection, Dancing Eyes-Dancing Feet Syndrome, Dandy-Walker Syndrome, Dawson Disease, De Morsier's Syndrome, Deep Brain Stimulation for Parkinson's Disease, Dejerine-Klumpke Palsy, Dementia, Dementia - Multi-Infarct, Dementia - Semantic, Dementia - Subcortical, Dementia With Lewy Bodies, Dentate Cerebellar Ataxia, Dentatorubral Atrophy, Dermatomyositis, Developmental Dyspraxia, Devic's Syndrome, Diabetic Neuropathy, Diffuse Sclerosis, Dravet Syndrome, Dysautonomia, Dysgraphia, Dyslexia, Dysphagia, Dyspraxia, Dyssynergia Cerebellaris Myoclonica, Dyssynergia Cerebellaris Progressiva, Dystonias, Early Infantile Epileptic Encephalopathy, Empty Sella Syndrome, Encephalitis, Encephalitis Lethargica, Encephaloceles, Encephalopathy, Encephalopathy (familial infantile), Encephalotrigeminal Angiomatosis, Epilepsy, Epileptic Hemiplegia, Erb-Duchenne and Dejerine-Klumpke Palsies, Erb's Palsy, Essential Tremor, Extrapontine Myelinolysis, Fabry Disease, Fahr's Syndrome, Fainting, Familial Dysautonomia, Familial Hemangioma, Familial Idiopathic Basal Ganglia Calcification, Familial Periodic Paralyses, Familial Spastic Paralysis, Farber's Disease, Febrile Seizures, Fibromuscular Dysplasia, Fisher Syndrome, Floppy Infant Syndrome, Foot Drop, Friedreich's Ataxia, Frontotemporal Dementia , Gaucher Disease, Generalized Gangliosidoses, Gerstmann's Syndrome, Gerstmann-Straussler-Scheinker Disease, Giant Axonal Neuropathy, Giant Cell Arteritis, Giant Cell Inclusion Disease, Globoid Cell Leukodystrophy, Glossopharyngeal Neuralgia, Glycogen Storage Disease, Guillain-Barré Syndrome, Hallervorden-Spatz Disease, Head Injury, Headache, Hemicrania Continua, Hemifacial Spasm, Hemiplegia Alterans, Hereditary Neuropathies, Hereditary Spastic Paraplegia, Heredopathia Atactica Polyneuritiformis, Herpes Zoster, Herpes Zoster Oticus, Hirayama Syndrome, Holmes-Adie syndrome , Holoprosencephaly, HTLV-1 Associated Myelopathy, Hughes Syndrome, Huntington's Disease, Hydranencephaly, Hydrocephalus, Hydrocephalus - Normal Pressure, Hydromyelia, Hypercortisolism, Hypersomnia, Hypertonia, Hypotonia, Hypoxia, Immune-Mediated Encephalomyelitis, Inclusion Body Myositis, Incontinentia Pigmenti, Infantile Hypotonia, Infantile Neuroaxonal Dystrophy , Infantile Phytanic Acid Storage Disease, Infantile Refsum Disease, Infantile Spasms, Inflammatory Myopathies, Iniencephaly, Intestinal Lipodystrophy, Intracranial Cysts, Intracranial Hypertension, Isaac's Syndrome, Joubert Syndrome, Kearns-Sayre Syndrome, Kennedy's Disease, Kinsbourne syndrome, Kleine-Levin Syndrome, Klippel-Feil Syndrome, Klippel-Trenaunay Syndrome (KTS), Klüver-Bucy Syndrome, Korsakoff's Amnesic Syndrome, Krabbe Disease, Kugelberg-Welander Disease, Kuru, Lambert-Eaton Myasthenic Syndrome, Landau-Kleffner Syndrome, Lateral Femoral Cutaneous Nerve Entrapment, Lateral Medullary Syndrome, Learning Disabilities, Leigh's Disease, Lennox-Gastaut Syndrome, Lesch-Nyhan Syndrome, Leukodystrophy, Levine-Critchley Syndrome, Lewy Body Dementia, Lipid Storage Diseases, Lipoid Proteinosis, Lissencephaly, Locked-In Syndrome, Lou Gehrig's Disease, Lupus - Neurological Sequelae, Lyme Disease - Neurological Complications, Machado-Joseph Disease, Macrencephaly, Megalencephaly, Melkersson-Rosenthal Syndrome, Meningitis, Meningitis and Encephalitis, Menkes Disease, Meralgia Paresthetica, Metachromatic Leukodystrophy, Microcephaly, Migraine, Miller Fisher Syndrome, Mini Stroke, Mitochondrial Myopathies, Moebius Syndrome, Monomelic Amyotrophy, Motor Neuron Diseases, Moyamoya Disease, Mucolipidoses, Mucopolysaccharidoses, Multifocal Motor Neuropathy, Multi-Infarct Dementia, Multiple Sclerosis, Multiple System Atrophy, Multiple System Atrophy with Orthostatic Hypotension, Muscular Dystrophy, Myasthenia - Congenital, Myasthenia Gravis, Myelinoclastic Diffuse Sclerosis, Myoclonic Encephalopathy of Infants, Myoclonus, Myopathy, Myopathy - Congenital, Myopathy - Thyrotoxic, Myotonia, Myotonia Congenita, Narcolepsy, Neuroacanthocytosis, Neurodegeneration with Brain Iron Accumulation, Neurofibromatosis, Neuroleptic Malignant Syndrome, Neurological Complications of AIDS, Neurological Complications of Lyme Disease, Neurological Consequences of Cytomegalovirus Infection, Neurological Manifestations of Pompe Disease, Neurological Sequelae Of Lupus, Neuromyelitis Optica , Neuromyotonia, Neuronal Ceroid Lipofuscinosis, Neuronal Migration Disorders, Neuropathy - Hereditary, Neurosarcoidosis, Neurosyphilis, Neurotoxicity, Nevus Cavernosus, Niemann-Pick Disease, Normal Pressure Hydrocephalus, Occipital Neuralgia, Ohtahara Syndrome, Olivopontocerebellar Atrophy, Opsoclonus Myoclonus, Orthostatic Hypotension, O'Sullivan-McLeod Syndrome, Overuse Syndrome, Pain - Chronic, Pantothenate Kinase-Associated Neurodegeneration, Paraneoplastic Syndromes, Paresthesia, Parkinson's Disease, Paroxysmal Choreoathetosis, Paroxysmal Hemicrania, Parry-Romberg, Pelizaeus-Merzbacher Disease, Pena Shokeir II Syndrome, Perineural Cysts, Periodic Paralyses, Peripheral Neuropathy, Periventricular Leukomalacia, Persistent Vegetative State, Pervasive Developmental Disorders, Phytanic Acid Storage Disease, Pick's Disease, Pinched Nerve, Piriformis Syndrome, Pituitary Tumors, Polymyositis, Pompe Disease, Porencephaly, Postherpetic Neuralgia, Postinfectious Encephalomyelitis, Post-Polio Syndrome, Postural Hypotension, Postural Orthostatic Tachycardia Syndrome, Postural Tachycardia Syndrome, Primary Dentatum Atrophy, Primary Lateral Sclerosis, Primary Progressive Aphasia, Prion Diseases, Progressive Hemifacial Atrophy, Progressive Locomotor Ataxia, Progressive Multifocal Leukoencephalopathy, Progressive Sclerosing Poliodystrophy, Progressive Supranuclear Palsy, Prosopagnosia, Pseudo-Torch syndrome, Pseudotoxoplasmosis syndrome, Pseudotumor Cerebri, Psychogenic Movement, Ramsay Hunt Syndrome I , Ramsay Hunt Syndrome II, Rasmussen's Encephalitis, Reflex Sympathetic Dystrophy Syndrome, Refsum Disease, Refsum Disease - Infantile, Repetitive Motion Disorders, Repetitive Stress Injuries, Restless Legs Syndrome, Retrovirus-Associated Myelopathy, Rett Syndrome, Reye's Syndrome, Rheumatic Encephalitis, Riley-Day Syndrome, Sacral Nerve Root Cysts, Saint Vitus Dance, Salivary Gland Disease, Sandhoff Disease, Schilder's Disease, Schizencephaly, Seitelberger Disease, Seizure Disorder, Semantic Dementia, Septo-Optic Dysplasia, Severe Myoclonic Epilepsy of Infancy (SMEI), Shaken Baby Syndrome, Shingles, Shy-Drager Syndrome, Sjögren's Syndrome, Sleep Apnea, Sleeping Sickness, Sotos Syndrome, Spasticity, Spina Bifida, Spinal Cord Infarction, Spinal Cord Injury, Spinal Cord Tumors, Spinal Muscular Atrophy, Spinocerebellar Atrophy, Spinocerebellar Degeneration, Steele-Richardson-Olszewski Syndrome, Stiff-Person Syndrome, Striatonigral Degeneration, Stroke, Sturge-Weber Syndrome, Subacute Sclerosing Panencephalitis, Subcortical Arteriosclerotic Encephalopathy, SUNCT Headache, Swallowing Disorders, Sydenham Chorea, Syncope, Syphilitic Spinal Sclerosis, Syringohydromyelia, Syringomyelia, Systemic Lupus Erythematosus, Tabes Dorsalis, Tardive Dyskinesia, Tarlov Cysts, Tay-Sachs Disease, Temporal Arteritis, Tethered Spinal Cord Syndrome, Thomsen's Myotonia, Thoracic Outlet Syndrome, Thyrotoxic Myopathy, Tic Douloureux, Todd's Paralysis, Tourette Syndrome, Transient Ischemic Attack, Transmissible Spongiform Encephalopathies, Transverse Myelitis, Traumatic Brain Injury, Tremor, Trigeminal Neuralgia, Tropical Spastic Paraparesis, Troyer Syndrome, Tuberous Sclerosis, Vascular Erectile Tumor, Vasculitis Syndromes of the Central and Peripheral Nervous Systems , Von Economo's Disease, Von Hippel-Lindau Disease (VHL), Von Recklinghausen's Disease, Wallenberg's Syndrome, Werdnig-Hoffman Disease, Wernicke-Korsakoff Syndrome, West Syndrome, Whiplash, Whipple's Disease, Williams Syndrome, Wilson Disease, and Wolman's Disease.

In certain embodiments, the methods and compositions disclosed herein are used in the treatment of non-neurological conditions. Exemplary non-neurological conditions include cancer, HIV infection, Inflammatory bowl disease, hyperlipidemia, emesis, retinoblastoma, hearing loss, tinnitus, acoustic neuroma, leprosy, gout, systemic lupus erythematosus (SLE), diabetic macular edema (DME), macular degeneration (AMD), and central retinal vein occlusion (CRVO). In certain embodiments, the cancer involves cancer stem cells (pluripotent or multipotent) that manifest upregulation of efflux transporters (e.g., P-GP and or BCRP efflux transporters), and thereby avoiding killing by therapeutic agents.

In certain embodiments, the present invention provides methods of preventing and/or treating and/or ameliorating a neurological condition in a mammalian (e.g., human) subject. In certain aspects, the invention is for use in combination therapy, whereby one or more inhibitors of BCRP and/or P-GP is administered to a mammalian (e.g., human) subject undergoing therapy with one or more inhibitors of tyrosine kinase, such that the distribution of the active ingredient into the sanctuary target tissues protected by the blood-organ barrier and or the P-GP and or BCRP efflux transporters to the one or more inhibitors of tyrosine kinase is enhanced. It is contemplated that the present invention may be useful for treating, preventing, or lessening the severity of a neurological disease, condition, or disorder where activation of c-kit and/or other tyrosine kinases are implicated in the disease. Specifically, the present invention may be useful for preventing and/or treating neurological conditions including, but not limited to, neurofibromatosis and the associated plexiform neurofibromas, neuro-cardio-facial-cutaneous syndromes, primary brain cancers including but not limited to astrocytic, oligodendroglial, oligoastrocytic, ependymal, choroid plexus, other neuroepithelial, neuronal and mixed neuronal-glial, pineal, embryonal, cranial and paraspinal nerve, meningeal, and sellar region tumors (e.g., glioblastoma multiforme, tumors of the brain stem, hypophtalmic glioma, cerebellar astrocytoma, cerebral astrocytoma, medulloblastoma, ependymoma, neuroectodermal or pineal tumor), secondary brain metastasis (for example, from brain cancer, lung cancer, chronic myelogenous leukemia, acute lymphoblastic leukemia, or gastrointestinal stromal tumor, e.g., breast cancer brain metastasis (BCBM)), HIV-associated neurological disorders, epilepsy, and multiple sclerosis.

Further, the present invention may also be useful for treating, preventing, or lessening the severity of any neurological disease, condition, or disorder where cognitive functions are impaired. For example, the present invention may also be useful in the treatment of neurodegenerative diseases including, but not limited to Alzheimer's disease, mild cognitive impairment, Trisomy 21 (Down Syndrome), cerebral amyloid angiopathy, degenerative dementia, Hereditary Cerebral Hemorrhage with Amyloidosis of the Dutch-Type (HCHWA-D), Creutzfeld-Jakob disease, prion disorders, amyotrophic lateral sclerosis, progressive supranuclear palsy, head trauma, and stroke.

The present invention also relates to methods for increasing the distribution of the active ingredient into the target tissues protected by the blood-organ barrier to the one or more inhibitors of tyrosine kinase is enhanced in a mammalian (e.g., human) subject. In one such method, one or more inhibitors of BCRP and/or P-GP are administered to the subject under conditions effective to increase the distribution of one or more therapeutic agents (e.g., inhibitors of tyrosine kinase) into the subject's nervous system. In another such method, the subject's blood-brain and/or blood-nerve barrier is contacted with one or more inhibitors of BCRP and/or P-GP prior to administration of one or more inhibitors of tyrosine kinase. "Blood-brain barrier permeability" and "blood-nerve barrier permeability", as used herein, refers to the degree to which large molecules such as tyrosine kinase inhibitors (e.g., having a molecular weight of at least 5 kDa, such as at least about 10 kDa, at least about 20 kDa, at least about 30 kDa, at least about 40 kDa, at least about 50 kDa, at least about 60 kDa, at least about 70 kDa, etc.) cross the blood-brain barrier and/or the blood-nerve barrier of a mammalian (e.g., human) subject and retain inside the target sanctuary tissue (e.g., brain or the endoneurial microenvironment) long enough, and at sufficient concentrations, to exert their pharmacological effects. "Increase or enhance," as used in this context, is meant to include any measurable increase in blood-brain and/or blood-nerve barrier permeability, such as, for example, an increase of greater than about 5% (e.g., greater than about 10%, greater than about 15%, greater than about 20%, greater than about 40%, greater than about 60%, greater than about 80%, and/or greater than about 100%). In certain embodiments, the present invention enhances the blood-brain barrier concentration of the therapeutic agent to achieve a brain (or cerebrospinal fluid):plasma ratio of at least about 50%, at least about 55%, at least about 60%, at least about 65%, at least about 70%, at least about 75%, at least about 80%, at least about 85%, at least about 90%, at least about 95%, at least about 100%, at least about 150%, at least about 200%, at least about 250%, at least about 300%, at least about 350%, %, at least about 450%, or at least about 500%.

The present invention provides methods utilizing an effective amount of one or more therapeutic agents and one or more efflux inhibitors (e.g., inhibitors of BCRP and/or P-GP). The term "effective amount" as used herein refers to an amount of a therapeutic agent or composition sufficient to treat a specified disorder, condition or disease such as ameliorate, palliate, lessen, and/or delay one or more of its symptoms in a mammalian (e.g., human) subject. In reference to tumors or other unwanted cell proliferation, an effective amount comprises an amount sufficient to cause a tumor to shrink and/or to decrease the growth rate of the tumor (such as to suppress tumor growth) or to prevent or delay other unwanted cell proliferation. In some embodiments, an effective amount is an amount sufficient to delay development. In some embodiments, an effective amount is an amount sufficient to prevent or delay occurrence and/or recurrence. An effective amount can be administered in one or more administrations. In the case of tumors, the effective amount of the therapeutic agent may: (i) reduce the number of tumor cells; (ii) reduce tumor size; (iii) inhibit, retard, slow to some extent and preferably stop tumor cell infiltration into peripheral organs; (iv) inhibit (*i.e.,* slow to some extent and preferably stop) tumor metastasis; (v) inhibit tumor growth; (vi) prevent or delay occurrence and/or recurrence of tumor; and/or (vii) relieve to some extent one or more of the symptoms associated with the cancer. Additionally, the term "effective amount" as used herein refers to an amount of a therapeutic agent or composition sufficient to enhance the blood-brain barrier and/or blood-nerve barrier permeability of a therapeutic agent as defined previously.

It is further contemplated that the therapy involving the use of one or more therapeutic agents and one or more efflux inhibitors (e.g., inhibitors of BCRP and/or P-GP) as described herein may be performed alone or in combination with another therapy, such as surgery, radiation, chemotherapy, immunotherapy, gene therapy, and the like. For example, the use of a therapeutic agent (e.g., one or more inhibitors of tyrosine kinase) and an efflux inhibitor (e.g., one or more inhibitors of BCRP and/or P-GP) as described herein may be used in combination with, for example, one or more of sirolimus, lovastatin, cediranib, sorafenib, and/or talaporfin in the treatment of neurofibromatosis.

### EXAMPLES

### Example 1

### Nanoparticle Formulations of Elacridar

Various nanoparticle formulations of elacridar were made as detailed in Table 1 below:

**Table 1 Nanoparticle formulations of Elacridar**

| Formulation | Elacridar HCl (w/w%) | Stabilizer 1 (w/w%) | Stabilizer 2 (0.05%) (w/w%) | Dmean (nm) |
|---|---|---|---|---|
| 1 | 5% | PVP K29/32 (1.5 %) | Sodium docusate | 169 |
| 2 | 5% | HPMC 603 (1.5 %) | Sodium docusate | 186 |
| 3 | 5% | Plasdone S630 (1.5 %) | Sodium docusate | 161,000 |
| 4 | 5% | Tween 80 (1.0 %) | - | 180 |
| 5 | 5% | Pluronic F127 (1.0 %) | - | 108 |

Specifically, each formulation was each processed in a roller mill (US Stoneware model 755), in which each formulation was milled in a 20 mL glass bottle of 30 mm diameter for 3 days at 192 rpm. Each bottle contained 5 g of formulation and 36.5 g of Yttria Zirconia ceramic milling media of 0.8 mm diameter. The formulations were separated from the milling media and thereafter evaluated by examining their mean particle size distribution using a Horiba LA-950 laser light diffraction particle sizing instrument.

Nanoparticle sizes for the various formulations were determined using the LA-950 Laser Particle Size Analyzer (Horiba Scientific). Particle size for formulations 1-5 are detailed in Table 2 below. Formulations 1, 2, 4 and 5 produced dispersions with a mean particle size below 200 nm. Formulation 3 had a particle size in excess of 100 µm.

**Table 2 Elacridar nanoparticle characteristics**

| Formulation | Elacridar HCl (w/w%) | Stabilizer 1 (w/w%) | Stabilizer 2 (w/w%) | Dmean (nm) |
|---|---|---|---|---|
| 1 | 5% | PVP K29/32 | DOSS | 169 |
| 2 | 5% | HPMC 603 | DOSS | 186 |
| 3 | 5% | Plasdone S630 | DOSS | 161,000 |
| 4 | 5% | Tween 80 | - | 180 |
| 5 | 5% | Pluronic F127 | - | 108 |

The formulations were also evaluated in terms of morphology and dispersion using an Olympus BX51 microscope equipped with an oil immersion objective producing a 1000x magnification. Formulations 1,2,4 and 5 displayed spherical like particles, free of aggregates and having Brownian motion. Formulation 3 displayed string like aggregates and was deemed not useful. Formulations 1 and 2 were both based on a polymeric stabilizer and a secondary anionic stabilizer, were each deemed viable formulations. Formulation 2 was selected over 1 based on superior optical appearance. Formulations 4 and 5 were both based on a single amphiphilic stabilizer. Both the particle size and optical appearance favored formulation 5 over 4. Thus, formulations 2 and 5 were chosen as viable development candidates. Formulations 1 and 4 were considered viable back-ups.

### Example 2

### Nanoparticle Manufacture

Nanoparticles formulations 2 and 5 from example 1 were prepared using a stirred media mill. Each formulation was processed in a custom built vertical media mill consisting of a10 mL stainless steel mill chamber equipped with a smooth agitator shaft. About 4.5 g of formulation and about 5.5 g of milling media were charged into the milling chamber and the mill was run at 5000 rpm for 30 min. The milling media consisted of 0.5 mm polystyrene beads.

After milling, the formulations were separated from the milling media and visually inspected. Formulations 2 and 5 were both free flowing, indicative of stable dispersions. The mean particle size distribution of formulations 2 and 5 was 140 and 110 nm, respectively, as measured using a Horiba LA-950 laser light diffraction particle sizing instrument.

The formulations were also evaluated in terms of morphology and dispersion using an Olympus BX51 microscope equipped with an oil immersion objective producing a 1000x magnification. Both displayed spherical like particles, free of aggregates and having Brownian motion. Both were considered viable candidates for late stage evaluation.

### Example 3

### In vitro permeability of elacridar

An *in vitro* permeability assay using the MDCK cell line was performed to investigate the ability of elacridar to cross cellular membranes. Experiments were performed essentially as described in van Breemen RB et al. Expert Opin Drug Metab Toxicol 2005; 1: 175-85 (which is incorporated by reference herein in its entirety), except that MDCK cells were employed rather than Caco-2 cells. Specifically, non-transduced MDCK cells, expressing only basal amounts of endogenous ABC-transporters, were seeded in the apical compartments of a 24mm Transwell plate (3.0µm Pore Polycarbonate Membrane Inserts) and incubated at 37 °C and 5.0% CO₂ conditions until confluency was reached. Both apical-to-basolateral and basolateral-to-apical transport was analyzed in triplicate. To each donor compartment, 2 ml of Minimal Essential Medium (supplemented with 20% Fetal Bovine Serum) containing 1 µM elacridar and 50 nCi/ml (1.85 kBq/ml) ¹⁴C-inulin was added, while each acceptor compartment was filled with 2 ml blank Minimal Essential Medium (supplemented with 20% Fetal Bovine Serum). Samples of 100 µl were taken from each acceptor compartment at t = 5 min, 30 min, 1 h, 2 h and 4 h as well as from all donor solutions for HPLC-MS/MS analysis of elacridar.

Samples for HPLC-MS/MS analysis were prepared as follows. Samples (50 µl) were pipetted into a 2 ml eppendorf vial and the internal standard (IS) solution (1 µM of elacridar-d4) in Minimal Essential Medium (supplemented with 20% Fetal Bovine Serum) and 1 ml of diethyl ether were added. Tubes were vigorously mixed for at least 5 min, centrifuged (2 min at 20,000g), then placed in a bath of ethanol with dry-ice, in order to freeze the aqueous bottom layer. The organic supernatant was decanted into a clean 1.5 ml Brand vial and evaporated under vacuum in a Speed-Vac (Savant). The residue was reconstituted in 100 µl of acetonitrile:water (30:70 v/v). An aliquot of 75 µl was subjected to HPLC-MS/MS using the conditions and set-up as described above for elacridar.

Leakage of the transwell membrane, as indicated by ¹⁴C-inulin accumulation in the acceptor compartment, was analyzed in 10 µl samples from all time points as well as donor solutions. To each sample, 3 ml of Ultima Gold solution was added and vials were mixed thoroughly prior to radioactivity analysis using a Liquid Scintillation Counter.

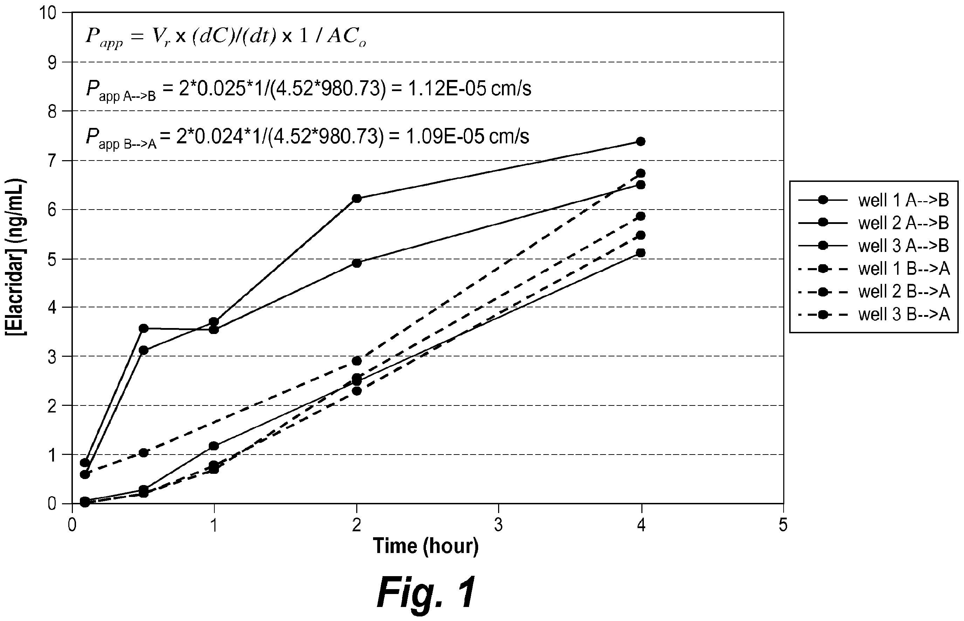

The results, set forth in Table 3, Table 4 and Figure 1 herein, show that the apical-to-basolateral and basolateral-to-apical apparent permeability coefficient (Pₐₚₚ) of elacridar were similar in both directions, viz. 1.12E-5 and 1.09E-5 cm/s, respectively.

**Table 3. Elacridar permeability through the MDCK cell monolayer.**

| **Apical-to-basolateral permeability** | | | | | | | |
|---|---|---|---|---|---|---|---|
| time (min) | well 1 | well 2 | well 3 | mean (ng/ml) | SD | n | SE |
| 0.083 | 0.808 | 0.57 | 0.0519 | 0.476633 | 0.3866 | 3 | 0.223204 |
| 0.5 | 3.55 | 3.11 | 0.299 | 2.319667 | 1.763723 | 3 | 1.018286 |
| 1 | 3.53 | 3.69 | 1.15 | 2.79 | 1.422533 | 3 | 0.8213 |
| 2 | 4.92 | 6.21 | 2.51 | 4.546667 | 1.87804 | 3 | 1.084287 |
| 4 | 6.49 | 7.37 | 5.1 | 6.32 | 1.144509 | 3 | 0.660782 |

| **Basolateral-to-apical permeability** | | | | | | | |
|---|---|---|---|---|---|---|---|
| time (min) | well 1 | well 2 | well 3 | mean (ng/ml) | SD | n | SE |
| 0.083 | 0.026 | <0 | 0.618 | 0.322 | 0.418607 | 2 | 0.296 |
| 0.5 | 0.211 | 0.172 | 1.03 | 0.471 | 0.484501 | 3 | 0.279727 |
| 1 | 0.685 | 0.753 | -- | 0.719 | 0.048083 | 2 | 0.034 |
| 2 | 2.55 | 2.29 | 2.9 | 2.58 | 0.306105 | 3 | 0.17673 |
| 4 | 5.83 | 5.45 | 6.69 | 5.99 | 0.635295 | 3 | 0.366788 |

**Table 4 Elacridar MDCK permeability assay parameters.**

| | Apical to Basal | Basal to Apical |
|---|---|---|
| dC/dt (ng/mL/s) | 0.024865 | 0.024119 |
| Vr (ml) | 2 | 2 |
| C0 (ng) | 980.73 | 980.73 |
| A (cm²) | 4.52 | 4.52 |
| Papp (cm/s) | 1.12E-05 | 1.09E-05 |

These data show for, the first time that, elacridar has very low permeability across cellular membranes. Indeed, the amount of elacridar at the receptor side was only about 6% of the drug content present at the donor side and this value is in the range that is accepted for the leakage marker, 14-carbon labelled inulin. These data indicate that in addition to poor solubility, elacridar also suffers from poor permeability. These properties of elacridar explain its poor oral bioavailability and indicate that the bioavailability of elacridar could be improved by the use of permeation enhancers. As elacridar is a P-GP substrate at low doses, and a competitive inhibitor at higher doses, elacridar serves partially as its own permeation enhancer when combined with a solubility enhancer (see e.g., Kawamura 2011 Mol Imaging Biol. Feb;13(1):152-60, which is incorporated by reference herein in its entirtey).

### Example 4

### Compatibility of Nanoparticles with Solubility and or Permeation Enhancers

The compatibility of elacridar nanoparticulate formulations with solubility and or permeation enhancing agents was determined. The permeation enhancing agents were used as primary stabilizers in the making of nanoparticulate formulations as set forth in Table 5.

**Table 5 Elacridar/solubility and or permeation enhancer nanoparticle formulations**

| Formulation | Elacridar HCl (w/w%) | Stabilizer/Permeation Enhancer (w/w%) |
|---|---|---|
| 6 | 5% | poloxamer 407 - 5% |
| 7 | 5% | TPGS - 1% |
| 8 | 5% | sodium caprate 1% |

Each formulation was processed in a custom built vertical media mill consisting of a10 mL stainless steel mill chamber equipped with a smooth agitator shaft. About 4.5 g of formulation and about 5.5 g of milling media (0.5 mm polystyrene beads) was charged into the milling chamber and the mill was run at 5000 rpm for 1 hr. After milling, the formulations were separated from the milling media and visually evaluated. Formulations 6 and 7 were free flowing dispersions and formulation 8 was a thick agglomerated paste, which could not be separated from the media bed. The formulations were further evaluated by examining their mean particle size distribution using a Horiba LA-950 laser light diffraction particle sizing instrument. Formulations 6 and 7 produced dispersions with a mean particle size of approximately 100 nm each. Formulation 8 was not sized.

The formulations were also evaluated in terms of morphology and dispersion by using an Olympus BX51 microscope equipped with an oil immersion objective producing a 1000x magnification. Formulations 6 and 7 displayed spherical like particles, free of aggregates and having Brownian motion. Both were considered viable candidates for further evaluation. Formulation 8 was not evaluated further.

### Example 5

### Nanoparticle Stability in Simulated Gastric and Intestinal Fluids

The stability toward of Elacridar nanoparticulate formulations in simulated gastric fluid (SGF) and simulated intestinal fluid (SIF) was determined. The absence of aggregation was the preferred outcome. The compositions of the simulated fluids are set forth in Table 6.

**Table 6 Simulated gastric and intestinal fluids**

| Simulated Gastric Fluid | | Simulated Intestinal Fluid | |
|---|---|---|---|
| NaCl | 34 mM | KH2PO4 | 6.805 g |
| HCl | 25 mM | NaOH | 0.896 g |
| | | Water | q.s. to 1.00 L |
| pH = 1.6 | | Corresponds to 50 mM phosphate buffer at pH = 6.8 | |

Formulations 1, 2, 4, and 5 from Example were evaluated first. Specifically, one part formulation (100 µL) was added to four part of simulated fluids (400 µL) into a microfuge tube and vortexed. This rendered the formulation diluted 1:5 and the test fluids (SGF and SIF) at 80% strength. The samples were evaluated under the optical microscope after about 10 min. The results for all tested formulations are summarized in the Table 7. With the exception of formulation 5, all formulations exhibited instability toward SGF and SIF. Formulation -5 was marginally stable toward SIF, however, eventually this aggregated as well.

**Table 7 Stability of elacridar nanoparticulate formulations 1, 2, 4 and 5 in simulated gastric and simulated intestinal fluids gastric and intestinal fluids**

| Formulation | Elacridar HCl (w/w%) | Stabilizer 1 | Stabilizer 2 | SGF | SIF |
|---|---|---|---|---|---|
| 1 | 5% | PVP K29/32 | DOSS | Aggregated | Aggregated |
| 2 | 5% | HPMC 603 | DOSS | Aggregated | Aggregated |
| 4 | 5% | Tween 80 | - | Aggregated | Aggregated |
| 5 | 5% | Pluronic F127 | - | Aggregated | Slow Aggregation |

The experiment was repeated using formulations 6 and 7 from Example 3 using the same procedure. The results, set forth in Table 8, show that formulations 6 and 7 were both stable in SIF but aggregated in SGF.

**Table 8 Stability of elacridar nanoparticulate formulations 6 and 7 in simulated gastric and simulated intestinal fluids gastric and intestinal fluids**

| Formulation | Elacridar HCl (w/w%) | Stabilizer system (w/w%) | SGF | SIF |
|---|---|---|---|---|
| 6 | 5.0% | 5% F127 | Aggregated | Stable |
| 7 | 5.0% | 1% TPGS | Aggregated | Stable |

The experiment was repeated using formulations 6 and 7 from Example 3, but with additional solubility and or permeation enhancers present. The solubility enhancer was poloxamer 407 or F127), added to formulation-6, and the stabilizer, solubility and permeability enhancer tochopherol polyethylene glycol succinate (TPGS), added to formulation-7. The dilution scheme was as follows: 1) 100 µL of formulation; 2) 125 µL of 20% F127 or TPGS solution; 3) 275 µL of SGF or SIF fluid are added to a microfuge tube and vortexed. This renders the formulation diluted 1:5 while ensuring a 5% strength of F127 or TPGS in the supernatant. The test fluids (SGF or SIF) are at 55% strength. The samples were evaluated under the optical microscope after about 10 min. The results, set forth in Table 9, show that enhanced formulations 6 and 7 were stable in both SIF and SGF.

**Table 9 Stability of elacridar nanoparticulate formulations 6 and 7 in simulated gastric and simulated intestinal fluids gastric and intestinal fluids**

| Formulation | Elacridar HCl (w/w%) | Stabilizer system (w/w%) | PE in supernatant*(w/w%) | SGF | SIF |
|---|---|---|---|---|---|
| HE-6 | 5.0% | 5% F127 | 5% F127 | Stable | Stable |
| HE-7 | 5.0% | 1% TPGS | 5% TPGS | Stable | Stable |

| | | | | | |
|---|---|---|---|---|---|
| Final conc. after all dilutions | | | | | |

### Example 6

### Pharmacokinetic Analysis pf Elacridar Nanoparticle Formulations and the Effect of Elacridar Nanoparticle Formulations on Brain Penetration of Therapeutic Agents

The following experiments investigated: 1) the PK parameters of elacridar nanoparticle (NP) formulations relative to a prior art conventional Elacridar suspension formulation in female Sprague-Dawley rats; and 2) the effect of the of the elacridar nanoparticle (NP) formulations on the brain penetration of three different therapeutic agents (Docetaxel, Imatinib, and Loperamide) in wildtype FVB mice.

### Materials and Methods:

### A. Pharmacokinetics of oral elacridar dosing formulations

Studies were conducted in female Sprague-Dawley rats. Ward et al. J Pharmacol Exp Ther2004; 310: 703-9 (which is incorporated by reference herein in its entirety) have reported that this species demonstrates a nonlinear oral bioavailability at dose levels higher than 30 mg/kg, more closely resembling the pharmacokinetics observed in higher species including humans. The selected 100 mg/kg dose is well above the level where nonlinear pharmacokinetics have been documented. Ten animals were used at weekly intervals between each dosing to allow washout of the drug from the previous dosing. All animals tolerated these doses of elacridar without any problem.

Elacridar suspension formulation was prepared by weighing 100.03 mg of elacridar, which was first suspended in 5 ml of water, followed by 5 ml of vehicle solution (2.5% w/v of hydroxypropyl methyl cellulose (HPMC) and 2% v/v of Tween 80). This suspension was stirred for about 2.5 hours prior to dosing. The final concentration was 10 mg/ml. This suspension was given at 10 µl per gram body weight to achieve a dose level of 100 mg/kg

Elacridar nanoparticle (NP) suspensions were used as received, except for a final 5-fold dilution step to obtain a NP suspension of 10 mg/ml of elacridar. There were two NP formulations: 1) 5% elacridar w/w plus 1% w/w TPGS, where 2 ml of NP formulation was diluted with 8 ml of 20% TPGS to give a final elacridar concentration of 10 mg/ml; and 2) 5% elacridar w/w plus 5% w/w Poloxamer 407, where 2 ml of NP formulation was diluted with 8 ml of water to give a final elacridar concentration of 10 mg/ml.

There were three study groups: 1) Elacridar Suspension (n=4) ; 2) Elacridar TPGS nanoparticles (n=5); and 3) Elacridar Poloxamer nanoparticles (n=5). All animals received elacridar orally by gavage under light isoflurane anesthesia. The animals were fasted for a period of 3 h before drug administration. Food pellets were returned to the cages after the 4 h sampling point. Blood samples from the tail vein at: 15, 30 min, 1, 2, 4, 7, 24, 30 and 48 hours. Samples were kept on ice and centrifuged within 2 h to separate plasma. Plasma was stored at -20 °C.

### B. Pharmacokinetics of intravenous (i.v.) elacridar

Formulation for i.v. administration was prepared by weighing 61.12 mg of elacridar and dissolving this in 2.056 ml of DMSO. The final concentration was 30 mg/ml. Animals (n=4) received 0.133 µl per gram body weight (5 mg/kg). Blood samples were taken from the tail vein at: 5, 15, 30 min, 1, 2, 4, 7, 24, 30 and 48 h.. Samples were kept on ice and centrifuged within 2 h to separate plasma. Plasma was stored at -20 °C.

### C. Therapeutic agent brain penetration studies

The following elacridar nanoparticle suspension was used in these experiements: 5% elacridar w/w plus 1% w/w TPGS, where 2 ml of NP formulation was diluted with 8 ml of 20% TPGS to give a final elacridar concentration of 10 mg/ml.

Imatinib was prepared as follows: 23.26 mg of imatinib mesylate (from Novartis) was diluted in 4.65 ml of saline to a final concentration of 5.0 mg/ml. The drug was mixed, sonicated and administered within 1 h. The dose used was 50 mg/kg (10 πl/g).

Docetaxel (Hospira) was prepared as follows: 10 mg/ml stock was diluted 1:3 in saline to final concentration: 3.3 mg/ml. The drug was mixed by vortex and administered within 1.5 h. The dose used was 33 mg/kg (10 πl/g).

Loperamide was prepared as follows: 1.75 mg of Loperamide hydrochloride (from Sigma-Aldrich) was dissolved in 87 µl of DMSO by vortex-mixing and sonication, and subsequently diluted in saline to final concentration of 1.0 mg/ml. The drug was administered within 1 h of preparation. The dose used was 5 mg/kg (5 πl/g).

Non-transgenic (wildtype) FVB mice, females, aged 8 to 10 weeks were used. Animals received food and water *ad libitum.* Mice in the treatment group (n=5) received elacridar NP formulation orally by gavage in the morning (9-10 am). Mice in the control group (n=5) received no elacridar. All mice (treatment and control group) received the therapeutic agent by i.v. injection in the tail vein in the afternoon (3-4 pm). Blood samples (50 µl) were drawn by bleeding from the tip of the tail at 5 min and 30 min after drug administration. At 1 h after API administration the animals were anesthetized using isoflurane and blood was drawn by cardiac puncture. Next, the animal was killed by cervical dislocation and the brains were harvested. Blood was kept on ice until centrifugation (5 min at 5000g) within 2 h. Brains were kept on ice for weighing and stored at -20 °C until homogenization. Brains were homogenized in 3 ml of 1% w/v of bovine serum albumin in water.

The mice received elacridar at the standard dose of 100 mg/kg. About 6 h later (near the Tmax), the animals received the therapeutic agent (Docetaxel, Imatinib, and Loperamide) by i.v. administration. The i.v. administration route was selected for the therapeutic agent in order to achieve the most similar plasma exposure of the therapeutic agent between animals receiving therapeutic agent alone or therapeutic agent with elacridar.

Dosing of docetaxel and imatinib went without complications in all groups. Dosing of loperamide went smoothly in the control group animals. However, for loperamide and elacridar treated animals, one animal died within 5 minutes after drug administration, whereas another animal had to be sacrificed at 45 min. All others survived the 1 h period until planned sacrifice.

Samples were pre-treated as follows. Plasma (5 to 100 µl) was made up to 100 µl with blank human plasma or brain homogenate (100 µl) and pipetted into a 2 ml eppendorf vial. 50 µl of the internal standard (IS) solution (1000 nM of the deuterated analyte) in acetonitrile:water (30:70; v/v) was added. 1000 µl of diethyl ether was added. Tubes were vigorously mixed for at least 5 min then centrifuged (5 min at 5000g) before being placed in a bath of ethanol with dry-ice in order to freeze the aqueous bottom layer. The organic supernatant was decanted into a clean 1.5 ml Brand vial and evaporated under vacuum in a Speed-Vac (Savant). The residue was reconstituted in 100 µl of acetonitrile:water (20:80; v/v) for imatinib or (30:70 v/v) for the other compounds. An aliquot of 50, 25, 10 and 10 µl for elacridar, docetaxel, imatinib and loperamide samples, respectively, was subjected to HPLC-MS/MS analysis.

For HPLC-MS/MS analyses, the HPLC systems consisted of an Ultimate DGP-3600A pump with a SRD-3600 Solvent Rack and a model WPS-3000TSL autosampler (Dionex, Sunnyvale, CA, USA). The HPLC column (100 x 2.1 mm) was packed with 3 um C18 Extend material. The column effluent was guided to an electrospray ionization (ESI) source of an API3000 mass spectrometer (ABSciex). The settings of the MS are listed in Table 10 below. The mobile phase composition was: Mobile phase A was 0.1% formic acid in water; and Mobile phase B was Methanol (LC-MS quality, Merck). Chromatographic conditions for each of the compounds were: 1) Elacridar: Gradient from 0-2 min, 45 to 95%B, 2-4 min: 95%B, 4-4.5 min: 95 to 45%B. Internal standard: Elacridar-d4 (Toronto Research Chemicals); 2) Imatinib: Gradient from 0-2 min, 30 to 95%B, 2-4 min: 95%B, 4-4.5 min: 95 to 30%B. Internal standard: Imatinib-d8 (gift of Novartis); 3) Docetaxel: Isocratic elution at 75%B. Internal standard: Docetaxel-d6 (Toronto Research Chemicals); and 4) Loperamide: Gradient from 0-2 min, 45 to 95%B, 2-4 min: 95%B, 4-4.5 min: 95 to 45%B. Internal standard: Lopermide-d6 (Toronto Research Chemicals).

Pharmacokinetic analyses were performed using the Microsoft Excel add-in program PKsolver (see e.g., Ward KW et al., J Pharmacol Exp Ther 2004; 310: 703-9, which is incorporated herein in its entirety). Calculations for oral administrations were done by the non-compartmental analyses - extravascular model, whereas we used the non-compartmental analysis - bolus model for i.v. injections. The maximum plasma levels (Cmax) were calculated by comparing the means of the plasma levels at each time point. The Tmax is considered the time point at which the Cmax was reached. The half-life (T1/2) was calculated from the final log-linear part of the plasma concentration - time curves. The area-under-the-curve for the plasma concentration (plasma AUC) was calculated using the linear trapezoidal rule. Both the AUC from time = 0 to the last sampling point (48 h) as well as the AUC extrapolated to infinity was calculated. The oral bioavailability was calculated based on the AUC(0-inf) values. Student t-test was used to compare the means of groups where applicable.

### Results

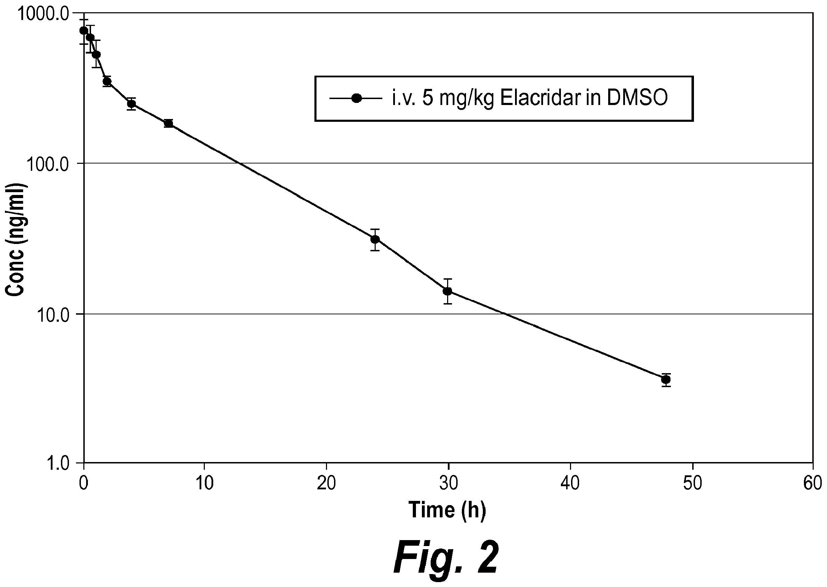

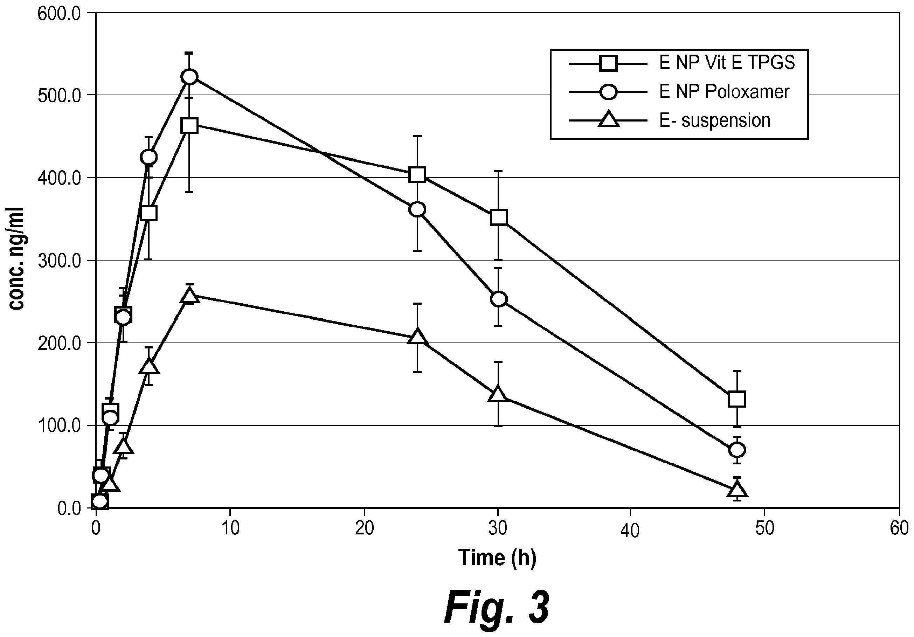

The pharmacokinetics of elacridar after i.v. administration (in DMSO) or p.o. administration (as nanoparticles or conventional suspension) are set forth in Table 11, Figure 2 and Figure 3. The plasma Cmax levels were reached at 7 h (Tmax) after oral drug administration with all three formulations. The plasma Cmax level of both novel nanoparticle formulations was significantly (*p* < 0.05) higher than with the suspension formulation. Similarly, the plasma AUC values were higher with both novel nanoparticle formulations. The plasma AUC₍₀₋₄₈ₕ₎ was more than 95% of the AUC_{(0-inf)} indicating a good coverage of the complete plasma curve by the selected time points for drug measurements. The oral bioavailability of elacridar was calculated using the plasma AUC_{(0-inf)} obtained after i.v. dosing of 5 mg/kg of DMSO solubilized elacridar. The oral bioavailability of the elacridar suspension was only 8.5% (0.085), this increased to 17.2 % (0.172) using the poloxamer elacridar nanoparticle formulation, and to 20.7% using the TPGS elacridar nanoparticle formulation. From these data it is clear that the elacridar nanoparticle formulations disclosed herein have superior pharmacokinetic properties to the suspensions of elacridar.

The results of experiments to determine the effect of the of the elacridar nanoparticle (NP) formulations on the brain penetration of three different therapeutic agents (Docetaxel, Imatinib, and Loperamide) in wildtype FVB mice are set forth in Tables 12-23, herein.

The systemic exposure of imatinib, docetaxel and loperamide in plasma was calculated by the AUC from 5 min until 1 h after drug administration. The plasma AUC of imatinib was not changed by concomitant elacridar dosing (Table 13 and 14), yet the brain penetration increased significantly by 14-fold from 1094 to 15582 ng/g (Table X 12; p < 0.0000001). The corresponding elacridar plasma levels at 5 min and 1 h after imatinib administration were 842 and 694 ng/ml respectively (Table 15). Docetaxel brain penetration was 3-fold increased from 370 to 1157 ng/g (*p* < 0.000001; Table 16), while plasma AUC remained unchanged by concomitant elacridar NP administration (Table 17 and 18). The corresponding elacridar plasma levels at 5 min and 1 h after Docetaxel administration were 3114 and 801 ng/ml respectively (Table 19). Loperamide brain penetration was 50-fold increased from 73 to 3698 ng/g (*p* < 0.00001; Table 20), while plasma AUC remained unchanged by concomitant elacridar NP administration (Table 21 and 22). The corresponding elacridar plasma levels 5 min and 1 h after Loperamide administration were 1137 and 972 ng/ml respectively (Table 23). From these data, it is clear that the novel nanoparticle formulations are effective at increasing the penetration of therapeutic agents of varying classes into the brain.

**Table 10 Parameter settings of the Mass Spectrometer**

| **Analyte** | **Docetaxel** | **Docetaxel-d9** | **Elacridar** | **Eladridar-d4** | **Imatinib** | **Imatinib-d8** | **Loperamide** | **Loperamide-d6** |
|---|---|---|---|---|---|---|---|---|
| **Product ion of** | 808.5 | 817.5 | 564.4 | 568.4 | 494.4 | 502.4 | 477.3 | 483.1 |
| **Primary product ion** | 527.5 | 527.5 | 252.1 | 252.1 | 394.4 | 394.4 | 266.3 | 272.1 |
| **Neb gas** | 8 | 8 | 8 | 8 | 8 | 8 | 8 | 8 |
| **Curtain gas** | 8 | 8 | 8 | 8 | 8 | 8 | 8 | 8 |
| **Ion spray** | 5000 | 5000 | 5000 | 5000 | 5000 | 5000 | 5000 | 5000 |
| **Temperature** | 300 | 300 | 300 | 300 | 300 | 300 | 300 | 300 |
| **Declustering potential** | 50 | 50 | 65 | 65 | 65 | 65 | 53 | 53 |
| **Focusing potential** | 180 | 180 | 150 | 150 | 180 | 180 | 160 | 160 |
| **Entrance potential** | 10 | 10 | 10 | 10 | 10 | 10 | 10 | 10 |
| **Collision energy** | 15 | 15 | 42 | 42 | 42 | 42 | 35 | 35 |
| **Collision cell exit pot.** | 16 | 16 | 20 | 20 | 20 | 20 | 24 | 24 |

**Table 11 Pharmacokinetic parameters of elacridar formulations in Sprague-Dawley rats**

| **Formulation** | **Dose (mg/kg)** | **route** | | **Cmax (ng/ml)** | **Tmax (h)** | **T1/2 (h)** | **AUC(0-48h) (min*µg/ml)** | **AUC(0-inf) (min*µg/ml)** | **F (%)** |
|---|---|---|---|---|---|---|---|---|---|
| DMSO | 5 | i.v. | Mean | 826.5 | 0 | 6.9 | 260.3 | 272.2 | 100,0% |
| | | | SE | | -- | | 16.3 | | |
| Elacridar suspension | 100 | p.o. | Mean | 258.0 | 7 | 7.6 | 442,8 | 458.2 | 8,4% |
| | | | SE | 10.8 | -- | | 56.7 | | |
| Elacridar NP 16.2% Vit E TPGS | 100 | p.o. | Mean | 465.4 | 7 | 14.1 | 965.1 | 1125.2 | 20,7% |
| | | | SE | 85.3 | -- | | 83.3 | | |
| Elacridar NP 5% Poloxamer diluted1:5 in water | 100 | p.o. | Mean | 522.7 | 7 | 10.0 | 874.5 | 934.3 | 17,2% |
| | | | SE | 26.0 | -- | | 84.7 | | |

**Table 12 Brain penetration of imatinib**

| Animal# | Conc | mean | SD | n | SE |
|---|---|---|---|---|---|
| | ng/g | | | | |
| 1135470-brain | 1049 | | | | |
| 1135471-brain | 1125 | | | | |
| 1135472-brain | 1642 | | | | |
| 1135473-brain | 630 | | | | |
| 1135474-brain | 1022 | 1094 | 362 | 5 | 162 |
| | | | | | |
| 1135500-brain | 15164 | | | | |
| 1135501-brain | 13507 | | | | |
| 1135502-brain | 16055 | | | | |
| 1135503-brain | 16974 | | | | |
| 1135504-brain | 16209 | 15582 | 1326 | 5 | 593 |

**Table 13 Plasma concentration of imatinib in FVB mice receiving imatinib (alone)**

| Imatinib concentration (ng/ml) | | | | | | | | | | |
|---|---|---|---|---|---|---|---|---|---|---|
| | | | | | | | | | | |
| Animal# | 1135470 | 1135471 | 1135472 | 1135473 | 1135474 | | mean | SD | n | SE |
| time (min) | | | | | | | | | | |
| 5 | 49100 | 50400 | 38200 | 61300 | 48900 | | 49580.0 | 8188.8 | 5 | 3662.2 |
| 30 | 6590 | 11500 | 7690 | 13200 | 11600 | | 10116.0 | 2826.1 | 5 | 1263.9 |
| 60 | 1920 | 1260 | 2350 | 2050 | 2760 | | 2068.0 | 555.3 | 5 | 248.3 |
| AUC(0-1h) | 946525 | 1071650 | 983225 | 1130250 | 1096900 | | 1045710 | 77753. 4 | 5 | 34772.4 |
| min x ng/ml | | | | | | | | | | |

**Table 14 Plasma concentration of imatinib in FVB mice receiving imatinib with elacridar**

| Imatinib concentration (ng/ml) | | | | | | | | | | |
|---|---|---|---|---|---|---|---|---|---|---|
| Animal# | 1135500 | 1135501 | 1135502 | 1135503 | 1135504 | | mean | SD | n | SE |
| time (min) | | | | | | | | | | |
| 5 | 90800 | 43500 | 50000 | 34400 | 37900 | | 51320.0 | 22847. 0 | 5 | 10217.5 |
| 30 | 11000 | 7860 | 11700 | 8910 | 12600 | | 10414.0 | 1972.4 | 5 | 882.1 |
| 60 | 3630 | 5580 | 3840 | 4710 | 4100 | | 4372.0 | 787.6 | 5 | 352.2 |
| AUC(0-1h) | 1093450 | 1036350 | 1115850 | 1052175 | 1144500 | | 1088465 | 44574. 8 | 5 | 19934.5 |
| min x ng/ml | | | | | | | | | | |

**Table 15 Plasma concentration of elacridar in FVB mice receiving elacridar + imatinib**

| Elacridar concentration (ng/ml) | | | | | | | | | | |
|---|---|---|---|---|---|---|---|---|---|---|
| | | | | | | | | | | |
| Animal# | 1135500 | 1135501 | 1135502 | 1135503 | 1135504 | | mean | SD | n | SE |
| time (min) | | | | | | | | | | |
| 5 min | 970 | 812 | 733 | 891 | 801 | | 841.5 | 91.2 | 5 | 40.8 |
| 1 h | 649 | 694 | 626 | 784 | 716 | | 693.7 | 61.8 | 5 | 27.6 |

**Table 16 Brain penetration of docetaxel**

| Animal# | Conc | mean | SD | n | SE |
|---|---|---|---|---|---|
| | ng/g | | | | |
| 1135452-brain | 427 | | | | |
| 1135453-brain | 362 | | | | |
| 1135454-brain | 440 | | | | |
| 1135455-brain | 323 | | | | |
| 1135456-brain | 298 | 369.9 | 62.5 | 5 | 27.9 |
| | | | | | |
| 1135447-brain | 1230 | | | | |
| 1135448-brain | 1105 | | | | |
| 1135449-brain | 1297 | | | | |
| 1135450-brain | 1133 | | | | |
| 1135451-brain | 1018 | 1156.5 | 108.9 | 5 | 48.7 |

**Table 17 Plasma concentration of docetaxel in FVB mice receiving docetaxel (alone)**

| Docetaxel concentration (ng/ml) | | | | | | | | | | |
|---|---|---|---|---|---|---|---|---|---|---|
| | | | | | | | | | | |
| Animal# | 1135452 | 1135453 | 1135454 | 1135455 | 1135456 | | mean | SD | n | SE |
| time (min) | | | | | | | | | | |
| 5 | 188000 | 218000 | 245000 | 253000 | 127000 | | 206200 | 51085.2 | 5 | 22846. 0 |
| 30 | 13900 | 11300 | 19900 | 8170 | 7940 | | 12242 | 4933.8 | 5 | 2206.5 |
| 60 | 3030 | 2540 | 3300 | 2190 | 1770 | | 2566 | 618.4 | 5 | 276.6 |
| AUC(0-1 h) | 3247700 | 3168850 | 3416750 | 3077525 | 3064900 | | 3195145 | 144361.1 | 5 | 64560. 2 |
| min x ng/ml | | | | | | | | | | |

**Table 18 Plasma concentration of docetaxel in FVB mice receiving docetaxel with elacridar**

| Docetaxel concentration (ng/ml) | | | | | | | | | | |
|---|---|---|---|---|---|---|---|---|---|---|
| | | | | | | | | | | |
| Animal# | 1135447 | 1135448 | 1135449 | 1135450 | 1135451 | | mean | SD | n | SE |
| time (min) | | | | | | | | | | |
| 5 | 168000 | 157000 | 123000 | 131000 | 153000 | | 146400 | 18756.3 | 5 | 8388.1 |
| 30 | 11200 | 12700 | 11600 | 15900 | 13500 | | 12980 | 1867.4 | 5 | 835.1 |
| 60 | 2880 | 2440 | 2730 | 2460 | 2600 | | 2622 | 185.8 | 5 | 83.1 |
| AUC(0-1 h) | 3171200 | 3205850 | 3179950 | 3294150 | 3230250 | | 3216280 | 49294.4 | 5 | 22045. 1 |
| min x ng/ml | | | | | | | | | | |

**Table 19 Plasma concentration of elacridar in FVB mice receiving elacridar + docetaxel**

| Elacridar concentration (ng/ml) | | | | | | | | | | |
|---|---|---|---|---|---|---|---|---|---|---|
| | | | | | | | | | | |
| Animal# | 1135447 | 1135448 | 1135449 | 1135450 | 1135451 | | mean | SD | n | SE |
| time (min) | | | | | | | | | | |
| 5 | 3672 | 3034 | 3345 | 2871 | 2651 | | 3114.4 | 401.3 | 5 | 179.5 |
| 60 | 1111 | 660 | 925 | 750 | 559 | | 801.0 | 219.4 | 5 | 98.1 |

**Table 20 Brain penetration of loperamide**

| Animal# | Conc | mean | SD | n | SE |
|---|---|---|---|---|---|
| | ng/g | | | | |
| 1143312-brain | 79.4 | | | | |
| 1143313-brain | 84.1 | | | | |
| 1143314-brain | 78.1 | | | | |
| 1143315-brain | 41.6 | | | | |
| 1143316-brain | 83.8 | 73.4 | 18.0 | 5.0 | 8.0 |
| | | | | | |
| 1142188-brain | 2909.2 | | | | |
| 1142189-brain | 4376.5 | | | | |
| 1142191-brain | 4288.7 | | | | |
| 1142192-brain | 3981.2 | | | | |
| 1142193-brain | 2933.5 | 3697.8 | 723.9 | 5.0 | 323.7 |

**Table 21 Plasma concentration of loperamide in FVB mice receiving loperamide (alone)**

| Loperamide concentration (ng/ml) | | | | | | | | | | |
|---|---|---|---|---|---|---|---|---|---|---|
| | | | | | | | | | | |
| Animal# | 1143312 | 1143313 | 1143314 | 1143315 | 1143316 | | mean | SD | n | SE |
| time (min) | | | | | | | | | | |
| 5 | 2500.0 | 430.0 | 599.0 | 679.0 | 925.0 | | 1026.6 | 842.7 | 5 | 376.9 |
| 30 | 191.0 | 166.0 | 168.0 | 354.0 | 226.0 | | 221.0 | 78.2 | 5 | 35.0 |
| 60 | 111.0 | 87.5 | 88.0 | 26.0 | 94.5 | | 81.4 | 32.4 | 5 | 14.5 |
| AUC(0-1 h) | 44417.5 | 43377.5 | 43440.0 | 47625.0 | 45132.5 | | 44798.5 | 1740.0 | 5 | 778.2 |
| min x ng/ml | | | | | | | | | | |

**Table 22 Plasma concentration of loperamide in FVB mice receiving loperamide with elacridar**

| Loperamide concentration (ng/ml) | | | | | | | | | | |
|---|---|---|---|---|---|---|---|---|---|---|
| | | | | | | | | | | |
| Animal# | 1142188 | 1142189 | 1142191 | 1142192 | 1142193 | | mean | SD | n | SE |
| time (min) | | | | | | | | | | |
| 5 | 1220.0 | 306.0 | 376.0 | 511.0 | 2530.0 | | 988.6 | 935.3 | 5 | 418.3 |
| 30 | -- | 115.0 | 178.0 | 163.0 | 282.0 | | 184.5 | 70.3 | 4 | 35.2 |
| 60 | -- | 106.0 | 134.0 | 270.5 | 48.3 | | 139.7 | 94.2 | 4 | 47.1 |
| AUC(0-1h) | -- | 42252.5 | 44405.0 | 46040.0 | 45978.8 | | 44669.1 | 1779.9 | 4 | 890.0 |
| min x ng/ml | | | | | | | | | | |

**Table 23 Plasma concentration of elacridar in FVB mice receiving elacridar + loperamide**

| Elacridar concentration (ng/ml) | | | | | | | | | | |
|---|---|---|---|---|---|---|---|---|---|---|
| | | | | | | | | | | |
| Animal# | 1142188 | 1142189 | 1142191 | 1142192 | 1142193 | | mean | SD | n | SE |
| time (min) | | | | | | | | | | |
| 5 | 1765 | 917 | 835 | 939 | 1230 | | 1137 | 382 | 5 | 171 |
| 60 | -- | 914 | 760 | 1324 | 888 | | 972 | 244 | 4 | 122 |

## Claims

1. A composition comprising a nanoparticle formulation of elacridar" wherein the elacridar achieves one or more of:
a. a Cmax of at least 500ng/ml;
b. bioavailability of at least 0.2;
c. an AUC(0-48h) of at least 900 ug/ml*min;
d. an AUC(0-∞) of at least 1100 ug/ml*min; and
e. an elimination half-life (T1/2) of at least 10 h;
when the composition is administered by oral gavage at 100 mg/kg to fasted, female Sprague-Dawley rats.

2. The composition of claim 1, wherein the composition comprises at least about 1% elacridar, weight/weight (w/w).

3. The composition of claim 1 or claim 2, wherein the composition or nanoparticle formulation further comprises a permeation enhancer, such as a permeation enhancer selected from the group consisting of D-α-Tocopherol polyethylene glycol succinate (TPGS), dioctyl sodium sulfosuccinate, sodium caprate, sodium N-[8(-2-hydroxybenzoyl)amino]caprylate (SNAC), sodium lauryl sulfate, sodium salicylate, oleic acid, lecithin, dehydrated alcohol, Tween, Span, polyoxyl 40 stearate, polyoxy ethylene 50 stearate, polyethylene glycol, polyvinyl alcohol, polyvinylpyrrolidone , hydroxy propyl methyl cellulose, polyvinylpyrrolidone/vinyl acetate (VP/VA) copolymer, poly(lactic-co-glycolic acid), edetate disodium, propylene glycol, glycerol monooleate, fusieates, bile salts, octoxynol, non-ionic surfactants, anionic surfactants and cationic surfactants.

4. The composition of claim 3, wherein the permeation enhancer is TPGS, preferably wherein the composition comprises at least about 1%, 5% or 16% TPGS w/w.

5. The composition of any one of claims 1 to 4, wherein the composition further comprises a solubility enhancer, such as a solubility enhancer selected from TPGS, polyethylene glycol 300, polyethylene glycol 400, ethanol, propylene glycol, glycerin, N-methyl-2-pyrrolidone, dimethylacetamide, and dimethylsulfoxide, Cremophor EL, Cremophor RH 40, Cremophor RH 60, polysorbate 20, polysorbate 80, Solutol HS 15, sorbitan monooleate, poloxamer 407, Labrafil M-1944CS, Labrafil M-2125CS, Labrasol, Gellucire 44/14, Softigen 767, mono- and di-fatty acid esters of PEG 300, 400, or 1750, water-insoluble lipids, organic liquids/semi-solids, and cyclodextrins.

6. The composition of claim 5, wherein the solubility enhancer is poloxamer 407, preferably wherein the composition comprises at least about 5% poloxamer 407 w/w.

7. A composition comprising a nanoparticle formulation of elacridar, wherein the nanoparticle formulation comprises elacridar and TPGS, preferably wherein the nanoparticle formulation comprises about 5% elacridar and about 1% TPGS w/w.

8. The composition of claim 7, wherein the nanoparticle formulation is diluted in a TPGS aqueous solution to a final concentration of at least 16% TPGS.

9. A composition comprising a nanoparticle formulation of elacridar, wherein the nanoparticle formulation comprises elacridar and poloxamer 407, preferably wherein the nanoparticle formulation comprises about 5% elacridar and about 5% poloxamer 407 w/w.

10. The composition of claim 9, wherein the nanoparticle formulation is diluted in an aqueous solvent.

11. The composition of any one of claims 1-10, wherein the composition or nanoparticle formulation further comprises a therapeutic agent, wherein the therapeutic agent is a modulator of a biological target, preferably wherein the biological target is selected from one or more members of the group consisting of enzymes, receptors, ion channels, nucleic acids, ribosomes, hormones, vitamins, cytokines, chemokines, substrates, metabolites, proteins, transport molecules, physiochemical mechanisms, and antigen-antibody interactions.

12. The composition of claim 11, wherein the therapeutic agent is a kinase inhibitor, such as a kinase inhibitor selected from the group consisting of ABT-869, afatinib (BIBW-2992), AMG-706, AMN-107, amuvatinib, AST-487, axitinib (AG-013736), AZD-152HQPA, AZD-2171, BIBF-1120, BIRB-796, BMS-540215, bosutinib, cabozantinib, canertinib (CI-1 033), CHIR-258/TKI-258, crizotinib, dasatinib, DMBI, dovitinib, erlotinib, everolimus, EXEL-2880/GSK-1363089, gefitinib, GW-786034, imatinib, JNJ-28312141, Ki-20227, Ki8751, lapatinib, masitinib (AB-1010), midostaurin (PKC-412), motesanib, neratinib (HKI-272), nilotinib, OSI-930, pazopanib, PD-173955, PLX-4720, ponatinib, PTK-787, quizartinib (AC220), R406, regorafenib, SKI-606, sorafenib, staurosporine, SU-14813, sunitinib, tandutinib (MLN-518), telatinib, temsirolimus, tivozanib, vandetanib, vatalanib, and vemurafenib.

13. A composition of any one of claims 1-12 for use in treating a condition wherein treatment with a therapeutic agent is inhibited by BCRP and/or P-GP activity, in combination with a therapeutic agent useful for treating the condition, wherein the composition increases the concentration of the therapeutic agent in the target tissue or cell relative to administration of the therapeutic agent alone.

14. The composition for use of claim 13, wherein the condition is a neurological condition, such as a neurological condition selected from neurofibromatosis; neuro-cardio-facial-cutaneous syndromes; primary brain cancer, such as gliobastoma multiforme; secondary brain metastasis, such as brain metastasis from breast cancer or lung cancer; multiple sclerosis; amyotrophic lateral scelerosis and Alzheimer's disease.

15. The composition for use of claim 13 or 14, wherein the composition and the therapeutic agent are administered simultaneously to the subject either (i) via separate routes of administration or (ii) in a single composition.

## Patentansprüche

1. Zusammensetzung, umfassend eine Nanopartikelformulierung von Elacridar, wobei das Elacridar eines der folgenden bewirkt:
a. eine Cmax. von mindestens 500 ng/ml;
b. Bioverfügbarkeit von mindestens 0,2;
c. eine AUC(0-48h) von mindestens 900 ug/ml*Min;
d. eine AUC(0-∞) von mindestens 1100 ug/ml*Min; und
e. eine Eliminationshalbwertszeit (T1/2) von mindestens 10 h;
wenn die Zusammensetzung nüchternen, weiblichen Sprague-Dawley-Ratten per oraler Sonde in einer Dosis von 100 mg/kg verabreicht wird.

2. Zusammensetzung nach Anspruch 1, wobei die Zusammensetzung mindestens etwa 1 % Elacridar, Gewicht/Gewicht (Gew./Gew.) umfasst.

3. Zusammensetzung nach Anspruch 1 oder 2, wobei die Zusammensetzung oder Nanopartikelformulierung ferner einen Permeationsverstärker umfasst, z. B. einen Permeationsverstärker ausgewählt aus der Gruppe, die aus D-α-Tocopherolpolyethylenglycolsuccinat (TPGS), Dioctylnatriumsulfosuccinat, Natriumcaprat, Natrium-N-[8(-2-hydroxybenzoyl)amino]caprylat (SNAC), Natriumlaurylsulfat, Natriumsalicylat, Ölsäure, Lecithin, dehydriertem Alkohol, Tween, Span, Polyoxyl-40-stearat, Polyoxyethylen-50-stearat, Polyethylenglycol, Polyvinylalkohol, Polyvinylpyrrolidon, Hydroxypropylmethylcellulose, Polyvinylpyrrolidon-/Vinylacetat-(VP/VA)-Copolymer, Poly(milch-co-glycolsäure), Dinatriumedetat, Propylenglycol, Glycerolmonooleat, Fusieaten, Gallensalzen, Octoxynol, nichtionischen Tensiden, anionischen Tensiden und kationischen Tensiden besteht.

4. Zusammensetzung nach Anspruch 3, wobei der Permeationsverstärker TPGS ist, vorzugsweise wobei die Zusammensetzung mindestens etwa 1 %, 5 % oder 16 % TPGS Gew./Gew. umfasst.

5. Zusammensetzung nach einem der Ansprüche 1 bis 4, wobei die Zusammensetzung ferner einen Löslichkeitsverstärker umfasst, z. B. einen Löslichkeitsverstärker ausgewählt aus TPGS, Polyethylenglycol 300, Polyethylenglycol 400, Ethanol, Propylenglycol, Glycerin, N-methyl-2-pyrrolidon, Dimethylacetamid und Dimethylsulfoxid, Cremophor EL, Cremophor RH 40, Cremophor RH 60, Polysorbat 80, Solutol HS 15, Sorbitanmonooleat, Poloxamer 407, Labrafil M-1944CS, Labrafil M-2125CS, Labrasol, Gellucire 44/14, Softigen 767, Mono- und Difettsäureestern von PEG 300, 400 oder 1750, wasserunlöslichen Lipiden, organischen Flüssigkeiten/halbfesten Stoffen und Cyclodextrinen.

6. Zusammensetzung nach Anspruch 5, wobei der Löslichkeitsverstärker Poloxamer 407 ist, vorzugsweise wobei die Zusammensetzung mindestens etwa 5 % Poloxamer 407 Gew./Gew. umfasst.

7. Zusammensetzung, umfassend eine Nanopartikelformulierung von Elacridar, wobei die Nanopartikelformulierung Elacridar und TPGS umfasst, vorzugsweise wobei die Nanopartikelformulierung etwa 5 % Elacridar und etwa 1 % TPGS Gew./Gew. umfasst.

8. Zusammensetzung nach Anspruch 7, wobei die Nanopartikelformulierung in einer wässrigen TPGS-Lösung bis zu einer Endkonzentration von mindestens 16 % TPGS verdünnt wird.

9. Zusammensetzung, umfassend eine Nanopartikelformulierung von Elacridar, wobei die Nanopartikelformulierung Elacridar und Poloxamer 407 umfasst, vorzugsweise wobei die Nanopartikelformulierung etwa 5 % Elacridar und etwa 5 % Poloxamer 407 Gew./Gew. umfasst.

10. Zusammensetzung nach Anspruch 9, wobei die Nanopartikelformulierung in einem wässrigen Lösungsmittel verdünnt wird.

11. Zusammensetzung nach einem der Ansprüche 1-10, wobei die Zusammensetzung oder Nanopartikelformulierung ferner ein Therapeutikum umfasst, wobei das Therapeutikum ein Modulator einer biologischen Zielsubstanz ist, vorzugsweise wobei die biologische Zielsubstanz aus einem oder mehreren Bestandteilen der Gruppe ausgewählt ist, die aus Enzymen, Rezeptoren, Ionenkanälen, Nukleinsäuren, Ribosomen, Hormonen, Vitaminen, Cytokinen, Chemokinen, Substraten, Metaboliten, Proteinen, Transportmolekülen, physiochemischen Mechanismen und Antigen-Antikörper-Wechselwirkungen besteht.

12. Zusammensetzung nach Anspruch 11, wobei das Therapeutikum ein Kinaseinhibitor ist, z. B. ein Kinaseinhibitor ausgewählt aus der Gruppe, die aus ABT-869, Afatinib (BIBW-2992), AMG-706, AMN-107, Amuvatinib, AST-487, Axitinib (AG-013736), AZD-152HQPA, AZD-2171, BIBF-1120, BIRB-796, BMS-540215, Bosutinib, Cabozantinib, Canertinib (CI-1033), CHIR-258/TKI-258, Crizotinib, Dasatinib, DMBI, Dovitinib, Erlotinib, Everolimus, EXEL-2880/GSK-1363089, Gefitinib, GW-786034, Imatinib, JNJ-28312141, Ki-20227, Ki8751, Lapatinib, Masitinib (AB-1010), Midostaurin (PKC-412), Motesanib, Neratinib (HKI-272), Nilotinib, OSI-930, Pazopanib, PD-173955, PLX-4720, Ponatinib, PTK-787, Quizartinib (AC220), R406, Regorafenib, SKI-606, Sorafenib, Staurosporin, SU-14813, Sunitinib, Tandutinib (MLN-518), Telatinib, Temsirolimus, Tivozanib, Vandetanib, Vatalanib und Vemurafenib besteht.

13. Zusammensetzung nach einem der Ansprüche 1-12 zum Verwenden in der Behandlung einer Erkrankung, wobei die Behandlung mit einem Therapeutikum durch BCRP- und/oder P-GP-Aktivität gehemmt wird, in Kombination mit einem für die Behandlung der Erkrankung geeigneten Therapeutikum, wobei die Zusammensetzung die Konzentration des Therapeutikums im Zielgewebe oder in der Zielzelle relativ zur alleinigen Verabreichung des Therapeutikums erhöht.

14. Zusammensetzung nach Anspruch 13, wobei die Erkrankung eine neurologische Erkrankung ist, z. B. eine neurologische Erkrankung ausgewählt aus Neurofibromatose; Neuro-kardio-faziokutanen Syndromen; primären Gehirntumoren, wie z. B. Glioblastoma multiforme; sekundären Gehirnmetastasen, wie z. B. Gehirnmetastasen bei Brust- oder Lungenkrebs; Multipler Sklerose; Amyotropher Lateralsklerose und Alzheimer-Krankheit.

15. Zusammensetzung zum Verwenden nach Anspruch 13 oder 14, wobei die Zusammensetzung und das Therapeutikum dem Probanden gleichzeitig entweder (i) über getrennte Verabreichungsrouten oder (ii) in einer einzelnen Zusammensetzung verabreicht wird.

## Revendications

1. Composition comprenant une formulation nanoparticulaire d'élacridar, dans laquelle l'élacridar atteint une ou plusieurs des suivantes :
a. une Cmax d'au moins 500 ng/ml ;
b. une biodisponibilité d'au moins 0,2 ;
c. une AUC(0-48 h) d'au moins 900 µg/ml*min ;
d. une AUC(0-∞) d'au moins 1 100 µg/ml*min ; et
e. une demi-vie d'élimination (T1/2) d'au moins 10 h ;
dans laquelle la composition est administrée par gavage oral à 100 mg/kg à des rats Sprague-Dawley femelles à jeun.

2. Composition selon la revendication 1, dans laquelle la composition comprend au moins environ 1 % d'élacridar, poids/poids (p/p).

3. Composition selon la revendication 1 ou la revendication 2, dans laquelle la composition ou la formulation nanoparticulaire comprend en outre un amplificateur de la perméation, tel qu'un amplificateur de la perméation choisi dans le groupe constitué de succinate de D-α-tocophérol polyéthylène glycol (TPGS), dioctyl-sulfosuccinate de sodium, caprate de sodium, N-[8(-2-hydroxybenzoyl)amino]caprylate de sodium (SNAC), laurylsulfate de sodium, salicylate de sodium, acide oléique, lécithine, alcool déshydraté, Tween, Span, stéarate de polyoxyéthylène 40, stéarate de polyoxy-éthylène 50, polyéthylène glycol, polyalcool vinylique, polyvinylpyrrolidone, hydroxypropylméthylcellulose, copolymère de polyvinylpyrrolidone/acétate de vinyle (VP/VA), poly(acide lactique-co-glycolique), édétate disodique, propylène glycol, monooléate de glycérol, fusidates, sels biliaires, octoxynol, tensioactifs non ioniques, tensioactifs anioniques et tensioactifs cationiques.

4. Composition selon la revendication 3, dans laquelle l'amplificateur de la perméation est le TPGS, de préférence dans laquelle la composition comprend au moins environ 1 %, 5 % ou 16 % de TPGS p/p.

5. Composition selon l'une quelconque des revendications 1 à 4, dans laquelle la composition comprend en outre un amplificateur de solubilité, tel qu'un amplificateur de solubilité choisi parmi le TPGS, le polyéthylène glycol 300, le polyéthylène glycol 400, l'éthanol, le propylène glycol, la glycérine, la N-méthyl-2-pyrrolidone, le diméthylacétamide, et le sulfoxyde de diméthyle, Crémophor EL, Crémophor RH 40, Crémophor RH 60, le polysorbate 20, le polysorbate 80, Solutol HS 15, le monooléate de sorbitane, le poloxamer 407, Labrafil M-1944CS, Labrafil M-2125CS, Labrasol, Gellucire 44/14, Softigen 767, les mono- et di-esters d'acides gras de PEG 300, 400, ou 1750, les lipides insolubles dans l'eau, les liquides/semi-solides organiques, et les cyclodextrines.

6. Composition selon la revendication 5, dans laquelle l'amplificateur de solubilité est le poloxamer 407, de préférence dans laquelle la composition comprend au moins environ 5 % de poloxamer 407 p/p.

7. Composition comprenant une formulation nanoparticulaire d'élacridar, dans laquelle la formulation nanoparticulaire comprend de l'élacridar et du TPGS, de préférence dans laquelle la formulation nanoparticulaire comprend environ 5 % d'élacridar et environ 1 % de TPGS p/p.

8. Composition selon la revendication 7, dans laquelle la formulation nanoparticulaire est diluée dans une solution aqueuse de TPGS jusqu'à une concentration finale d'au moins 16 % de TPGS.

9. Composition comprenant une formulation nanoparticulaire d'élacridar, dans laquelle la formulation nanoparticulaire comprend de l'élacridar et du poloxamer 407, de préférence dans laquelle la formulation nanoparticulaire comprend environ 5 % d'élacridar et environ 5 % de poloxamer 407 p/p.

10. Composition selon la revendication 9, dans laquelle la formulation nanoparticulaire est diluée dans un solvant aqueux.

11. Composition selon l'une quelconque des revendications 1 à 10, dans laquelle la composition ou la formulation nanoparticulaire comprend un agent thérapeutique, dans laquelle l'agent thérapeutique est un modulateur d'une cible biologique, de préférence dans laquelle la cible biologique est choisie parmi un ou plusieurs membres du groupe constitué d'enzymes, de récepteurs, de canaux ioniques, d'acides nucléiques, de ribosomes, d'hormones, de vitamines, de cytokines, de chimiokines, de substrats, de métabolites, de protéines, de molécules de transport, de mécanismes physiochimiques, et d'interactions antigène-anticorps.

12. Composition selon la revendication 11, dans laquelle l'agent thérapeutique est un inhibiteur de kinase, tel qu'un inhibiteur de kinase choisi dans le groupe constitué de l'ABT-869, d'afatinib (BIBW-2992), de l'AMG-706, de l'AMN-107, d'amuvatinib, de l'AST-487, d'axitinib (AG-013736), de l'AZD-152HQPA, de l'AZD-2171, du BIBF-1120, du BIRB-796, du BMS-540215, du bosutinib, du cabozantinib, du canertinib (CI-1033), du CHIR-258/TKI-258, du crizotinib, du dasatinib, du DMBI, du dovitinib, de l'erlotinib, de l'évérolimus, de l'EXEL-2880/GSK-1363089, du géfitinib, du GW-786034, de l'imatinib, du JNJ-28312141, du Ki-20227, du Ki8751, du lapatinib, du masitinib (AB-1010), du midostaurine (PKC-412), du motésanib, du nératinib (HKI-272), du nilotinib, de l'OSI-930, du pazopanib, du PD-173955, du PLX-4720, du ponatinib, du PTK-787, du quizartinib (AC220), du R406, du régorafénib, du SKI-606, du sorafénib, du staurosporine, du SU-14813, du sunitinib, du tandutinib (MLN-518), du télatinib, du temsirolimus, du tivozanib, du vandétanib, du vatalanib et du vémurafénib.

13. Composition selon l'une quelconque des revendications 1 à 12 pour une utilisation dans le traitement d'une pathologie dans laquelle le traitement avec un agent thérapeutique est inhibé par l'activité de la BCRP et/ou de la P-GP, en combinaison avec un agent thérapeutique utile pour le traitement de la pathologie, dans laquelle la composition augmente la concentration de l'agent thérapeutique dans le tissu ou la cellule cible par rapport à une administration de l'agent thérapeutique seul.

14. Composition pour une utilisation selon la revendication 13, dans laquelle la pathologie est une pathologie neurologique, telle qu'une pathologie neurologique choisie parmi la neurofibromatose ; les syndromes neuro-cardio-facio-cutanés ; le cancer du sein primitif, tel que le glioblastome multiforme ; les métastases cérébrales secondaires, telles que les métastases cérébrales issues du cancer du sein ou du cancer du poumon ; la sclérose en plaques ; la sclérose latérale amyotrophique et la maladie d'Alzheimer.

15. Composition pour une utilisation selon la revendication 13 ou 14, dans laquelle la composition et l'agent thérapeutique sont administrés simultanément au sujet soit (i) par des voies d'administration séparées soit (ii) dans une seule composition.
